# EUROPEAN PATENT SPECIFICATION

(11) **EP 1 434 800 B1**
(45) Date of publication and mention of the grant of the patent: **01.12.2010**
(21) Application number: 02759649.3
(22) Date of filing: 13.09.2002
(51) Int. Cl.: C07K 16/00, C12N 15/00, G01N 33/53

(54) **IMMUNOGLOBULIN HAVING PARTICULAR FRAMEWORK SCAFFOLD AND METHODS OF MAKING AND USING**
IMMUNGLOBULIN MIT EINEM BESTIMMTEN RAHMENGERÜST UND VERFAHREN ZU DESSEN HERSTELLUNG UND VERWENDUNG
IMMUNOGLOBULINE DOTEE D'UN SQUELETTE PARTICULIER ; METHODES DE FABRICATION ET D'UTILISATION

(30) Priority: 14.09.2001 US 318904 P
(43) Date of publication of application: 07.07.2004
(73) Proprietor: Fraunhofer-Gesellschaft zur Förderung der angewandten Forschung e.V., 80686 München (DE)
(72) Inventor: ZHANG, Mei, Yun, Frederick, MD 21702 (US); SCHILLBERG, Stefan, 52074 Aachen (DE); ZIMMERMANN, Sabine, 50674 Koeln (DE); DI FIORE, Stefano, 41462 Neuss (DE); EMANS, Neil, B-4890 Thimister-Clermont (BE); FISCHER, Rainer, 52156 Monschau (DE)
(74) Representative: King, Hilary Mary
(86) International application number: PCT/US2002/029003
(87) International publication number: WO 2003/025124

(56) References cited:
- WO-A-00/25804
- WO-A-01/48017
- WO-A-01/49713
- WO-A-96/13594
- WO-A-03/089475
- WO-A1-91/09967
- DESIDERIO ANGIOLA ET AL: "A semi-synthetic repertoire of intrinsically stable antibody fragments derived from a single-framework scaffold" JOURNAL OF MOLECULAR BIOLOGY, vol. 310, no. 3, 13 July 2001 (2001-07-13), pages 603-615, XP004464207 ISSN: 0022-2836
- AUF DER MAUR A ET AL: "Antigen-independent selection of stable intracellular single-chain antibodies" FEBS LETTERS, ELSEVIER, AMSTERDAM, NL, vol. 508, no. 3, 23 November 2001 (2001-11-23), pages 407-412, XP004324279 ISSN: 0014-5793
- TAVLADORAKI PARASKEVI ET AL: "A single-chain antibody fragment is functionally expressed in the cytoplasm of both Escherichia coli and transgenic plants" EUROPEAN JOURNAL OF BIOCHEMISTRY, vol. 262, no. 2, June 1999 (1999-06), pages 617-624, XP002167437 ISSN: 0014-2956
- FISCHER RAINER ET AL: "Achieving plant disease resistance by antibody expression" CANADIAN JOURNAL OF PLANT PATHOLOGY, vol. 23, no. 3, September 2001 (2001-09), pages 236-245, XP009061451 ISSN: 0706-0661
- RADER ET AL.: 'The rabbit antibody repertoir as a novel source for the generation of therapeutic human antibodies' THE JOURNAL OF BIOLOGICAL CHEMISTRY vol. 275, no. 18, 05 May 2000, pages 13668 - 13676, XP001023515

## Description

### FIELD OF INVENTION

This invention relates to immunoglobulin molecules, either full-size or domains thereof, particularly single chain Fv antibodies, that accumulate to high levels in plant cells, preferably in predetermined cellular compartments, particularly the cytosol. The immunoglobulin molecules of this invention are suitable for production in prokaryotic cells and in eukaryotic cells, particularly plant cells. The molecules have preferred "framework scaffolds" associated with high level expression and accumulation of the molecules within the cells. Also disclosed are methods for generating the immunoglobulin molecules of this invention and methods of using the immunoglobulin molecules.

### BACKGROUND OF INVENTION

Plant disease constitutes a major and ongoing threat to human food stocks and animal feed. Most crop plants are regularly exposed to one or more fungal, bacterial or viral pathogens that result in substantial economic losses every year. One approach used to control infection and spread of plant pathogens and pests is to treat the plants with chemical based compounds. However, many of these chemical based compounds have the potential to accumulate and have undesirable environmental consequences. Another approach for pathogen control is to develop plants that are resistant to pathogens and pests thus reducing the need to treat plants with chemical compounds. Such plants may be generated, e.g., by introducing genes which confer resistance to the pathogens either by genetic engineering techniques or through conventional plant breeding techniques. Attempts have also been made to introduce pathogen specific recombinant antibodies into plant cells in an effort to control the onset or progression of plant disease (Voss et al. "Reduced virus infectivity in N. tabacum secreting a TMV specific full size antibody", Mol. Breeding, 1:39-50 (1995); Zimmermann et al. "Intracellular expression of TMV specific single chain Fv fragments leads to improved virus resistance in Nicotiana tabacum," Mol Breeding, 4:369-378 (1998); LeGall et al., "Engineering of a single chain variable fragment (scFv) antibody fragment specific for the stolbur phytoplasma (Mollicute) and its expression in Escherichia coli and tobacco plants" Appl. Environ Micro, 64:4566-4572 (1998), and; Schillberg et al., "Plasma membrane display of antiviral single chain Fv fragments confers resistance to tobacco mosaic virus," Mol. Breeding 6, 317-326 (2000)).

The expression of antibodies and antibody fragments in plants, "plantibodies", as a means to mediate resistance to pathogens or to modulate plant cellular functions is principally based on an antigen-antibody interaction: The antibodies or antibody fragments specifically bind to and inactivate pathogens. Therefore, accumulation of functional antibodies at sufficiently high levels in appropriate cell compartments in the transgenic plants is required. Since most processes involved in viral replication and spread take place within the plant cytosol (Wilson, "Strategies to protect crop plants against viruses: pathogen-derived resistance blossoms." Proc Natl Acad Sci USA, 90:3134-3141 (1993); Baulcombe, "Novel strategies for engineering virus resistance in plants." Curr Opin Biotechnol, 5: 117-124 (1994).), expression of specific antibodies for viral proteins in this compartment is desirable (Tavladoraki et al., "Transgenic plants expressing a functional single-chain Fv antibody are specifically protected from virus attack." Nature 366(6454): 469-472 (1993)). Expression of antibodies in plant cells is also advantageous for antibody or antibody fragments that are to be used in isolated form. In situations where antibodies or antibody fragments, e.g., the scFvs, are used in an isolated form, e.g., in therapeutic or diagnostic applications, expression in plant cells affords several advantages to expression in bacterial or mammalian systems, e.g., no requirement for complex culture media, sterility or large culture vessels (bioreactors), the possibility of disposing of plant waste material in an environmentally friendly manner, e.g., composting, and no contamination with mammalian viruses, oncogenes, or bacterial endotoxins.

Full-size immunoglobulin molecules comprise four polypeptide chains, two identical heavy chains and two identical light chains, complexed together by hydrophobic interactions and stabilized by disulfide bonds. The heavy and light chains comprise a constant "C" domain and a variable "V" domain. The variable domain binds antigen and is composed of framework regions (FR), which are those parts of the variable domain that are substantially conserved among heavy chains or light chains having different specificities, and complementarity determining regions (CDRs). CDRs are hypervariable regions in the variable domain and are responsible predominantly for antigen specificity. The framework regions and CDRs are generally organized into the variable domain as follows: FR1-CDR1-FR2-CDR2-FR3-CDR3-FR4.

Achieving high levels of full size antibodies in plant cells is problematic in the cytosol, due in part to the reducing conditions within this subcellular compartment and the absence of protein disulfide isomerase and homologs of the chaperonins, BiP (heavy-chain binding protein) and GRP94. BiP and GRP94 are involved in antibody folding and assembly, and the absence of these chaperonins, as well as the reducing conditions within the plant cytosol, may lead to degradation of unassembled heavy and light chains, Hiatt et al., "Production of antibodies in transgenic plants." Nature 342: 76-78 (1989). Attention has turned to producing V_{L} domains, V_{H} domains, and single chain Fv antibody fragments in plants, especially when the antibody fragments have to accumulate in the plant cytosol.

Single chain Fv (scFv) antibodies are small recombinant antibodies which consist of a variable light chain domain of an antibody and a variable heavy chain domain of an antibody molecule joined together by a flexible peptide linker (Bird et al., Science, 242:423-426 (1988) and Huston et al., Proc. Natl. Acad. Sci., 85:5879-5883 (1988)). ScFv antibodies can retain full antigen-binding activity but in contrast to full-size antibodies do not require post-translational assembly and complex protein folding to be functional ScFvs are used frequently in diagnostic and therapeutic applications, e.g., as radiolabeled molecules or fused with immunotoxins. ScFvs with different antigen specificities may also be joined to form bispecific antibodies (Mallender and Voss, J. Biol. Chem. 269:199-206 (1994)). In addition, scFv antibodies are suitable for the construction of bivalent bifunctional molecules, such as fusions to staphylococcal protein A, biotin, to an enzyme, or to another scFv (Ito and Kurosawa, "Development of an artificial antibody system with multiple valency using an Fv fragment fused to a fragment of protein A." J Biol Chem 27: 20668-20675 (1993); Gandecha et al., "Antigen detection using recombinant, bifunctional single-chain Fv fusion proteins synthesized in Escherichia coli." Prot Express Purif 5: 385-390 (1994); Fischer et al., "Expression and characterization of bispecific single chain Fv fragments produced in transgenic plants." Eur J Biochem, 262: 810-816 (1999)).

Mammalian-based ScFv antibodies, those generated from mRNA from mammalian cells, have been produced in plant cells as well as bacteria (see, e.g., Schouten et al. Plant Mol. Biol., 30:781-793 (1996);Schillberg et al., Trans. Res. 8:255-263 (1999); DeJaeger et al. Eur J. Bioc. 259:426-434 (1998); Tavladoraki et al., Nature 366:469-472 (1993); Fiedler et al., Immunotechnology 3: 205-216 (1997)). Constitutive cytosolic expression of an scFv antibody in tobacco mediated resistance against artichoke mottled crinkle virus was reported by Hiatt (Hiatt et al.,"Production of antibodies in transgenic plants", Nature 342: 76-78 (1989); and Owen et al. reported cytosolic expression of an anti-phytochrome scFv antibody (Owen et al., "Synthesis of a functional anti-phytochrome single-chain Fv protein in transgenic tobacco", Biotechnology (NY) 10: 790-794) (1992)). While scFvs have been successfully produced in plants, in the majority of reported transgenic plants expressing cytosolic scFv antibodies, the accumulation levels were found to be very low or at the detection limit (sub ng/g leaf material)(Owen, Biotechnology 1992; Zimmermann et al., Mol Breeding, 4:369-378 (1998); Bruyns et al. "Bacterial and plant-produced scFv proteins have similar antigen-binding properties" FEBS Letters 386: 5-10 (1996); Fecker et al., "Expression of single chain antibody fragments (scFv) specific for beet necrotic yellow vein virus coat protein in Escherichia coli and Nicotiana benthamiana", Plant Mol Biol 32: 979-986 (1996); Schouten et al., Plant Mol Biol.(1996*)).* Exceptions were reported by Tavladoraki et al., Nature 366:469-472 (1993) and DeJaeger et al Eur. J. Bioc. 259:426-434 (1998) who disclosed mouse derived cytosolic scFv that accumulated to a concentration of up to 0.1% to 1.0% of total soluble proteins respectively.

The factors that influence the accumulation of scFvs to high levels within plant cytosol are not clear (see e.g., Fiedler et al. Immunotechnology, 3:205-216 (1997); De Jaeger et al., Eur J Biochem. 259:426-434 (1999); Schouten et al., FEBS Letters, 415:235-241 (1997), and; Schillberg et al. Transgenic Research, 8: 255-263 (1999)) but may depend somewhat on the intrinsic properties of the antibody. For example conserved intrachain disulfide bonds are considered to be an important factor in the folding of the native antibody domain structure (De Jaeger et al., Eur J. Biochem. (1999)): However, some single chain antibodies can tolerate the deletion of a disulfide bridge and retain stability and functionality in yeast and mammalian cells (see, e.g., Biocca et al., Bio/Technology, 13:1110-1115 (1995) and Biocca et al., EMBO J., 9:101-108 (1990)). To date the expression of chicken-based scFv antibodies in plant cells has not been reported.

A strategy for achieving high level accumulation of scFv antibodies within particular cellular compartments is to construct scFv antibodies having particular framework scaffolds to enhance scFv accumulation. Disclosed herein are scFv antibodies having particularly preferred framework regions that accumulate to high levels within plant cells, including the cytosol. Also disclosed are methods for making and using the scFv antibodies of this invention.

### SUMMARY OF THE INVENTION

Described herein are immunoglobulin molecules comprising variable light chain (V_{L}) antibody domains or variable heavy chain (V_{H}) antibody domains or both V_{L} and V_{H} domains that are expressed at high levels within a plant cell, preferably within particular cellular compartments such as, e.g., the cytosol, endoplasmic reticulum, chloroplasts or apoplast. The V_{L} and V_{H} domains comprise framework scaffolds of particularly preferred framework regions and also comprise complementarity determining regions (CDRs). Immunoglobulin molecules as used herein include full-size antibodies comprising variable light chain (V_{L}) and variable heavy chain (V_{H}) domains, as well as single chain antibodies, diabodies, triabodies, and tetrabodies. Particularly preferred immunoglobulin molecules are single chain Fv fragment antibodies ("scFv"), The immunoglobulin molecules may optionally comprise additional sequences which facilitate their accumulation in particular plant compartments. Also disclosed are nucleic acid molecules encoding the immunoglobulin molecules of this invention the framework regions identified herein, V_{L} domains comprising one or more of the framework regions identified herein, V_{H} domains comprising one or more of the framework regions described herein or scFv antibodies comprising one or more of the framework regions described herein. The invention described herein also relates to vectors comprising the nucleic acid molecules of this invention useful for the production of the immunoglobulin molecules and to diverse libraries of immunoglobulin molecules having randomized complementarity determining regions (CDRs), but essentially the same framework scaffolds. The libraries may be screened for immunoglobulin molecules having desirable binding specificities and the nucleic acid molecules encoding the selected immunoglobulin molecules may be isolated and expressed in plant cells at high levels. Also described are host cells and transgenic plants expressing the immunoglobulin molecules, particularly the scFv antibodies, of this invention. Also described are methods to generate scFv antibodies with chimeric variable domains. The scFv antibodies with chimeric variable domains are composed of the framework regions (FRs) of the scFv antibody scaffolds of the present invention and the CDRs of donor antibodies, e.g., avian, piscean, mammalian, e.g., murine, human or camelid, donor antibodies, which are grafted to replace the native CDRs. The rationale behind these methods is to combine the stability and high level of protein accumulation in host cells of the scFv antibody scaffold disclosed in the present invention with the binding features of other antibodies of different origin that are poorly expressed in the host cells. Therefore, the scFv antibodies with chimeric variable domains are designed to keep the original binding properties of the parental antibodies or incorporate improved binding kinetics.

The immunoglobulin molecules of this invention may be used in any diagnostic and therapeutic assay that use immunoglobulin molecules, e.g., ELISA, protein chips or tumor imaging. The nucleic acid molecules encoding the immunoglobulin molecules of this invention may also be used to generate phage display libraries by any method known in the art.

### BRIEF DESCRIPTION OF THE FIGURES

Fig. 1: Analysis of phagemid-scFv DNA on agarose gels.
Fig: 2: Reactivity of monoclonal phage from full-length NS_{M} panned library using GST fusion proteins in capture phage ELISA.
Fig. 3: pTMZ1 expression vector map and nucleotide sequence of the expression cassette (SEQ ID NO: 20 and 21).
Fig. 4 Immunoblot analysis of bacterially expressed soluble ScFvs.
Fig. 5 Reactivity of affinity purified soluble scFvs in indirect ELISA using NS_{M} protein from inclusion bodies (A) and capture ELISA using soluble NS_{M} protein obtained from the IMPACT system (B).
Fig. 6: Strategy for cloning of scFv genes into the plant expression pSSH1 vector. NS_{M}-specific or non-specific scFv genes (Table 3) were cloned into the pSSH1 vector for the expression and targeting of scFvs to the plant cytosol (A) or secretion into the apoplastic space (B).
Fig. 7: Immunoblot analysis of cytosolically expressed scFvs in transgenic T₀ tobacco plants.
Fig. 8: Immunoblot analysis of cytosolically expressed scFvs in transgenic T₁ plants.
Fig. 9: Sequence of the scFvG19-29 compiled pursuing the semi-conservative strategy described herein.
Fig. 10 A-D: Depiction of the preparation of plasmid scFvG19-29-H.
Fig. 11 A-F: Depiction of the preparation of plasmid scFvG19-29.

### DETAILED DESCRIPTION OF THE INVENTION

The immunoglobulins described herein comprise essentially identical framework regions (FRs), which form a framework scaffold, and also comprise various complementarity determining regions (CDRs). The CDRs may be randomized or may be specific for a predetermined antigen. The immunoglobulins are particularly useful for production in plant cells, as they accumulate to high levels and can be directed to particular plant cell compartments. A library of such immunoglobulin molecules can be screened or "panned" for those having specificity to a predetermined antigen and immunoglobulin with a desired specificity and the nucleic acid encoding these immunoglobulin molecules can be used to produce the immunoglobulin molecules at high levels within host cells, avian, piscean, mammalian and plant cells.

There are various methods available in the art for generating single chain antibodies. Generally mRNA is isolated from an immunoglobulin producing cell and cDNA encoding the variable domain of antibody light chains or the variable domains of antibody heavy chains are synthesized by RT-PCR using primers designed to amplify variable regions of the heavy or light chains. Mammalian genomes comprise approximately 100 unique variable, "V", genes that encode V segments, which comprise the N-terminal FR1-CDR1-FR2-CDR2-FR3-CDR3 portion of the variable domain. Mammalian genomes also comprise four to six joining "J" minigenes, which encode the J segment, which is the carboxy terminal CDR3-FR4 portion of the variable domain. The V genes and J minigenes rearrange within the antibody producing cells to generate a diverse population of genes comprising both V gene and J minigene sequences. To amplify the variable region of the mRNAs transcribed from this diverse population of mammalian rearranged genes, consensus primers complementary to conserved sequences are used to generate cDNAs from the mRNAs encoding the variable regions. Once the cDNAs are prepared and isolated they may be cloned into a suitable vector in operable linkage with a promoter to express the V_{L} or V_{H} domains individually. The V_{L} and V_{H} encoding cDNAs may also be cloned into a vector that further comprises a nucleotide sequence encoding a "linker", which is a flexible chain of amino acid residues that serves to link the variable domain of the heavy chain and the variable domain of the light chain to form a single polypeptide, a single chain Fv (scFv) antibody. In general, the V_{L} and V_{H} cDNAs are cloned into the vector such that a single polypeptide is produced comprising the variable region of the heavy chain (V_{H}) and the light chain (V_{L}) linked together via the flexible linker molecule in a V_{H}-linker-V_{L} orientation or a V_{L}-linker-V_{H} orientation. For a general discussion of methods for producing scFv libraries see, e.g., Hoogenboom, "Designing and optimizing library selection strategies for generating high affinity antibodies" Trends Biotechnol., 15: 62-70 (1997); Hoogenboom, et al. "Antibody phage display technology and its applications", Immunotechnology 4:1-20 (1998); McGregor "Selection of proteins and peptides from libraries displayed of filamentous bacteriophage", Mol. Biotechnol, 6:155-62 (1996); and Bird et al., "Single-chain antigen binding proteins", Science, 242:423-426 (1988) (all incorporated herein by reference.)

In contrast to mammals, chickens encode single functional "V" and "J" segments for the heavy and light chains. In chicken, the nucleotide sequences encoding the 3' region of framework 4 (FR4), which is derived from J_{λ} or J_{H}, is highly conserved in mature antibody encoding mRNA. Gene conversion is frequent and thus the 5' region of framework 1 (FR1) is also conserved (McCormack and Thompson, "Chicken IgL variable region gene conversions display pseudogene donor preference and 5' to 3' polarity." Genes Dev 4: 548 (1990)). In addition, the sequences of pseudogenes corresponding to this region do not show much divergence (Reynaud and Anquez, et al., "A hyperconversion mechanism generates the chicken light chain preimmune repertoire", Cell 48: 379-386 (1987)). Thus, it is possible to perform RT-PCR of the heavy and light chain V regions of a diverse population of immunoglobulin-encoding mRNAs from chickens with a single pair of primers for the heavy chain and a single pair for the light chain. It has been demonstrated that chicken derived scFv antibodies can be expressed in *E. coli* as scFv-gene III fusion proteins on the surface of filamentous phage, where they retain the capacity for antigen specific binding. Specific clones can be retrieved from a phage library whose diversity is based on either the 'naïve' repertoire present in the bursa of non-immunized chicken (Davies, et al., "Selection of specific phage-display antibodies using libraries derived from chicken immunoglobulin genes", J Immunol Methods 186: 125-135 (1995)) or a biased repertoire present in the spleen of immunized chicken (Yamanaka et al., "Chicken monoclonal antibody isolated by a phage display system", J Immunol 175(3): 1156-1162 (1996)). In the experiments described herein messenger RNA was isolated from chicken cells and the variable regions of the heavy and light chains were amplified using the following primers:

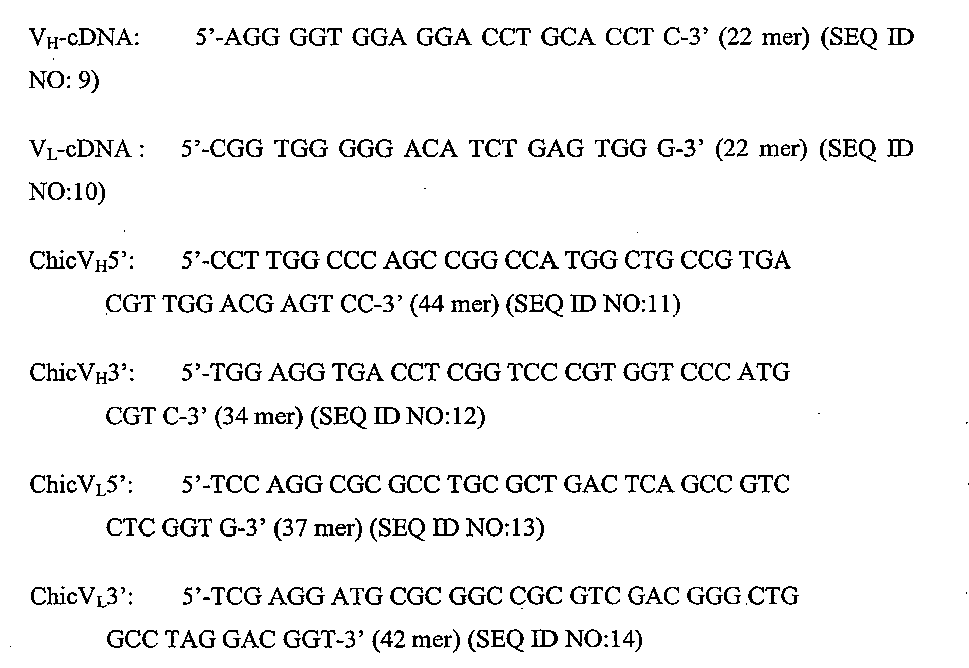

V_{H}-cDNA and V_{L}-cDNA primers were used for reverse transcription (RT) of V_{H} and V_{L} domain mRNA. ChicV_{H}5' and ChicV_{H}3' were used for amplification of heavy chain variable domain (V_{H}) by PCR, ChicV_{L}5' and ChicV_{L}3' for amplification of light chain variable domain (V_{L}). Although particular primers are disclosed above, those of skill in the art could readily determine other V_{H} and V_{L} primers suitable for amplifying the V_{L} and V_{H} domains based on the information known in the art and disclosed herein.

The amplified nucleic acid molecules may be isolated and subsequently cloned into appropriate vectors and expressed. Thus an aspect of this invention are vectors containing either V_{L}-encoding nucleic acid molecule(s), or a V_{H}-encoding nucleic acid molecule(s) or a nucleic acid molecule that encodes an immunoglobulin molecule comprising both V_{L} and V_{H} joined together via a linker polypeptide, e.g., a scFv antibody, a diabody, a triabody or a tetrabody, as well as bispecific antibodies comprising two scFvs connected via a linker.

We identified framework scaffolds associated with high level accumulation in plant cells by preparing chicken V_{L}, V_{H} and scFv antibody molecules and then determining which framework regions were common among "high producers," that is, those which accumulated the chicken derived immunoglobulin molecules to a concentration to least about 0.2% of the total soluble protein in plant cells to about 30%, preferably at least about 0.4% up to about 30% and more preferably at least about 1% to about 15%. The immunoglobulin may also accumulate to at least about 2% to 15% of the TSP. Briefly, *E coli* cells were transformed with nucleic acid molecules encoding scFv antibody molecules and individual clones assayed for the expression level of the scFv antibody. The nucleic acid molecules encoding the scFvs were then isolated and the nucleotide sequence and amino acid sequence of the scFv antibodies were determined and compared. The cDNA clones that produced scFv antibody molecules at high levels in *E. coli* were introduced into plant cells and the expression of the antibody molecules were assayed in both transient assays and long term assays in stably transformed plants. Those of skill in the art are familiar with assays which measure transient expression and long term expression of transgenes. See, for example, Kapila et al. who describe a process wherein transient expression of an *Agrobacterium* based expression vector is assayed in tobacco leaves (Kapila et al., "An Agrobacterium mediated transient gene expression system for intact leaves", Plant Sci. 122: 101-108 (1997), incorporated herein by reference). The production of scFv, V_{L} and V_{H} molecules can also be assayed in a long term assay wherein an expression vector becomes established within a transformed cell, e.g., integrated into the cellular genome, and the transformed cells are cultured *in vitro* under conditions which promote expression of the immunoglobulin molecules. Alternatively, transgenic plants may be assayed for long term expression of the immunoglobulin by assaying the level of immunoglobulin molecules in the whole plant or selected plant tissue.

By comparing the sequences of the scFv antibodies expressed in plant cells, preferably the cytosol, at levels of at least about 0.2% of total soluble protein, we established a sequence of framework regions that is associated with high level expression of scFv antibodies in plant cells, including the cytosol. Thus, the immunoglobulin molecules of this invention are based generally on the structure of a light chain variable region (V_{L}) and heavy chain variable region (V_{H}) of an avian antibody and comprise the structure:
(a) LFR1- -CDR-L1--LFR2- -CDR-L2--LFR3- -CDR-L3--LFR4 and
(b) HFR1 - -CDR-H1--HFR2- -CDR-H2--HFR3- -CDR-H3--HFR4
as defined in claim 1. In one embodiment, (a) and (b) may be joined together via a peptide linker.

The abbreviation in the structures above, are as follows:
LFR1, LFR2, LFR3 and LFR4 are respectively a first, second, third and fourth light chain framework region;
CDR-L1, CDR-L2 and CDR-L3 are respectively, a first, second and third light chain complementarity determining region;
HFR1, HFR2, HFR3 and HFR4 are respectively a first, second, third and fourth heavy chain framework region, and;
CDR-H1, CDR-H2 and CDR-H3 are respectively, a first, second and third heavy chain complementarity determining region.

The length of the amino acid sequence of the framework regions and complementarity determining regions are:
LFR1, about 22 to about 23 amino acid residues, preferably about 22 amino acid residues and most preferably LFR1 comprises SEQ ID NO: 5;
LFR2, preferably 16 amino acid residues, wherein a proline or leucine must be at position 10 if the sequence is 15 amino acid residues long or position 11 if the sequence is 16 amino acid residues long, and most preferably LFR2 comprises SEQ ID NO: 6;
LFR3, about 32 amino acid residues, preferably 32 amino acid residues, and most preferably LFR3 comprises SEQ ID NO: 7;
LFR4, about 12 to about 13 amino acid residues, preferably 13 amino acid residues, wherein the first amino acid residue is Phe, and most preferably LFR4 comprises SEQ ID NO: 8;
CDR-L1, preferably about 5 to about 14 amino acid residues, more preferably about 8, 9, 10 or 13 amino acid residues;
CDR-L2, preferably about 5 to about 7 amino acid residues, more preferably 7 amino acid residues;
CDR-L3 is preferably about 5 to about 15 amino acid residues, more preferably about 8 to about 12 amino acid residues;
HFR1, about 30 amino acid residues, preferably HFR1 comprises SEQ ID NO: 1;
HFR2, about 14 amino acid residues, preferably HFR2 comprises SEQ ID NO: 2;
HFR3, about 29 to about 32 amino acid residues, preferably 32 amino acid residues. The first amino acid residue may be Arginine (Arg) and the tenth amino acid residue may be glutamine (Gln), and most preferably HFR3 comprises SEQ ID NO: 3;
HPR4, about 9 amino acid residues, wherein the first amino acid residue is Trp, and most preferably HFR4 comprises SEQ ID NO: 4;
CDR-H1, preferably about 5 to about 7 amino acid residues, more preferably 5 amino acid residues;
CDR-H2, preferably about 16 to about 18 amino acid residues, more preferably 17 amino acid residues, and;
CDR-H3 of about 9 to about 21 amino acid residues, preferably about 9 to about 19 amino acid residues, more preferably about 14 to about 19 amino acid residues.

The V_{L} and V_{H} domains may be linked such that V_{L} is attached to the carboxy terminal of the linker and V_{H} is attached to the amino terminal of the linker or vice versa, V_{H} is attached to the carboxy tenninal of the linker and V_{L} is attached to the amino terminal of the linker.

The immunoglobulin molecules of this invention may be multimers of V_{L} or V_{H} or both, and V_{L} and V_{H} may differ in affinity or specificity for one or more antigens. Examples of such multimers include, e.g., diabodies, triabodies and tetrabodies. The diabodies, triabodies and tetrabodies may also be multivalent, comprising a plurality of subunits comprising V_{L} or V_{H} wherein the specificity of V_{L} and V_{H} differ. Those of skill in the art are familiar with methods for the production of such antibody forms, see e.g., Perisic et al., Structure 2:1217-1226 (1994); Pei et al., PNAS, 94:9637-9642 (1997) Hollinger et al., Protein Engineering 9:299-305 (1996); Millstein and Cuello, Nature 305, 537-539 (1983); Yamanaka et al. (1996). "Chicken monoclonal antibody isolated by a phage display system." J Immunol 175(3): 1156-1162.

Immunoglobulin molecules e.g., V_{L}, V_{H} or scFvs, comprising the framework regions of this invention are useful for generating diverse libraries of immunoglobulin molecules, wherein the immunoglobulin molecules comprise the framework regions described herein and various CDRs. The CDRs may be completely randomized by any process that is known in the art (see e.g., Knappik et al., J. Mol. Biol. 296:57-86 (2000), and U.S. Patent No. 6,096,551.)

Molecular analysis of naturally occurring and artificial libraries has been greatly improved by the development of various "display" methodologies. The general scheme behind display techniques is to screen a multitude of peptides that are presented on a biological surface (phage, eukaryotic cell or prokaryotic cell e.g., bacteria, etc), for those having a particular biological function and then isolating the nucleic acid encoding the selected peptide.

In U.S. Patent 5,821,047, monovalent phage display is described. This method provides for the selection of novel proteins, and variants thereof. The method comprises preparing a nucleic acid molecule encoding a fusion of a polypeptide of interest, e.g., the immunoglobulin molecule of this invention, to the carboxy terminal domain of the gene III coat protein (cp) of the filamentous phage M13 to form a library of structurally related fusion proteins that are expressed in low quantity on the surface of phagemid candidates.

U.S. Patent 5,571,698 describes directed evolution using an M13 phagemid system. A protein is expressed as a fusion with the M13 gene III protein. Successive rounds of mutagenesis are performed, each time selecting for improved biological function, e.g., binding of a protein to a cognate binding partner.

Heterodimer phage libraries are described in U.S. Patent 5,759,817. Filamentous phage comprising a matrix of coat protein (cp)VIII proteins encapsulating a genome encoding first and second polypeptides of an autogenously assembling receptor, such as an antibody, are provided. The receptor is surface-integrated into the phage coat matrix via the cpVIII membrane anchor, presenting the receptor for biological assessment.

Another system, lambdoid phage, also can be used for display purposes. In U.S. Patent 5,672,024, lambdoid phage comprising a matrix of proteins encapsulating a genome encoding first and second polypeptides of an autogenously assembling receptor are prepared. The surface-integrated receptor is available on the surface of the phage for characterization.

Immunoglobulin heavy chain libraries may also be displayed by phage as described in U.S. Patent 5,824,520. A single chain antibody library is generated by creating highly divergent, synthetic hypervariable regions, followed by phage display and selection. The resulting antibodies were used to inhibit intracellular enzyme activity. Another patent describing antibody display is U.S. Patent 5,922,545.

Bacteria also have been used successfully to display proteins. U.S. Patent 5,348,867, describes expression of proteins on bacterial surfaces. The compositions and methods provide stable, surface-expressed polypeptide from recombinant gram-negative bacterial cell hosts. A tripartite chimeric gene and its related recombinant vector include separate DNA sequences for directing or targeting and translocating a desired gene product from a cell periplasm to the external cell surface. A wide range of polypeptides may be efficiently surface expressed using this system. See also, U.S. Patents 5,508,192 and 5,866,344.

U.S. Patent 5,500,353 describes another bacterial display system. Bacteria (e.g., Caulobacter) having a S-layer modified such that the bacterium S-layer protein gene contains one or more in-frame fusions coding for one or more heterologous peptides or polypeptides is described. The proteins are expressed on the surface of the bacterium, which may be cultured as a film.

Antibody display libraries may be screened for molecules specific for a predetermined antigen, e.g., an antigenic determinant of a virus, bacteria, fungus, nematode, insect, or an enzyme or particular cellular determinant. The nucleic acid molecules encoding the immunoglobulin molecules specific for the predetermined antigen may then be introduced into a plant cell by any process known in the art to generate a transgenic plant expressing the immunoglobulin molecule of this invention. The nucleic acid molecules may comprise a sequence encoding a targeting polypeptide such that an immunoglobulin molecule comprising a targeting polypeptide is produced within the plant cell to direct accumulation of the immunoglobulin molecules of this invention to a particular plant compartment.

A plant that is resistant to a pathogen, e.g., a virus, bacteria, fungus, insect or nematode can be generated by transforming a plant cell with a nucleic acid molecule encoding an immunoglobulin molecule, e.g., one derived from a chicken antibody, preferably a V_{L}, V_{H} or scFv antibody molecule, which comprises the framework regions identified herein and which specifically binds to a plant pathogen, then regenerating a transgenic plant from the transformed plant cell and growing the plant under conditions such that the immunoglobulin molecule of this invention is produced at a concentration sufficient to promote resistance of the plant to the pathogen, preferably the concentration is at least about 0.2% to 30% of total soluble cellular protein (TSP), preferably at least 0.4% to 30% the TSP and more preferably about 1-15% of the TSP. The immunoglobulin may accumulate to a level of at least 2% to 15%.

Also an aspect of this invention is a transgenic plant expressing an immunoglobulin molecule of this invention that is specific for a plant pathogen. Preferably the transgenic plant is resistant to infection or propagation of the pathogen. Such pathogens include, e.g., viruses, bacteria, fungi, nematodes and insects.

The transgenic plants expressing the immunoglobulin molecules of this invention may also serve as a source for isolated immunoglobulin molecules, preferably isolated V_{L}, V_{H} or scFv antibody molecules. The immunoglobulin molecules may be isolated from the transgenic plant for use, e.g., in therapeutic or diagnostic applications, such as, e.g., ELISA assays, protein chips or tumor imaging. Immunotherapy has been used to promote tumor regression, e.g., colorectal and gastric carcinomas, see e.g., US Patent 5, 851,526 and US Patent 5,958,412.

One embodiment of this invention is a plant composition comprising immunoglobulin molecules comprising V_{L} or V_{H} domains, which comprise the framework regions identified herein, that accumulate in plant cells to high levels, at least about 0.2% to 30% of total soluble cellular protein (TSP), preferably at least 0.4% to 30% the TSP and more preferable about 1-15% of the TSP. The immunoglobulin may accumulate to levels of at least 2% to 15% of TSP. Preferably the immunoglobulin molecules are isolated V_{L} domains, V_{H} domains or single chain Fv antibodies comprising the V_{L} and V_{H} domains.

The amino acid residues within the framework regions are preferably: position 1 in HFR3 is preferably Arginine (Arg) and position 10 is preferably Glutanime (Gln); position 1 in HFR4 is preferably Tryptophan (Trp); position 10 or 11 in LFR2 is preferably Proline (Pro) or Leucine (Leu) and position 6 in LFR12 is preferably serine; and position 1 in LFR4 is preferably Phenylalanine (Phe). In one embodiment the framework regions comprise one or more of SEQ ID NO:1, 2, 3, 4, 5, 6, 7, or 8. In another embodiment the framework regions comprise comprise one or more of SEQ ID NO:1, 2, 3, 4, 5, 6, 7, or 8 having conservative substitutions at one of more amino acid residue positions provided position 1 in HFR3 is Arginine (Arg) and the tenth amino acid residue is glutamine (Glu); position 1 in HFR4 is Tryptophan (Trp); position 10 or 11 in LFR2 is Proline (Pro) or Leucine (Leu) and position 6 in LFR2 is serine; and position 1 in LFR4 is Phenylalanine (Phe).

Preferably the V_{H} framework scaffold comprises one or more framework regions set forth in Table 5 or Table 7 and complementarity determining regions (CDRs H1, H2 and H3). More preferably the V_{H} framework scaffold comprise one or more framework regions having amino acid sequences selected from the group consisting of SEQ ID NO: 1(HFR1), SEQ ID NO: 2 (HFR2), SEQ ID NO: 3 (HFR3), or SEQ ID NO: 4 (HFR4). Most preferably the framework scaffold comprises framework regions SEQ ID NO:1(HFR1), SEQ ID NO: 2(HFR2), SEQ ID NO: 3(HFR3) and SEQ ID NO: 4(HFR4). The variable domain of the light chain (V_{L}) also comprises complementarity determining regions (CDR-L1, L2 and L3) and a framework scaffold. Preferably the V_{L} framework scaffold comprises one or more framework regions set forth in Table 5 or Table 7. Preferably the V_{L} framework scaffold comprises one or more framework regions having amino acid sequences selected from the group consisting of SEQ ID NO: 5 (LFR1), SEQ ID NO: 6 (LFR2), SEQ ID NO: 7 (LFR3), and SEQ ID NO: 8 (LFR4). More preferably the light chain framework scaffold comprises framework regions SEQ ID NO: 5(LFR1), SEQ ID NO: 6(LFR2), SEQ ID NO: 7(LFR3) and SEQ ID NO: 8(LFR4).

The scFv antibodies comprise a light chain variable domain (V_{L}) and a heavy chain variable domain (V_{H}) as set for the *supra* joined together via a linker polypeptide. The variable light chain may be joined via its carboxy terminal end to the amino terminal end of a linker and the carboxy terminal end of the linker is joined to the amino terminal end of the variable region of the heavy chain, in a "V_{L}-Linker V_{H}" orientation. Alternatively the variable heavy chain may be joined via its carboxy terminal end to the amino terminal end of the linker and the carboxy terminal end of the linker is joined to the amino terminal end of the variable region of the light chain, in a "V_{H}-linker-V_{L}" orientation. Preferable the scFv antibody comprises a V_{L} domain and a V_{H} domain having one or more framework regions set forth in Table 5 or 8. More preferably the scFv antibodies comprise the framework regions identified in Table 5 or 8 and most preferably the scFv antibodies comprise the amino acid sequence:

Or:

One or more of the amino acid residues at positions 18, 19 or 20 in SEQ ID NO:3, or the amino acid residues at position 6 in SEQ ID NO:6, or the amino acid residues at position number 10 in SEQ ID NO:8 may be absent. In alternate embodiments of the invention, Xaa in SEQ ID NO: 5 may be absent or may be any amino acid, preferably serine.

Almost all of the amino acid residues crucial for the stability of human antibodies disclosed by Knappik et al. (JMB, 296:57-86 (2000)) are also present in chicken antibodies. Those of skill in the art appreciate that certain amino acid residues may be substituted for other amino acid residues in a protein structure without appreciable loss of interactive capacity with structures such as, for example, substrate-binding regions. These changes are termed "conservative" in the sense that they preserve the structural and, presumably, required functional qualities of the starting molecule. Conservative amino acid residue substitutions generally are based on the relative similarity of the amino acid residue side-chain substituents, for example, their hydrophobicity, hydrophilicity, charge, size, and the like. An analysis of the size, shape and type of the amino acid residue side-chain substituents reveals that arginine, lysine and histidine are all positively charged residues; that alanine, glycine and serine are all a similar size; and that phenylalanine, tryptophan and tyrosine all have a generally similar shape. Therefore, based upon these considerations, arginine, lysine and histidine are defined herein as equivalent to each other; alanine, glycine and serine are defined herein as equivalent to each other; and phenylalanine, tryptophan and tyrosine are defined herein as equivalent to each other.

In making such conservative substitutions, the hydropathic index of amino acid residues also may be considered. Each amino acid residue has been assigned a hydropathic index on the basis of their hydrophobicity and charge characteristics, these are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

The importance of the hydropathic amino acid index in conferring interactive biological function on a protein is generally understood in the art (Kyte and Doolittle, J. Mol. Biol. 157, 105-132(1982). It is known that certain amino acid residues may be substituted for other amino acid residues having a similar hydropathic index or score and still retain a similar biological activity. In making changes based upon the hydropathic index, the substitution of amino acid residues whose hydropathic indices are within +/-2 is preferred, those which are within +/-1 are particularly preferred, and those within +/-0.5 are even more particularly preferred.

It also is understood in the art that conservative substitutions of like amino acid residues can be made effectively on the basis of hydrophilicity, particularly where the polypeptide created is intended for use in immunological embodiments, as in the present case. U.S. Pat. No. 4,554,101, incorporated herein by reference, states that the greatest local average hydrophilicity of a protein, as governed by the hydrophilicity of its adjacent amino acid residues, correlates with its immunogenicity and antigenicity, i.e., with a biological property of the protein.

As detailed in U.S. Pat. No. 4,554,101, the following hydrophilicity values have been assigned to amino acid residues: arginine (+3.0); lysine (+3.0); aspartate (+3.0+/-1); glutamate (+3.0+/-1); serine (+0.3); asparagine (+0.2); glutamine (+0.2); glycine (0); threonine (-0.4); proline (-0.5+/-1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4).

In making conservative substitutions based upon similar hydrophilicity values, the substitution of amino acid residues whose hydrophilicity values are within +/-2 is preferred, those which are within +/-1 are particularly preferred, and those within +/-0.5 are even more particularly preferred.

Numerous scientific publications have been devoted to the prediction of secondary structure, and to the identification of epitopes, from analyses of amino acid sequences (Chou and Fasman, Biochemistry, 13(2):211-222, 1974b; Chou and Fasman, Ann. Rev. Biochem., 47:251-276, 1978b; Chou and Fasman, Bioplays. J., 26:367-384. 1979; Chou and Fasman, Biochemistry, 13(2):222-245, 1974a, and; Chou and Fasman, Adv. Enzymol. Relat. Areas Mol. Biol., 47:45-148, 1978a). Any of these may be used, if desired, to supplement the teachings of Hopp in U.S. Pat. No. 4,554,101. Moreover, computer programs are currently available to assist with predicting antigenic portions and epitopic core regions of proteins. Examples include those programs based upon the Jameson-Wolf analysis (Jameson and Wolf, Comput. Appl. Biosci., 4(1):181-186, 1988; Wolf et al., Comput. Appl. Biosci., 4(1):187-191, 1988), the program PepPlot.RTM. (Brutlag et al., Comput Appl Biosci, 6(3):237-45, 1990; . Weinberger et al., Science, 228(4700):740-2, 1985), and other new programs for predicting protein tertiary structure (Fetrow and Bryant, Biotechnology, 11(4):479-84 (1993)),

Two designations for amino acid residues are used interchangeably throughout this application, as is common practice in the art. Alanine=Ala (A); Arginine=Arg (R); Aspartate=Asp (D); Asparagine=Asn (N); Cysteine=Cys (C); Glutamate=Glu (E); Glutamine=Gln (Q); Glycine=Gly (G); Histidine=His (H); Isoleucine=Ile (I); Leucine=Leu (L); Lysine=Lys (K); Methionine=Met (M); Phenylalanine=Phe (F); Proline=Pro (P); Serine=Ser (S); Threonine=Thr (T); Tryptophan=Trp (W); Tyrosine=Tyr (Y); Valine=Val (V).

Because of the degeneracy of the genetic code, a given polypeptide may be encoded by many nucleic acids. For example, four different three-base codons encode the amino acid residues alanine, glycine, proline, threonine and valine, while six different codons encode arginine, leucine and serine. Only methionine and tryptophan are encoded by a single codon. Table 1 lists the amino acid residues by name, three-letter and one-letter codes, and their corresponding codons for use in such embodiments.

**TABLE 1**

| Amino Acids | Code | | Codons | | | | | |
|---|---|---|---|---|---|---|---|---|
| Alanine | la | A | GCA | GCC | GCG | GCU | | |
| Cysteine | Cys | C | UGC | UGU | | | | |
| Aspartic acid | Asp | D | GAC | GAU | | | | |
| Glutamic acid | Glu | E | GAA | GAG | | | | |
| Phenylalanine | Phe | F | UUC | UUU | | | | |
| Glycine | Gly | G | GGA | GGC | GGG | GGU | | |
| Histidine | His | H | CAC | CAU | | | | |
| Isoleucine | Ile | I | AUA | AUC | AUU | | | |
| Lysine | Lys | K | AAA | AAG | | | | |
| Leucine | Leu | L | UUA | UUG | CUA | CUC | CUG | CUU |
| Methionine | Me | M | AUG | | | | | |
| Asparagine | Asn | N | AAC | AAU | | | | |
| Proline | Pro | P | CCA | CCC | CCG | CCU | | |
| Glutamine | Gln | Q | CAA | CAG | | | | |
| Arginine | Arg | R | AGA | AGG | CGA | CGC | CGG | CGU |
| Serine | Ser | S | AGC | AGU | UCA | UCC | UCG | UCU |
| Threonine | Thr | T | ACA | ACC | ACG | ACU | | |
| Valine | Val | V | GUA | GUC | GUG | GUU | | |
| Tryptophan | Trp | W | UGG | | | | | |
| Tyrosine | Tyr | Y | UAC | UAU | | | | |

Thus another aspect of this invention are immunoglobulins wherein one or more amino acid residues in the framework region identified in the high producers in Table 5 and the master gene in Table 7 are altered by conservative substitutions as discussed *supra* with the *proviso* that the amino acid residues corresponding to positions 66 (residue 1 in SEQ ID NO: 3) and 104 (residue 1 in SEQ ID NO: 4)in the heavy chain (SEQ ID NO: 15) (Table 7) are only arginine and tryptophan, and the amino acid residues at position 98 (residue 1 in SEQ ID NO: 8) in the light chain (SEQ ID NO: 16)(Table 7) is only phenylalanine.

The "linker" joining the two variable regions of the single chain antibody may be any suitable polypeptide linker known in the art. U.S. Patent 5,856,456 and Whitlow et al. "An improved linker for single-chain Fv with reduced aggregation and enhanced proteolytic stability", Prot. Eng. 6: 989-995 (1993) provide examples of peptide linkers for use in connecting polypeptide constituents to make fusion proteins, e.g., single chain antibodies. The linker is generally up to about 50 amino acid residues in length, preferably the linker is a sequence of about 14 to 25 amino acid residues. Preferably the linker is the 218 linker or derivative thereof. More preferably the linker comprises the sequence Ser Ser Gly Ser Thr Ser Gly Ser Gly Lys Pro Gly Pro Gly Glu Gly Ser Thr Lys Ser Gly (SEQ ID NO:17).

The complementarity determining regions (CDR) of the heavy and light chains are denoted above as CDR-H1, H2 or H3 and CDR-L1, L2 or L3. The amino acid sequences of the CDRs may be randomized by any means known in the art, e.g., Knappik et al. (2000), or Vimekäs et al., Nucleic Acids Res., 22;5600-5607 (1994)

Preferably CDR-H1, a first complementarity determining region of the heavy chain variable region, comprises about 5 to about 7 amino acid residues. More preferably CDR-H1 consists of about 5 amino acid residues.

Preferably CDR-H2, a second complementarity determining region of heavy chain variable region, comprises about 16 to about 18 amino acid residues, more preferably 17 amino acid residues.

Preferably CDR-H3, a third complementarity determining region of heavy chain variable region, comprises about 9 to about 19 amino acid residues, more preferably about 14 to about 19 amino acid residues.

Preferably CDR-L1, a first complementarity determining region of the light chain variable region, comprises about 5 to about 14 amino acid residues, more preferably about 8, 9, 10 or 13 amino acid residues.

Preferably CDR-L2, a second complementarity determining region of light chain variable region, comprises about 5 to about 7 amino acid residues, more preferably about 7 amino acid residues.

Preferably CDR-L3, a third complementarity determining region of light chain variable region, comprises about 5 to about 15 amino acid residues, more preferably about 8 to about 12 amino acid residues.

The immunoglobulin molecules of this invention may further comprise an N-terminal or C-terminal cellular targeting polypeptide. If the immunoglobulin molecules of this invention do not contain a cellular targeting signal sequence, the molecules will be located predominantly in the cytosol of the cells. In order to direct the immunoglobulin molecules to a particular compartment of a cell it is preferable to include a cellular targeting sequence in the molecule. Thus a further aspect of this invention is a fusion of the immunoglobulin molecules described herein and a targeting polypeptide.

Those of skill in the art appreciate that there are many cellular targeting polypeptides that would direct a polypeptide to a particular component in the plant cell , e.g., the apoplast, the endoplasmic reticulum (ER), vacuole, the plastid, the chloroplast, or a protein body (see, e.g., Gomod and Fay, "Signals and mechanisms involved in intracellular transport of secreted proteins in plants", Plant Physiol. Biochem, 34:165-181 (1996) and Moloney and Holbrook, "Subcellular targeting and purification of recombinant proteins in plant production systems" Biotechnol. Genet. Eng. Rev., 14:321-336 (1997).

Cellular targeting sequences joined to the coding sequence of an expressed gene which may be removed post-translationally from the initial translation product and facilitate the transport of the protein into or through intracellular or extracellular membranes, are termed transit sequence (usually into plastids and other intracellular organelles) and signal sequences (usually to vacuoles, vesicles, the endoplasmic reticulum, golgi apparatus and outside of the cellular membrane). By facilitating the transport of the protein into compartments inside and outside the cell, these sequences may increase the accumulation of gene product by protecting them from proteolytic degradation. In addition, mRNA being translated by ribosomes is more stable than naked mRNA, thus the presence of translatable mRNA upstream of sequences encoding a desired polypeptide may increase the overall stability of the mRNA transcript and thereby increase synthesis of the desired gene product. Since transit and signal sequences are usually post-translationally removed from the initial translation product, the use of the transit and signal sequences allows for the addition of extra translated sequences without additional amino acid residues attached to the final polypeptide. It further is contemplated that targeting of certain proteins may be desirable in order to enhance the stability of the protein (U.S. Patent No. 5,545,818).

Additionally, vectors may be constructed and employed for intracellular targeting of a specific gene product to particular compartments or cells of a transgenic plant or for directing a protein to the extracellular environment. This generally will be achieved by joining a DNA sequence encoding a transit or signal peptide sequence to the coding sequence of a particular gene. The resultant transit or signal sequence will transport the protein to a particular intracellular or extracellular destination respectively, and will then be post-translationally removed.

A particular example of such a use concerns directing a protein conferring herbicide resistance, such as a mutant EPSPS protein, to a particular organelle such as the chloroplast rather than to the cytoplasm. This is exemplified by the use of the rbcS transit peptide, the chloroplast transit peptide described in U.S. Patent No. 5,728,925, or the optimized transit peptide described in U.S. Patent No. 5,510,471, which confers plastid-specific targeting of proteins. In addition, it may be desirable to target certain immunoglobulin molecules to the mitochondria, to the extracellular spaces, or to target immunoglobulin molecules to the vacuole.

In certain embodiments, the cellular targeting polypeptide may be a leader peptide sequence to foster secretion of the immunoglobulin molecules from a host cell. In general embodiments, a nucleic acid segment encoding a leader peptide sequence upstream and in reading frame with the coding sequence for a immunoglobulin molecule of the present invention is used for recombinant expression of a immunoglobulin molecule in a host cell. In certain aspects, a leader peptide sequence comprises a signal recognized by a host cell that directs the transport of an expressed immunoglobulin through the outer membrane of a bacterial cell or into the bacterial periplasmic space. In aspects wherein the immunoglobulin molecule is transported into the extracellular medium, e.g., for plant cells or bacterial cells growing *in vitro,* an immunoglobulin molecule may be readily purified from the cells. In some aspects, the leader sequences may be removed by enzymatic cleavage. Such leader peptide sequences and nucleic acids encoding them are known in the art, and non-limiting examples include the secretary leader sequences of *E. coli* alkaline phosphatase (PhoA) OmpA, immunoglobulins, LamB, MalE, outer membrane proteins PelB or penicillinase, StII, T-cell receptors, Lpp, etc.

Such targeting polypeptides may also include, e.g., chlorophyll a/b binding protein transit peptide, small subunit of ribulose bisphosphate carboxylase oxygenase transit peptide, EPSPS transit peptide, dihydrodipocolinic acid synthase transit peptide and murine heavy chain leader sequence or murine light chain leader sequence. Preferably the apoplastic targeting polypeptide is a murine antibody 24 heavy chain signal sequence. More preferably the murine antibody 24 heavy chain signal sequence is encoded by a plant codon optimized nucleic acid molecule. Methods for optimizing a nucleic acid sequence for expression in a particular host are well known in the art.

The targeting peptide may be one which transports the immunoglobulin molecule of this invention to the endoplasmic reticulum (ER) or may be a peptide that causes the immunoglobulin molecule to be retained within the ER after it has been transported there, e.g., KDEL or HDEL. The targeting peptides may be fused to either the N-terminal or the C-terminal end of the immunoglobulin molecule. For example, a signal sequence for transport to the ER may be present on the N-terminal of the immunoglobulin molecule or a signal sequence for retention in the ER may be present on the C-terminal of the immunoglobulin molecule, or *vice versa,* resulting in the accumulation of the molecule in the ER.

Those of skill in the art are aware that many organisms display a translational or transcriptional preference for particular codons. That preference is manifested as more efficient translation of the RNA. Thus it is preferred that the nucleic acid molecules of this invention be codon optimized for expression in either bacterial or plant cells in accordance with Angenon et al., FEBS 271: 144-146 (1990). For example, the codon optimized leader sequence of the murine heavy chain antibody 24 codons optimized for expression in pea, wheat and tobacco may be: ATG GAG TGG AGC TGG ATC TTT CTC TTT CTC CTC TCA GGA ACT GCA GGT GTT CAC TCC (SEQ ID NO: 18) and the codon optimized leader sequence of the murine light chain antibody 24 codon optimized for expression in pea, wheat and tobacco may be : ATG GAC TTT CAA GTG CAG ATT TTC AGC TTC CTC CTC ATC AGC GCC TCA GTT ATC ATC TCT AGG GGA (SEQ ID NO: 19).

The immunoglobulin molecules of this invention may further comprise an epitope "tag" useful for the isolation, purification or detection of the molecule. The tag may be any tag that is routinely used for the detection, purification or isolation of a polypeptide. The immunoglobulin molecules of this invention may be linked to a detectable label. "Detectable labels" are compounds and/or elements that can be detected due to their specific functional properties, and/or chemical characteristics, the use of which allows the antibody to which they are attached to be detected, and/or further quantified if desired. The immunoglobulin molecules may also be linked to an "immunotoxin," a cytotoxic or an anti-cellular agent

The immunoglobulin molecules of this invention may be used as diagnostic agents, e.g., for use in *in vitro* diagnostics, such as in a variety of immunoassays, and/or those for use in *in vivo* diagnostic protocols, generally known as "antibody-directed imaging".

Many appropriate imaging agents are known in the art, as are methods for their attachment to antibodies (see, for e.g., U.S. Patent Nos. 5,1,236; 4,938,948; and 4,472,509). The imaging moieties used can be paramagnetic ions; radioactive isotopes; fluorochromes; NNR-detectable substances; X-ray imaging.

Another type of immunoglobulin molecule contemplated in the present invention are those intended primarily for use *in vitro,* where the immunoglobulin molecule is linked to a secondary binding ligand and/or to an enzyme (an enzyme tag) that will generate a colored product upon contact with a chromogenic substrate. Examples of suitable enzymes include urease, alkaline phosphatase, (horseradish) hydrogen peroxidase or glucose oxidase. Preferred secondary binding ligands are biotin and/or avidin and strcptavidin compounds. The use of such labels is well known to those of skill in the art and are described, for example, in U.S. Patents 3,817,837; 3,850,752; 3,939,350; 3,996,345; 4,277,437; 4,275,149 and 4,366,241. The immunoglobulin molecules may also be attached to magnetic beads or silica beads.

Yet another known method of site-specific attachment of molecules to immunoglobulin molecules of this invention comprises the reaction of immunoglobulins with hapten-based affinity labels. Essentially, hapten-based affinity labels react with amino acid residues in the antigen binding site, thereby destroying this site and blocking specific antigen reaction. However, this may not be advantageous since it results in loss of antigen binding by the immunoglobulin molecule.

Molecules containing azido groups may also be used to form covalent bonds to proteins through reactive nitrene intermediates that are generated by low intensity ultraviolet light (Potter & Haley, "Photoaffinity Labeling of Nucleotide Binding Sites with 8-Azidopurine Analogs: Techniques and Application," Meth. Enzymol., 91, 613-633 (1982)). In particular, 2- and 8-azido analogues of purine nucleotides have been used as site-directed photoprobes to identify nucleotide binding proteins in crude cell extracts (Owens et al., "Characterization of the guanosine-3'-diphosphate-5'-diphosphate binding site on E. coli RNA polymerase using a photoprobe, 8-azidoguanosine-3'-5'-bisphosphate", Biochem. Biophys. Res. Commun. 142:964-971 (1987); Atherton et al., "A Study of Rat Epididymal Sperm Adenosine 3N,5N-Monophohate-Dependent Protein Kinase: Maturation Differences and Cellular Location", Bill of Reproduction, 32, 155-171 (1985). The 2- and δ-azido nucleotides have also been used to map nucleotide binding domains of purified proteins (Khatoon et al., "Aberrant guanosine triphosphate-beta-tubulin interaction in Alzheimer's disease", Ann. Neurol. 26:210-215(1989); King et al., "Structure of the alpha and beta heavy chains of the outer arm dynein from Chlamydomonas flagella. Nucleotide binding site", J. Biol. Chem. 264:10210-10218(1989); and Dholakia et at, J. Biol. Chem., 264:2063 8-20642 (1989)) and may be used as antibody binding agents.

Several methods are know in the art for the attaclunent or conjugation of an immunoglobulin molecule to another moiety. Some attachment methods involve the use of a metal chelate complex and an organic chelating agent such a dieihylenetriamiriepentaacetic acid anhydride (DTPA); ethylenediaminetetraacetic acid; N-chloro-p-toluenesulfonamide; and/or tetrachloro-3-6-diphenylglycouril-3 attached to the antibody (U.S. Patent Nos. 4,472,509 and 4,938,948). Antibodies may also be reacted with an enzyme in the presence of a coupling agent such as glutaraldehyde or periodate. Inununoglobulin conjugates with fluorescein markers are prepared in the presence of these coupling agents or by reaction with an isothiocyante. In U.S. Patent No. 4,938,948, imaging of breast tumors is achieved using monoclonal antibodies and the detectable imaging moieties are bound to the antibody using linkers such as methyl-p-hydroxybenzilnidate or N-succinimidyl-3-(4-hydroxyphenyl)propionate.

Some immunodetection methods include enzyme linked immunosorbent assay (ELISA), radioinununoassay (RIA), immunoradiometric assay, fluoroimmunoassay, chemiluminescent assay, bioluminescent assay, and Western blot. Various useful immunodetection methods are well known in the art.

The polypeptide may also be labeled with a tag that is recognized by an antibody, or a specific organic receptor (e.g., NiNTA), which may be labeled or bound to a solid support. Preferably the epitope tag is a c-myc polypeptide or a his6 polypeptide (Evan et al. Mol. Cell. Biol., 5:3610-3616 (1985) and Mukhija, "High level production and one-step purification of biologically active human growth hormone in Escherichia coli", Gene 165:303-306 (1995)). See, also Goldenberg "Current status of cancer imaging with radiolabeled antibodies, Cancer Res. Clin. Oncol., 113: 203-208 (1987); Carter and Merchant, "Engineering antibodies for imaging and therapy", Curr. Opin. Biotechnol., 8:449-454 (1997), and; Bailey, "Labeling of peptides and proteins by radioiodination", Method Mol. Biol., 32:441-448 (1994) for a general discussion of methods useful for labeling antibody molecules and other polypeptides for identification or isolation purposes.

This invention further relates to nucleic acid molecules which encode the immunoglobulin molecules comprising the framework scaffolds identified herein. Preferably the nucleic acid molecules encode variable domains of both heavy and light chains, e.g., scFv or diabodies, triabodies or tetrabodies. Preferably the isolated nucleic acid molecule of this invention comprise a sequence that encodes a variable domain of a light chain having the sequence set forth in Tables 6 or 8. The nucleic acid molecules may encode the variable heavy or light chain domains, or both, that contain conservative substitutions in the sequences set forth in Table 5 or 8. Most preferred is a nucleic acid molecule that encodes a variable domain of a light chain having the sequence: wherein one or more of the amino acid residues at position 10 in SEQ ID NO: 5 or at position 6 in SEQ ID NO:6 or the amino acid residue at position 10 in SEQ ID NO:8, may be absent and not substituted by another amino acid;
or a variable domain of a heavy chain having the sequence: wherein one or more of the amino acid residues at positions 18, 19 or 20 in SEQ ID NO: 3 may be absent and not substituted by another amino acid;
or both the variable heavy chain domain and the variable light chain domain. Preferably the nucleic acid molecule encodes a scFv antibody molecule consisting of the foregoing amino acid sequences joined via a linker as described *supra.*

The nucleic acid molecule of this invention may comprise a nucleotide sequence encoding a framework region set forth in Table 5 or Table 7, preferably the nucleotide sequences are selected from the group consisting of SEQ ID NO: 22, 23, 24, 25, 27, 28, 29, 30, 31, 32, 33, 34, 36, 37, 38, 39, 40, 41, 42, 43, 45, 46, 47, and 48.

The nucleic acid molecules may further comprise untranslated 5' and 3' sequences which may facilitate the expression or stability of the nucleic acid molecule. The 5' untranslated regions may be, e.g., omega sequence (= 5' untranslated region of TMV) or the 5` untranslated region of the chalcone synthase. The 3' untranslated regions may be, e.g., termination sequence of CaMV or the 3` untranslated region of nopaline synthase.

Another embodiment of this invention are the isolated nucleic acid molecules that encode the immunoglobulin molecules comprising the framework regions of this invention, fused to a cellular targeting polypeptide. Preferably the immunoglobulin molecule is a V_{L}, V_{H} or scFv antibody molecule fused to a cellular targeting polypeptide.

Those of skill in the art appreciate that there is a certain amount of degeneracy in the genetic code (see Table 1) and therefore different nucleotide sequences may encode a particular amino acid sequence. However, once provided with a particular protein sequence it is possible to determine all the possible nucleotide sequence permutations that would encode the given protein sequence. These nucleotide sequences may be determined using, e.g., a commercially available computer program such as DNASIS for Windows Version 2.5 or GCG^{™} (Genetics Computer Group Sequence Analysis Software Package version 8.1 or Wisconsin Sequence Analysis Package). Thus another embodiment of this invention are all the isolated nucleic acid molecules having nucleotide sequences that encode the framework regions, the framework scaffolds, the V_{L} and V_{H} domains and the scFv antibodies of this invention.

As discussed *supra,* those of skill in the art also appreciate that certain nucleotide codons are preferred for expression within plant cells or bacteria and thus a preferred embodiment of this invention are nucleic acid molecules encoding the framework regions, framework scaffolds, V_{L} or V_{H} domains or scFv antibodies of this invention that have been codon optimized for expression in bacterial cells or plant cells, preferably plant cells. Particularly preferred nucleic acid molecules for expression in bacteria, particularly *E. coli,* and those wherein the codons that are used by *E. coli* at a frequency of <1% were replaced. A cut-off of <1% was used to arbitrarily define rare todons (according to: Kane, "Effects of rare codon clusters on high-level expression of heterologous proteins in Escherichia coli", Current Opinion in Biotechnology, 6, 494-500 1995 (Particularily perferred nucleic acid molecules for expression in bacteria are set forth below. New codons are underlined and bold).

Most preferred codon optimized sequence is (underlined and bold codons are optimized for *E. coli* expression):

For expression in plants, particularly dicots, e.g., tobacco, the codon usage was adapted to the codon usage of the tobacco Rubisco, which is the most abundant protein in plant cells: (The preferred nucleic acid for expression in dicots are set forth below. New codons are underlined and bold).

For expression in monocots the optimized sequence would be adjusted to those codons preferably used in monocots.

This invention also relates to a diverse library expressing the immunoglobulin molecules of this invention. Preferably the nucleic acid molecules encode immunoglobulin variable light chain domain, variable heavy chain domain, or scFv antibodies, diabodies, triabodies or tetrabodies. Those of skill in the art appreciate that there are many strategies for producing display libraries, as discussed *supra.*

The libraries may be in the form of phagemid libraries wherein the immunoglobulin molecules comprise essentially identical framework scaffolds as identified herein, but the CDRs are randomized by any technique that is well known in the art (see for example Knappik et al., (2000) and Barbas et al., US Patent No. 6,096,551). Preferably the immunoglobulin molecules are V_{L} domain, V_{H} domain, or scFv antibodies comprising the framework scaffolds identified herein.

The libraries may be screened for molecules having a desirable specificity or affinity for a predetermined antigen and the nucleic acid molecules encoding the immunoglobulin molecules having a desired specificity may be isolated. Methods for screening peptide libraries and isolating molecules having a desired specificity or affinity for a predetermined antigen are known in the art. For example, Hoogenboom Trends Biotechnol., 15: 62-70 (1997) and Hoogenboom et al., Immunotechnology 4:1-20 (1998).

Another aspect of this invention is a vector comprising the isolated nucleic acid molecule encoding the immunoglobulin molecules of this invention. Preferably the nucleic acid molecule encodes a V_{L} domain, V_{H} domain, scFv antibody, diabodies, triabodies or tetrabodies or this invention. Those skilled in the art are well able to construct vectors and design protocols for recombinant gene expression in almost any host background, e.g., bacteria, e.g., *E. coli, Bacilli* such as *B. subtilis, Pseudomonas* species such as *P. aeruginosa, Salmonella typhimurium,* or *Serratia marcescans,* yeast, e.g., *S. cerevisiae,* algae, mammalian cells, e.g., mouse, rat, hamster, monkey, canine cells or human cells, and plant cells, monocotyledonous or dicotyledonous cells. Suitable vectors can be chosen or constructed, containing appropriate regulatory sequences, including promoter sequences, terminator fragments, polyadenylation sequences, enhancer sequences, marker genes and other sequences as appropriate. For further details see, for example, Molecular Cloning: a Laboratory Manual: 2nd edition, Sambrook et al, 1989, Cold Spring Harbor Laboratory Press. Many known techniques and protocols for manipulation of nucleic acid, for example in preparation of nucleic acid constructs, mutagenesis, sequencing, introduction of DNA into cells and gene expression, and analysis of proteins, are described in detail in Current Protocols in Molecular Biology, Second Edition, Ausubel et al. eds., John Wiley & Sons (1992).

Specific procedures and vectors previously used with wide success upon plants are described by Bevau (Nucl. Acids Res. 12, 8711-8721 (1984)) and Guerineau and Mullineaux (Plant transformation and expression vectors. In Plant Molecular Biology Labfax (Croy RRD ed) Oxford, BIOS Scientific Publishers, pp 121-148 (1993)). For example, the vector may be *Agrobacterium tumefaciens,* a plasmid, e.g., pTMZ1 or pSSH1, a phagemid, a cosmid or a viral vector known in the art.

The vector may comprise a promoter in operable linkage with the nucleic acid molecules of this invention. Promoters suitable for expression in a variety of host cells are known, e.g., viral promoters of adenoviruses, herpes viruses, lentiviruses, and retroviruses, including but not limited to, adenovirus 2, avian sarcoma virus, bovine papilloma virus, cytomegalovirus (CMV), hepatitis-B virus, polyoma virus, fowlpox virus, Simian Virus 40 (SV40), Epstein Barr virus (EBV), feline immunedeficiency virus (FIV), and mammalian promoters, e.g., the actin promoter, heat-shock promoters, and immunoglobulin promoters, and functional derivatives thereof, provided such promoters are compatible with the host cell systems. Promoters suitable for use with prokaryotic hosts include, e.g., alkaline phosphatase, A-lactaxnase and lactose promoter systems (Chang et al., Nature, 275: 615 (1978); and Goeddel et al., Nature 281: 544 (1979)), tryptophan (trp) promoter (Goeddel Nucleic Acids Res. 8: 4057 (1980) and EPO Appln. Publ. No. 36,776) and hybrid promoters such as the tac promoter (H. de Boer et al., Proc. Natl. Acad. Sci. USA 80: 21-25 (1983)). Suitable promoters for use with yeast hosts include the promoters for 3-phosphoglycerate kinase (Hitzeman et al., J. Biol. Chem. 255: 2073 (1980)) or other glycolytic enzymes (Hess et al., J. Adv. Enzyme Reg. 7: 149 (1968); and Holland, Biochemistry 1.7: 4900 (1978)), such as enolase, glyceraldehyde-3-phosphate dehydrogenase, hexokinase, pyruvate decarboxylase, phosphofructokinase, glucose-6-phosphale isomerase, 3-phosphoglycerate mutate, pyruvate kinase, triosephosphate isomerase, phosphoglucose isomerase, and glucokinase. Other yeast promoters, which are inducible promoters having the additional advantage of transcription controlled by growth conditions, are the promoters for alcohol dehydrogenase 2, isocyt6chrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in R. Hitzeman et al., European Patent Publication No. 73,657A.

Promoters for plant specific expression are also known to those of skill in the art, and include but are not limited to, any constitutive, inducible, tissue or organ specific, or developmental stage specific promoter which can be expressed in the particular plant cell or at a particular developmental stage and include viral, synthetic, constitutive as described (Poszkowski et al., EMBO J., 3:2719 (1989); Odell et al., Nature, 313:810-812 (1985)), temporally regulated, spatially regulated, and spatio-temporally regulated (Chau et al., Science, 244:174-181 (1989)). Those of skill in the art appreciate that the appropriate promoter will depend on the system being used for expression of the nucleic acid molecules.

Promoters suitable for use in plants include, but are not limited to, at least one regulatory sequence from the T-DNA of *A. tumefaciens,* including mannopine synthase, nopaline synthase, and octopine synthase; alcohol dehydrogenase promoter from corn; light inducible promoters such as ribulose-biphosphate-carboxylase-oxygenase small subunit gene from a variety of species and the major chlorophyll a/b binding protein gene promoter; *Gmhsp 17.3-B* a heat shock inducible promoter from soybean (Schoffl et al., EMBO J., 3:2491 (1984)) *GH3* an auxin-inducible promoter (Hagen et al., Plant Mol. Biol. 17:567 (1991)) also from soybean, and *win6.39b* a wounding-inducible promoter from poplar (Clarke, et al., Plant Mol. Biol. 25:799 (1994)); the maize ubiquitin promoter and intron (US-A-5510474); the wheat low molecular weight glutenin promoter (Colot et al., EMBO J 6: 3559-3564 (1987)) or the glutelin-1 promoter, histone promoters (EP 507 698), actin promoters; maize ubiquitin 1 promoter (Christensen et al., Transgenic Res. 5:213 (1996)); 35S and 19S promoters of cauliflower mosaic virus (Gardner et al., NAR 9: 2871-2888 (1981)); developmentally regulated promoters such as the waxy, zein, or bronze promoters from maize, or rice calmodulin promoters (Choi et al., Mol. Cells, Vol. 6, No. 5: 541-546 (1996)); as well as synthetic or other natural promoters which are either inducible or constitutive, including those promoters exhibiting organ specific expression or expression at specific development stage(s) of the plant, e.g., the alpha-tubulin promoter disclosed in U.S. Pat. No. 5,635,618, and; the soybean SbPRP1 promoter. Preferably the promoter is a tissue constitutive promoter.

The vectors may also comprise a marker gene. "Marker genes" are genes that impart a distinct phenotype to cells expressing the marker gene and thus allow such transformed cells to be distinguished from cells that do not have the marker. Such genes may encode either a selectable or screenable marker, depending on whether the marker confers a trait which one can "select" for by chemical means, *i.e.,* through the use of a selective agent (*e.g.,* a herbicide, antibiotic, or the like), or whether it is simply a trait that one can identify through observation or testing, *i.e*., by "screening"' (*e.g*., the green fluorescent protein). Of course, many examples of suitable marker genes are known to the art and can be employed in the practice of the invention.

Many selectable marker coding regions may be used in present invention including, but not limited to the NPTII gene or *neo* (Potrykus et al., Mol. Gen. Genet., 199:183-188 (1985)), which provide kanamycin resistance and can be selected for using kanamycin, G418, paromomycin, *etc*.; *bar,* which confers bialaphos or phosphinothricin resistance; a mutant EPSP synthase protein (US Patent 4,971,908) conferring glyphosate resistance; a nitrilase such as *bxn* from *Klebsiella ozaenae* which confers resistance to bromoxynil (Stalker, et al., "Herbicide resistance in transgenic plants expressing a bacterial detoxification gene," Science (Washington) 242: 419-423 (1988)); a mutant acetolactate synthase (ALS) which confers resistance to imidazolinone, sulfonylurea or other ALS inhibiting chemicals (European Patent Application 154,204, 1985); a methotrexate resistant DHFR (Thillet et al., "Site-directed mutagenesis of mouse dihydrofolate reductase. Mutants with increased resistance to methotrexate and trimethoprim", J Biol Chem; 263:12500-12508 (1988)), a dalapon dehalogenase that confers resistance to the herbicide dalapon; or a mutated anthranilate synthase that confers resistance to 5-methyl tryptophan. Where a mutant EPSP synthase is employed, additional benefit may be realized through the incorporation of a suitable chloroplast transit peptide, CTP (U.S. Patent No. 5,188,642) or OTP (U.S. Patent No. 5,633,448) and use of a modified maize EPSPS (PCT Application WO 97/04103).

Included within the terms selectable or screenable marker genes also are genes which encode a "secretable marker" whose secretion can be detected as a means of identifying or selecting for transformed cells. Examples include marker genes which encode a secretable antigen that can be identified by antibody interaction, or even secretable enzymes which can be detected by their catalytic activity. Secretable proteins fall into a number of classes, including small, diffusible proteins detectable, e.g., by ELISA; small active enzymes detectable in extracellular solution (e.g., α-amylase, β-lactamase, phosphinothricin acetyltransferase); and proteins that are inserted or trapped in the cell wall (e.g., proteins that include a leader sequence such as that found in the expression unit of extensin or tobacco PR-S).

An illustrative embodiment of selectable markers capable of being used in systems to select transformants are the enzyme phosphinothricin acetyltransferase, such as *bar* from *Streptomyces hygroscopicus* or *pat* from *Streptomyces viridochromogenes.* The enzyme phosphinothricin acetyl transferase (PAT) inactivates the active ingredient in the herbicide bialaphos, phosphinothricin (PPT). PPT inhibits glutamine synthetase, (Murakami et al., Mol. Gen. Genet., 205:42-50, (1986); Twell et al., Plant Physiol 91:1270-1274 (1989)) causing rapid accumulation of ammonia and cell death.

Screenable markers that may be employed include a β-glucuronidase (GUS) or *uid*A gene which encodes an enzyme for which various chromogenic substrates are known; an R-locus gene, which encodes a product that regulates the production of anthocyanin pigments (red color) in plant tissues (Dellaporta et al., In: Chromosome Structure and Function: Impact of New Concepts, 18th Stadler Genetics Symposium, 11:263-282 (1988)); a β-lactamase gene (Sutcliffe, Proc. Natl Acad. Sci. USA, 75:3737-3741(1978)); which encodes an enzyme for which various chromogenic substrates are known (*e.g*., PADAC, a chromogenic cephalosporin); a *xyl*E gene (Zukowsky et al., Proc. Natl Acad. Sci. USA, 80:1101-1105 (1983)) which encodes a catechol dioxygenase that can convert chromogenic catechols; an α-amylase gene (Ikuta et al., Bio/technol., 8:241-242 (1990)); a tyrosinase gene (Katz et al., J. Gen. Microbiol., 129:2703-2714 (1983)) which encodes an enzyme capable of oxidizing tyrosine to DOPA and dopaquinone which in turn condenses to form the easily-detectable compound melanin; a β-galactosidase gene, which encodes an enzyme for which there are chromogenic substrates; a luciferase (*lux*) gene (Ow et al., Science, 234:856-859(1986)), which allows for bioluminescence detection; an aequorin gene (Prasher et al., Biochem. Biophys. Res. Commun., 126(3):1259-1268(1985)) which may be employed in calcium-sensitive bioluminescence detection; or a gene encoding for green fluorescent protein (Sheen et al., Plant Journal, 8(5):777-784(1995); Haseloff et al., Proc. Natl Acad. Sci. USA 94(6):2122-2127(1997); Reichel et al., Proc. Natl Acad. Sci. USA, 93 (12): 5888-5893. (1996); Tian et al., Plant Cell Rep., 16:267-271(1997); WO 97/41228).

Another embodiment of this invention are host cells expressing the immunoglobulin molecules of this invention, preferably a V_{L} domain, a V_{H} domain or an scFv antibody, a diabody, a triabody or a tetrabody, more preferably a scFv antibody. The host cell may be, e.g., bacteria, e.g., *E. coli , Bacilli* such as *B. subtilis, Pseudomonas* species e.g., *P. aeruginosa, Salmonella typhimurium,* or *Serratia marcescans,* yeast, e.g., *Pichia pastoris, S. cerevisiae,* algae, insect cells, mammalian cells, e.g., mouse, rat, hamster, monkey, canine or human cells, and plant cells, monocotyledonous or dicotyledonous cells. Particularly preferred host cells are plant cells expressing the immunoglobulin molecules of this invention. The plant cells may be dicotyledonous, such as, e.g., tobacco, tomato, ornamentals, potato, sugarcane, soybean, cotton, canola, alfalfa and sunflower, or monocotyledonous plant cells, such as, e.g., amaranth, barley, maize, millet, oat, rice, rye, sorghum, tufgrass or wheat cells. More preferably the plant cells are maize, rice, wheat, tobacco, potato, tomato or rapeseed. The immunoglobulin molecules of this invention may accumulate to a concentration of at least about 0.2% to about 30% of the total soluble protein of the host cell. Preferably the immunoglobulin molecules of this invention accumulate to a concentration of at least about 0.4 % to about 30% of the total soluble cellular protein, more preferably the scFv antibodies make up at least about 1.0% to about 15% of the total soluble cellular protein. The scFv antibodies may accuinulate least about 2.0% to 15% of total soluble cellular protein.

Also an aspect of this invention is a transgenic plant and plant part expressing the immunoglobulins of this invention, preferably a scFv antibody. The plant part may be e.g., a leaf, a flower, a root, a stem, a seed or a fruit. Preferably the plant part is a leaf. Preferably the transgenic plant and plant part comprise the immunoglobulin molecule at a concentration of at least about 0.2% to about 30% of total soluble cellular protein. More preferably, the transgenic plants and plant parts comprise immunoglobulin molecule at a concentration of at least about 0.4% to about 30% of total soluble cellular protein. Most preferably the transgenic plants and plant parts comprise immunoglobulin molecule at a concentration of at least about 1.0% to about 15% of total soluble cellular protein. The scFv antibodies may make up at least about 2.0% to 15% of total soluble cellular proteins.

Plants, which include a plant cell according to the invention, are also provided, along with any part or propagules thereof, seed selfed or hybrid progeny and descendants. A plant according to the present invention may be one which does not breed true in one or more properties. Plant varieties may be excluded, particularly registrable plant varieties according to Plant Breeders' Rights. It is noted that a plant need not be considered a "plant variety" simply because it contains stably within its genome a transgene, introduced into a cell of the plant or an ancestor thereof.

Many techniques are available for transforming a host cell, e.g., Van Solingen et al., J. Bact., 130:946 (1977), Hsiao et al., Proc. Nall. Acad. Sci. (USA), 76:3829 (1979), Cheng-Ting et al., PNAS USA, 88:9578-9582 (1991), Fields and Song, Nature, 340:245-246 (1989), and Chevray and Nathans, PNAS USA, 89:5789-5793 (1992), describe general methodologies for the transformation of yeast. Methods for introducing DNA into cells, such as by nuclear microinjection, biolistics, electroporation, bacterial protoplast fusion with intact cells, or polycations, e.g., polybrene, polyornithine, may be used. For various techniques for transforming mammalian cells, see Keown et al., Methods in Enzymology, 185:527-537 (1990) and Mansour et al., Nature, 336:348-352 (1988). Expression in mammalian cells is also described in Sambrook, et al., Molecular Cloning, 2nd Ed., Vol. 3, Chapter 16 (1989). Many techniques are available in the art for producing transgenic plants. See, e.g., various techniques which are already known for the genetic manipulation of plants. DNA can be transformed into plant cells using any suitable technology, such as a disarmed Ti-plasmid vector carried by *Agrobacterium* exploiting its natural gene transfer ability (EP-A-270355, EP-A-0116718, NAR 12(22) 8711 - 87215 (1984)), particle or microprojectile bombardment (US 5100792, EP-A-444882, EP-A-434616) microinjection (WO 92/09696, WO 94/00583, EP 331083, EP 175966, Green et al. (1987) Plant Tissue and Cell Culture, Academic Press), electroporation (EP 290395, WHO 8706614) other forms of direct DNA uptake (DE 4005152, WO 9012096, US 4684611), liposome mediated DNA uptake (e.g., Freeman et al., Plant Cell Physiol. 29: 1353 (1984)), or the silicon carbide fiber mediated transformation (e.g., Kindly, PNAS U.S.A., 87: 1228 (1990)). Physical methods for the transformation of plant cells are reviewed in Oard, Biotech. Adv. 9: 1-11 (1991).

*Agrobacterium* transformation is widely used by those skilled in the art to transform dicotyledonous species. Recently, there has been substantial progress towards the routine production of stable, fertile transgenic plants in almost all economically relevant monocot plants (Toriyama et al., Bio/Technology 6: 1072-1074 (1988); Zhang, et al. (1988) Plant Cell Rep. 7: 379-384; Zhang, et al., Theor Appl Genet 76, 835-840 (1988); Shimamoto et al., Nature 338: 274-276 (1989); Datta et al., Bio/Technology 8: 736-740 (1990); Christou, et al., Bio/Technology 9, 957-962 (1991); Peng, et al., International Rice Research Institute, Manila, Philippines 563-574 (1991); Cao, et al., Plant Cell Rep. 11, 585-591 (1992); Li et al., Plant Cell Rep. 12, 250-255 (1993); Rathore, et al., Plant Molecular Biology 21, 871-884 (1993); Fromm, et al., BiolTechnology 8, 833-839 (1990); Gordon-Kamm, et al., Plant Cell 2, 603-618 (1990); D'Halluin, et al., Plant Cell 4, 1495-1505 (1992); Walters, et al. Plant Molecular Biology 18, 189-200 (1992); Koziel, et al., Biotechnology 11, 194-200 (1993); Vasil, Plant Molecular Biology 25, 925-937 (1994); Weeks, et al. Plant Physiology 102, 1077-1084 (1993); Somers, et al., Bio/Technology 10, 1589-1594 (1992); WO92/14828). In particular, *Agrobacterium* mediated transformation is now emerging as ahighly efficient alternative transformation method in monocots (Hiei et al., The Plant Journal 6,271-282. (1994)).

The generation of fertile transgenic plants has been achieved in the cereals rice, maize, wheat, oat, and barley (reviewed in Shimamoto, Current Opinion in Biotechnology 5, 158-162(1994); Vasil et al., Bio/Technology 10, 667-674 (1992); Vain et al., Biotechnology Advances 13 (4): 653-671 (1995); Vasil, Nature Biotechnology 14:702 1996)(all incorporated herein by reference).

Microprojectile bombardment, electroporation and direct DNA uptake are preferred where *Agrobacterium* is inefficient, ineffective or undesireable, e.g., for transforming plants destined for human or animal consumption. Alternatively, a combination of different techniques may be employed to enhance the efficiency of the transformation process, e.g., bombardment with *Agrobacterium,* coated microparticles (EP-A-486234) or microprojectile bombardment to induce wounding followed by co-cultivation with *Agrobacterium* (EP-A-486233).

Following transformation, a plant may be regenerated, e.g., from single cells, callus tissue, leaf discs, immature or mature embryos, as is standard in the art. Almost any plant can be entirely regenerated from cells, tissues and organs of the plant. Available techniques are reviewed in Vasil et al., Cell Culture and Somatic Cell Genetics of Plants, Vol I, II and III, Laboratory Procedures and Their Applications, Academic Press, 1984, and Weissbach and Weissbach, Methods for Plant Molecular Biology, Academic Press, 1989

The particular choice of a transformation technology will be determined by its efficiency to transform the selected host cells as well as the experience and preference of the person practicing the invention with a particular methodology of choice. It will be apparent to the skilled person that the particular choice of a transformation system to introduce nucleic acid into host cells is not essential to or a limitation of the invention, nor is the choice of technique for plant regeneration essential to or a limitation of the invention.

A further aspect of the present invention provides a method of making a host cells, e.g., prokaryotic or eukaryotic, e.g., avian, mammalian or plant cells, as disclosed involving introduction of a suitable vector including the relevant expression cassette comprising a nucleic acid molecule encoding the immunoglobulin molecule of this invention into a host cell and causing or allowing recombination between the vector and the host cell genome to introduce the sequence of nucleotides into the genome. The invention extends to plant cells containing nucleic acid molecules encoding the immunoglobulin molecules of this invention as a result of introduction of the nucleic acid molecule into an ancestral cell.

The term "heterologous" may be used to indicate that the gene/sequence of nucleotides in question have been introduced into said cells of the plant or an ancestor thereof, using genetic engineering, i.e. by human intervention. A transgenic plant cell, i.e. transgenic for the nucleic acid in question, is provided. The transgene may be on an extra-genomic vector, such as a cow-pea mosaic viral vector, or incorporated, preferably stably, into the genome. Preferable the transgene is incorporated stably into the genome.

Following transformation of a plant cell, a plant may be regenerated from the cell or descendants thereof using methods known in the art.

Transgenic plants in accordance with the present invention may be cultivated under conditions in which the desired product is produced in cells and/or seed of the plant. Cells producing the product may be in an edible part of the plant, such as leaves or fruit tubers or roots. Seed may be stored, e.g., for at least six months without the immunoglobulin molecule significantly losing its affinity or specificity to the antigen.

The scFv antibodies of this invention may be isolated from the transformed host cells, regenerated transformed plants or plant parts. Many suitable techniques are available in the art for purifying proteins from host cells, prokaryotic or eukaryotic, e.g., avian, mammalian or plant cells. Once isolated the product may be used as desired, for instance in formulation of a composition including at least one additional component.

A framework scaffold of an immunoglobulin molecule may be identified, wherein the framework scaffold comprises framework regions 1-8 (4 framework regions in each of the light and heavy chains) and provides for high level accumulation of the immunoglobulin molecule in plant cells.

A semi-synthetic library may be based on the identified scaffold. The semi-synthetic library can be used to identify and isolate antibodies specific for predetermined antigens. The isolated antibodies may be expressed in plant cells. One embodiment comprises:
(a) introducing isolated nucleic acid molecules that encode immunoglobulin molecules into a plant cell wherein the nucleic acid molecules are in operable linkage with a promoter to generate transformed plant cells, then
(b) assaying the transformed plant cells for expression of the immunoglobulin molecule,
(c) identifying high producer transformed plant cells, wherein the high producers produce immunoglobulin molecules at a concentration of at least 0.2 % of the total soluble cellular protein;
(d) isolating nucleic acid molecules encoding the immunoglobulin molecules from the high producer transformed plant cells;
(e) determining the sequence of immunoglobulin-encoding nucleic acid molecules of the high producer transformed plant cells and deducing the amino acid sequence by any method known in the art;
(f) comparing the amino acid sequences of the immunoglobulin molecules and determining the constant framework regions and complementarity determining regions of the immunoglobulin molecules in accordance with Kabat et al., (Kabat et al., Sequences of Proteins of Immunological Interest 5th Edition NID (1991); Martin, "Accessing the Kabat Antibody Sequence Database by Computer." PROTEINS: Structure, Function and Genetics 25: 130-133(1996),
(g) determining the variability of the common amino acid residues at each position in the FR and CDRs to generate consensus FRs, which encompass the natural diversity of the FR amino acid sequence,
(h) determining the length variation of each CDR,
(i) preparing a semi synthetic library of DNA molecules that encode a framework scaffold, which comprises one or more of the identified consensus FRs of (g), and CDRs, which encompass the natural diversity in CDR length and amino acid residue composition,
wherein the CDRs in (i) could be the same or different than the CDRs in (g), Preferably the nucleic acid molecules that encode the immunoglobulin molecules are prepared from avian mRNA and encode avian antibodies or avian antibody domains, more preferably chicken antibodies or chicken antibody domains. Preferably the immunoglobulin molecule is a V_{L} domain, a V_{H} domain, a scFv antibody, a diabody, a triabody, or a tetrabody. (The numbering of the CDRs and FRs in the immunoglobulin molecules is in accordance with Kabat et al. (Sequences of Proteins of Immunological Interest 5th Edition NID (1991); Martin, "Accessing the Kabat Antibody Sequence Database by Computer." PROTEINS: Structure, Function and Genetics 25: 130-133(1996)).

If the nucleic acid molecules introduced into the plants in step (a) are already isolated or known, step (d) may be omitted. In one embodiment, mRNA is converted into cDNA encoding an immunoglobulin molecule, either a full-size immunoglobulin or an immunoglobulin domain, which may then be expressed in a host cell that is other than a plant cell, e.g., prokaryotic cells, e.g., a bacterial cell (e.g., E. coli, B. thuringis), other eukaryotic cells e.g., yeast cell, algae or mammalian cells, e.g., camelid, mouse, hamster (CHO cells), monkey (COS cells) or human cells, to identify DNA clones that express immunoglobulins at high levels, about at least 0.2% of TSP. Once the high producers are identified, this subset of DNA clones may be used to transform plant cells to identify those that also produce high levels of the immunoglobulin molecules in plant cells. The consensus FRs may be used to generate a variable domain "master gene" comprising 4 consensus framework regions and 3 CDRs for each light chain variable domain or heavy chain variable domain or a scFv master gene comprising 4 consensus heavy chain framework regions and 4 consensus light chain framework regions, 3 heavy chain CDRs and 3 light chain CDRs. A semi synthetic library may be prepared by randomizing the CDRs by using any method known in the art, see, e.g., Knappik et al. (JMB, 296:57-86 (2000)).

The (semi)-synthetic antibody library may be screened for immunoglobulin molecules which bind to predetermined antigens. The nucleic acid molecules which encode such immunoglobulin molecules may then be used to transform plant cells. The predetermined antigen may be, for example, a plant pathogen e.g., a virus, bacteria, fungus, insect or nematode, or a subpart of a plant pathogen, e.g., a outer surface protein, an enzyme or a membrane protein.

The chicken scFv scaffold described in the present invention, particularly the scaffold disclosed in Table 7, supplies a superior framework for rational grafting of CDRs derived from donor antibodies, e.g., avian, piscean, or mammalian, e.g., camelid, murine or human antibodies. The chicken FRs of the present invention provide for stability and high levels of protein accumulation in plant cells and bacteria, as well as in yeast, human or animal cells. The CDRs of donor antibodies, e.g., avian, piscean, mammalian, e.g., camelid, murine, or human, are grafted onto the scaffold of the present invention by means of rational protein design and provide the required affinities and binding kinetics to any given target molecule. Thus, another embodiment of the present invention is a method to generate scFv antibodies with chimeric variable domains e.g., chimeric chicken-murine or chicken-human variable domains. The method herein described makes use of the FRs of the chicken antibody scaffold described in the present invention and the CDRs of the donor antibodies, e.g., murine or human donor antibodies. Once generated the scFv antibodies with chimeric variable domains may be evaluated for their expression levels. In an aspect of this invention the stability and affinity of the scFv antibodies with chimeric variable domains can be altered by substituting one or more amino acid residues within the chicken framework regions with the amino acid residue in the corresponding position in the donor antibody wherein the position is required for maintaining proper CDR function in binding to antigen. The method comprises, e.g.,:
a) identifying suitable donors antibodies for the construction of the scFv antibodies with chimeric variable domains,
b) aligning the amino acid sequences of the variable heavy or light chains of the donor antibodies with those of the chicken framework regions of the present invention to evaluate the percentage of amino acid residue identity of the light and heavy chain,
c) comparing the amino acid residue present in corresponding positions in the donor antibodies and the chicken framework regions wherein the which are:
   - highly conserved in the existing antibody repertoire (Kabat et al. 1991)
   - that are part of the conformational loops of the three heavy and three light chain CDRs
   - involved in the three-dimensional conformations (known as canonical structures) of the six CDRs
   - located at the interface of the variable heavy and light chain and more precisely at the core of the heavy and light chain interface
   - located at the "Vernier zone" well known to those skilled in the art,
d) based on the results of the analysis above, generate an amino acid sequence of a variable domain comprising the chicken framework regions wherein one or more amino acid residues in the framework regions are replaced with the amino acid residue present in corresponding position in the murine donor antibody
e) designing appropriate primers to splice nucleic acid molecules encoding the heavy and light chain chicken frameworks to nucleic acid molecules encoding the heavy and light chain CDRs of the donors,
f) assembling a nucleic acid molecule encoding a scFv antibody having chimeric variable domains by PCR and standard cloning techniques using the primers of (e),
g) sub-cloning the nucleic acid molecule encoding the scFv antibody having chimeric variable domains into an expression vector suitable for expression in a host cell, e.g., a plant, bacteria, yeast, insect, mammalian, e.g., camelid, murine or human, avian, e.g., chicken, or pisces cell, and transforming a suitable host cell,
h) expressing and purifying the scFv having chimeric variable domains from the transformed host cell, e.g., yeast cells, mammalian cells, plant cells and more preferably the cytosol of plant cells, bacteria and more preferably the bacterial periplasm or cytosol,
i) evaluating the expression level and the binding properties of the scFv through, e.g., immunoblot analysis, ELISA formats, surface plasmon resonance and/or other methods known to those skilled in the art, where the parental chicken scFv antibody and the donor antibodies, e.g., the avian, piscean or mammalian, e.g., camelid, murine or human, antibodies are used for comparison, and if desired,
j) altering the stability and the affinity of the scFv antibody having chimeric variable domains, if necessary, by re-iterate mutagenesis of single amino acid residues of chicken origin with those of the donor antibody, e.g., the avian, piscean, mammalian, e.g., camelid, murine or human, donor antibody, at key positions within the FRs of the chicken scaffold according to the analysis described in (c).

### EXAMPLE 1 - EXPRESSION, PURIFICATION AND ANALYSIS OF RECOMBINANT PROTEINS

### A. Expression and purification of recombinant proteins used as antigen

### 1. Expression and purification of GST fusion proteins in pGEX-5X-3 vector

A single colony of *E. coli* strain BL21 (Invitrogen, Leek, the Netherlands) harboring recombinant pGEX-5x-3 plasmid DNA (Pharmacia Biotech) containing a full-length NSM (putative movement protein of Tomato Spotted Wilt Virus) cDNA or parts of the NSM fused to the GST sequences in the plasmid was inoculated in 10 ml of LB medium containing 2% (w/v) glucose and 100 µg/ml ampicillin (LBGA) and cultivated o/n at 37°C. Five milliliters of the overnight (o/n) culture was transferred into 250 ml of LBGA medium and cultured at 37°C to OD₆₀₀ₙₘ of 0.6-1.0. Cells were collected by centrifugation (5,000g/4°C/l0 min), resuspended in 250 ml of LB medium containing 100 µg/ml ampicillin (LBA) and cultured for 30 min at 37°C. The culture was induced for 1-3 h at 30°C by addition of IPTG to a final concentration of 0.2-1.0 mM. Cells were harvested by centrifugation (5,000g/4°C/l0 min) and resuspended in 10 ml of cold PBS buffer containing 5 mM DTT, 1 mM EDTA and 1 mM Pefa-block (Boehringer Mannheim).

Cells were disrupted by sonication (120 W, 3 x 1 min with 4-min interval) on ice. Triton X-100 was added to the sonication solution to a final concentration of 1% (v/v) and mixed. The mixture was incubated on ice for 30 min, followed by centrifugation (12,000g/4°C/30 min). The clarified supernatant was used for batch affinity purification on glutathione agarose according to the manufacturer's instructions (Pharmacia). Briefly, the supernatant was mixed with 1 ml of pre-equilibrated GST matrix with PBS buffer and incubated o/n at 4°C. The matrix was centrifuged (500g/RT/5 min) and washed three times by centrifugation (500g/RT/5 min) with 10 ml cold PBS. Bound GST fusion proteins (GST fusions were produced from fusion of full-length NS_{M} cDNA or parts of NS_{M} gene were fused in-frame with the GST encoding sequences in the pGEX-5x-3 vector from Pharmacia Biotech) were eluted three times with 1 ml of elution buffer by incubation at RT for 10 min followed by centrifugation. The eluates were dialyzed against PBS buffer to remove the glutathione.

### 2. Expression and purification of full-length NS_{M} protein in pTYB4 vector

A single colony of *E. coli* strain ER2566 harboring the recombinant pTYB4 plasmid (New England Biolabs) comprising an NS_{M} cDNA, fused to sequences encoding intein and a chitin binding domain (CBD) to produce an NS_{M} -intein-CBD fusion protein was inoculated in 5 ml of LB medium containing 2% (w/v) glucose and 100 µg/ml ampicillin (LBGA) and cultivated o/n at 37°C. Two milliliters of an o/n culture was transferred into 300 ml of LBGA medium and cultured at 37°C to an OD₆₀₀ₙₘ of 0.6-0.7. Cells were collected by centrifugation (5,000g/4°C/10 min), resuspended in 300 ml of LB medium containing 100 µg/ml ampicillin (LBA) and cultured for 30 min at 37°C. Expression of NS_{M} domain-CBD fusion proteins was induced with 0.5 mM IPTG at 30°C for 3 h. Cells were harvested by centrifugation (5,000g/4°C/10 min) and resuspended in 25 ml of cold cell lysis buffer (CLB).

Cells were disrupted by sonication as above. The clarified bacterial supernatant was subjected to batch affinity purification with chitin beads (New England Biolabs) as follows: Chitin beads were equilibrated three times with cold CLB and 2 ml (bed volume) of bead slurry was mixed with 25 ml of supernatant on a head-over-tail rotator (4 rpm/20°C/30 min). The chitin beads were collected by centrifugation (200g/4°C/5 min) and washed three times with cold CLB and twice with cleavage buffer (CB). NS_{M} CBD fusion proteins (i.e., d1-, d2-and d3-intein-CBD fusion proteins in this study) were directly released from the beads using a stripping buffer (SB) followed by dialysis against PBS to remove SDS, or, the fused partner (i.e., full-length NS_{M} protein in this study) was released prior to the stripping step as follows: After addition of 2 ml of CB, chitin beads were resuspended, transferred to a column and kept at 4°C. Intein-mediated self-cleavage in the presence of 50 mM DTT was carried out on the column at 4°C for up to 6 days or at 4°C for 16 h in a 12-ml falcon tube with gentle mixing on a head-over-tail rotator (4 rpm). Cleaved proteins were eluted three times with 2 ml of CB followed by the stripping step as above.

| | | |
|---|---|---|
| Cell Lysis Buffer (CLB): | Tris-HCl, pH 8.0 | 20 mM |
| | NaCl | 500 mM |
| | EDTA | 0.1 mM |
| Cleavage Buffer (CB): | Tris-HCl, pH8.0 | 20 mM |
| | NaCl | 50 mM |
| | EDTA | 0.1 mM |
| | DTT | 50 mM |
| Stripping Buffer (SB): | Tris-HCl, pH 8.0 | 20 mM |
| | NaCl | 500 mM |
| | SDS | 1% (w/v) |

### B. Analysis of GST fusion proteins

DNA sequencing revealed that the modified d1, d2 and d3 NS_{M} gene sequences were in frame with the C-terminus of GST between the *Nco*I and *Xho*I sites of the *E. coli* expression pGEX-5X-3 vector (Pharmacia Biotech). The resulting recombinant plasmid DNAs were transformed separately into *E. coli* strain BL21 and expression was induced by IPTG. Affinity purified GST fusion proteins showed high purity the yields of purified GST-d1, -d2 and -d3 fusion proteins were 5.7, 0.2 and 8.8 mg per litre culture medium, respectively. GST-d1 and d2 showed the predicted sizes (33.0 and 32.5 kDa, respectively) on SDS-PAGE, whereas GST-d3 fusion migrated more slowly than expected according to the calculated molecular weight (30.1 kDa). Occasionally, degradation was observed in GST-d1 fusion proteins.

In addition to the three NS_{M} domains, the full-size NS_{M} cDNA was also cloned between the *Nco*I and *Xho*I restriction sites of pGEX-5X-3 and expressed in *E. coli* strain BL21. After affinity purification, the GST-NS_{M} fusion was analyzed by SDS-PAGE. Most of GST-NS_{M} fusions were truncated and only about 5% of the fusion protein showed expected size (61.5 kDa). This large proportion of truncated products is likely to be the result of the high level of rare codons present in the NS_{M} sequence, causing premature termination in protein translation. Because the GST affinity purification tag is located at the N-terminus, truncated GST-NS_{M} fusion proteins will be affinity purified. Immunoblot analysis indicated that all GST fusion proteins reacted with GST monoclonal antibody G1 and G4, confirming their identity as GST fusions. Similarly, immunoblot analysis using NS_{M} specific polyclonal antibodies revealed that GST-d1, -d3 and truncated GST-NS_{M} fusions were recognized by the rabbit polyclonal antibodies raised against native NS_{M} protein. This result indicates that all these GST fusion proteins carried sequences derived from the NS_{M} protein. GST-d2 fusion protein gave a very weak signal on immunoblot using the rabbit polyclonal antibodies against native NS_{M} protein, although the identical amount of protein was loaded onto the SDS-PAGE gel when compared to the other samples. This result may reflect either the lower quantities of d2-specific antibodies present in the rabbit antisera or conformational changes of this domain while linked to GST. The three purified GST-domain and GST-NS_{M} fusions were used as antigens for immunization of chicken and phage-displayed antibody selection.

### C. Analysis of Full-length NS_{M} protein

Because of the premature translation termination observed for the GST-NS_{M} fusion protein, the IMPACT system was evaluated for the expression of full-length NS_{M} protein. The NS_{M} cDNA was cloned between the *Nco*I and *Xho*I sites of the pTYB4 expression vector and the recombinant DNA was transformed into the *E. coli* strain ER 2566. NS_{M}-intein-CBD fusion proteins were expressed under the control of T7 promoter upon IPTG induction and expressed fusion proteins were batch purified on chitin beads. NS_{M} protein was subsequently released from the intein-CBD carrier by intein-mediated self-cleavage in the presence of DTT.

NS_{M} expression was dependent on both the time and temperature of induction. Induction at 15°C for 8 h gave the highest yield (2.5 mg/litre culture medium) among the conditions used.

Intein-mediated self-cleavage of affinity purified fusion proteins in the presence of 50 mM of DTT was carried out at 4°C either for 6 days on column or for 16 h in a 12=ml Falcon tube with gentle mixing, bound proteins were eluted three times with 2 ml of cleavage buffer (CB). The chitin matrix was stripped three times with 2 ml of stripping buffer (SB) containing 1% SDS. SDS-PAGE analysis indicated that the majority of NS_{M} was released into the stripping buffer together with the carrier protein. To solubilize the NS_{M} protein, seven different elution buffers varying in salt concentration (50 mM, 500 mM or 1 M NaCl) or detergents (0.1% Triton X-100 and / or 0.1% Tween-20) were used. Only in one case were low levels of NS_{M} released in an elution buffer containing 20 mM Tris-HCl (pH 8.0), 1 M NaCl, 0.1 mM EDTA, 50 mM DTT, 0.1% Triton X-100 and 0.1% Tween 20. This may be because NS_{M} is highly hydrophobic and solubilized only by addition of SDS either in the CB or SB.

Immunoblot analysis indicated that the bacterially expressed NS_{M} using IMPACT system was specifically recognized by NS_{M} specific antisera and had the predicted molecular size (33.5 kDa) confirming the identity of full-length NS_{M}. The purified NS_{M} protein was used for phage-displayed antibody selection, using as PVDF membrane as a support and for characterization of monoclonal antibody phage clones.

### D. Immunization of animals and determination of antibody titer

The treatment and maintenance of laboratory animals was approved by the "Regierungsprasidium des Landes NRW" (RP-Nr.: 23.203.2 AC 12, 21/95) and supervised by Dr. Hirsch who is responsible for biological safety at the Insitute of Biologie I, RWTH Aachen.

### 1. Immunization of chicken

"White leghorn" chickens (*Gallus domesticus)* were intramuscularly injected with 120 µg of the mixture of GST-d1, -d2 and -d3 fusion proteins (40 µg each) in complete Freund's adjuvant. Two further injections were given at 4 weeks intervals in incomplete Freund's adjuvant and a final boost performed 4 days prior to sacrifice with antigen dissolved in PBS.

### EXAMPLE 2 - CONSTRUCTION OF PHAGEMID-scFv LIBRARIES

### A. Construction of phage-displayed scFv libraries

### 1. Isolation of total RNA and mRNA of spleen cells

Spleens from immunized chickens were removed and the splenocytes collected by disrupting the spleens in ice-cold PBS. The cells were washed with PBS and total RNA and mRNA isolated from 10⁸ cells using the "RNeasy Midi kit" or "Oligotex™ mRNA kit" (Quiagen).

Total RNA and mRNA were isolated from spleen cells of immunized chickens. Agarose gel analysis showed good integrity of the isolated chicken RNA.

From 10⁸ spleen cells, 128 µg total RNA was isolated from chicken resulting in 1500 ng. Isolated chicken mRNA was used for subsequent construction of phagemid-scFv libraries.

### 2. Construction of scFv libraries

First-strand cDNA was synthesized in two separate tubes using the "SuperScript preamplification system (for first strand cDNA synthesis)" kit and the V_{H}-cDNA and V_{L}-cDNA primers that bind to the constant region of chicken heavy chain and λ chain, respectively. Each reaction contained 150 ng of spleen mRNA. Variable regions were amplified by PCR using two specific sets of primers binding to the framework region 1 and 4: chicV_{H}5' (SEQ ID NO: 11) and chicV_{H}3' (SEQ ID NO: 12), chicV_{L}5' (SEQ ID NO: 13) and chicV_{L}3' (SEQ ID NO: 14). PCRs were carried out in a total volume of 50 µl by adding 10 pmol of each primer and one fifth of the first strand cDNA reaction under the following conditions: an initial denaturation for 5 min at 94°C followed by 30 cycles at 94°C for 1 min, 55°C (53°C for V_{H}) for 2 min, 72°C for 2 min and a filling cycle of 72°C for 10 min. V_{H} and V_{L} PCR products with expected size were obtained and used for subsequent cloning.

PCR amplified heavy (V_{H}) and light (V_{L}) chain domains were gel purified using a "QIAquick gel extraction kit" (Quiagen) and digested with *Sfi*I and *Bst*EII (V_{H}), or *Asc*I and *Not*I (V_{L}) enzymes, respectively. The pHEN4II phagemid DNA was digested with *Sfi*I and *Bst*EII or *AscI* and *Not*I and gel purified. 75 ng of purified vector pHEN4II were ligated with a fivefold molar excess of purified V_{H} fragments and the ligation products electroporated into electrocompetent *E. coli* XL1-blue cells to create a V_{H} library. The V_{L} library was separately constructed in parallel resulting in a V_{H} library containing 1x10⁴ independent clones harboring inserts with a size of 380 bp. A V_{L} library constructed in parallel had 3x10³ independent clones containing inserts with a size of 340 bp. Two scFv libraries designated (pHEN4II+LH) and (pHEN4II+HL) were constructed by recovering V_{H} fragments from the V_{H} library and cloning into the V_{L} library or *vice versa.*

### 3. Construction of phage-display scFv libraries

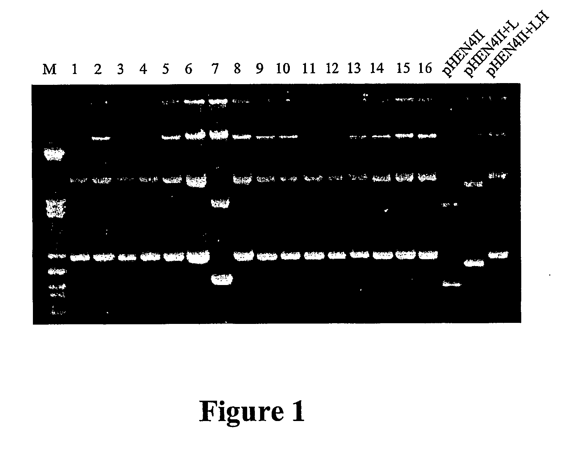

To create scFv libraries with high diversity, two scFv libraries were constructed by recovering V_{H} fragments from the V_{H} library and cloning into the V_{L} library or *vice versa* to generate the pHEN4II+LH and pHEN4II+HL scFv libraries. The percentage of positive clones in these two final libraries was 92% (Fig. 1) and 50%, resulting in 2x10⁵ and 1x10⁶ independent clones respectively. These two libraries were used for biopanning with the selected antigen.

Fig. 1 displays an analysis of phagemid-scFv DNA on agarose gels. Undigested plasmid DNA was prepared and separated on a 1.2% agarose gel. M: *Pst*I digested λDNA; 1-16: plasmid DNA of 16 independent clones from the pHEN4II+LH library; pHEN4II: phagemid control; pHEN4II+L: pHEN4II phagemid containing a V_{L} region; pHEN4II+LH: pHEN4II phagemid containing an scFv gene.

### EXAMPLE 3 - SELECTION AND CHARACTERIZATION OF SPECIFIC PHAGE CLONES

### A. Phage-display antibody selection

### 1. Biotinylation of antigens

Affinity purified GST-domain (d1), GST-domain (d2), and GST-domain (d3) fusion proteins were reversibly biotinylated separately using biotin disulfide N-hydroxysuccinimide ester (Sigma, St. Louis, Missouri, USA). 0.5 mg of GST-domain fusion protein in 2.5 ml of PBS was mixed with 0.25 ml of 1 M NaHCO₃ (pH 8.6) and a 15-20 molar excess of biotin solution (2 mg/ml in DMSO) added at room temperature for 30 min. The reaction was stopped by adding 0.125 ml of 2 M glycine. Free biotinylation reagent was removed by dialysis against PBS at 4°C overnight.

### 2. Determination of biotinylation efficiency by dot blot

Biotinylation efficiency of the GST-fusion protein was determined by dot-blot using the same non-biotinylated fusion protein as a standard (Hawkins et al., Selection of phage antibodies by binding affinity. Mimicking affinity maturation. J Mol Biol 226: 889-896(1992.). Dialyzed biotinylated GST-domain fusion protein was adsorbed with increasing amount of avidin-beads. After removal of protein-biotin-avidin beads complex, the solution was loaded onto an Immobilon membrane assembled in a dot-blot cell. Non-biotinylated GST-domain fusion proteins were serially diluted and blotted onto the membrane. After blocking with 2% (w/v) MPBS at RT for 1 h, the membrane was incubated with a 1:10,000 fold diluted GST-specific monoclonal antibody at RT for 2 h. Bound antibody was detected by 1:5,000 diluted AP-labeled goat anti-mouse polyclonal antibodies and NBT/BCIP substrate.

### 3. Solution-phase panning with biotinylated antigens

Two phage libraries (pHEN4II+LH) and (pHEN4II+HL) were prepared from initial transformations upon infection with the replication defective helper phage M13KO7, as described (Clackson et al., "Making antibody fragments using phage display libraries." Nature, 352(6336): 624-628(1991). incorporated herein by reference). The phage titers were determined by the addition of dilutions to exponentially growing *E*. *coli* XL1-blue. The biotinylated GST-d1, -d2 and -d3 fusions were used to separately pan the libraries in solution. To exclude GST-specific phage clones, 10¹² colony-forming units (cfu) from each library were preincubated with 50 mM GST at RT for 30 min. 50 nM biotinylated GST fusion protein (20 nM and 10 nM for the subsequent two rounds of panning) was added to the reaction system and the mixture was incubated at RT for 2 h on a head-over-tail rotator. Phage binding to biotinylated proteins were separated from the phage library using streptavidin-M280-Dynabeads and a magnetic separator. The beads were washed 20 times with 1 ml of PBS containing 0.1% (v/v) Tween-20 and another 20 times with 1 ml of PBS. Bound phage were eluted by incubation at RT for 5 min with 100 µl of 50 mM 1,4-dithiotheitol (DTT). *E. coli* XL1-blue cells infected with eluted phage were plated onto 2xYT agar containing 2% (w/v) glucose and 100 µg/ml ampicillin (2xYTGA-agar) and incubated o/n at 37°C. The titer of infectious phage was determined by the addition of dilutions to exponentially growing *E. coli* XL1-blue. Cells were scraped off the agar by adding 5 ml of 2xYTGA medium and a new phage library was prepared for the next round of selection.

The phage-displayed scFv library pHEN4II+LH and pHEN4II+HL were panned three times against biotinylated GST-NS_{M}, GST-d1, -d2 and -d3 fusion proteins. For each round of panning, 10¹² cfu of recombinant phage were incubated with 50 mM of bacterially expressed GST prior to panning with each biotinylated antigen in solution to remove GST-specific phage clones. Enrichment was observed after the second round of panning. However, the third round of panning led to a decrease of phage titer except for the mixture of three GST-domain fusion proteins. The decreased phage titers after the 3^{rd} round of panning could be related to the decreased concentration of antigens used in the 3^{rd} round of panning. There were no significant differences in phage titer between the two libraries after each round of panning.

### 4. Solid-phase panning using PVDF membrane as a support

Full-length NS_{M} protein obtained from IMPACT expression system was used to pan two phage libraries (pHEN4II+LH) and (pHEN4II+HL) using a PVDF membrane as a solid support. The mixture of NS_{M} and intein-CBD proteins in stripping buffer was resolved on a SDS-PAGE and then blotted onto a PVDF membrane. The membrane was transiently stained with Ponceau S solution and the NS_{M} band excised. Approximately 2 µg of NS_{M} protein per band was blotted. Following blocking with 5% MPBS (w/v) at RT for 1 h, five pieces of membrane (10 µg of NS_{M} in total) were incubated with 1x 10¹³ cfu of phage (equal mixture of two phage libraries) at RT for 1 h and at 4°C o/n with gentle shaking. After extensive washing (10 times with PBST and 10 times with PBS), bound phage were cleaved either by acidic solution (100 mM, glycine/HCl, pH 2.2) followed by neutralization with 1 M, Tris-HCl buffer (pH 7.5) or by bacterial elution by adding log-phase TG1 cells at 37°C for 30 min. The rescued phage were titered and used to prepare phage library for the next round of panning.

### B. Characterization of phage displayed scFv (Phage ELISA)

Monoclonal phage stocks were prepared in microtiter plates according to the Recombinant Phage Antibody System protocol (RPAS; Pharmacia) and used for indirect and capture phage ELISA.

### 1. Indirect phage ELISA

Cross-reactivity of phage antibodies was analyzed by indirect phage ELISA. GST-d1 protein was coated on a microtiter plate and used for screening of monoclonal phage isolated from GST-d2 and GST-d3 panned library or GST-d2 protein was coated and used for screening of monoclonal phage isolated from GST-d1 and GST-d3 panned library.

Individual microplate wells were coated with affinity purified GST-domain fusions or GST as control at 37°C for 2 h. Wells omitting antigens were used as negative controls for each analyzed phage clone. All microplate wells were then blocked o/n with MPBS at 4°C. 50 µl of monoclonal phage (approx. 10⁷ cfu) from the second and the third round of library panning were preincubated with 50 µl of MPBS at RT for 30 min and then transferred into the plate followed by incubation at 37°C for 2 h. The remaining steps for the ELISA were carried out as described in the manufacturers' instructions, incorporated herein by reference (RPAS, Pharmacia) using HRP-conjugated IgG raised against M13 bacteriophage and ABTS as substrate. The OD₄₁₀ₙₘ was measured after incubation for 30 min in the presence of the substrate. For each phage clone, measurements were performed in triplicate.

### 2. Capture phage ELISA

To characterize phage clones after the second and the third rounds of panning, monoclonal phage were analyzed by phage ELISA. Microplate wells were coated with 1 µg/ml of rabbit polyclonal antibodies in MPBS raised against GST and blocked with MPBS. Following addition of 1 µg/ml of GST-domain fusion proteins, GST or MPBS, monoclonal phage were applied (about 10⁷ cfu) to the microplate. The remaining steps were the same as described for indirect phage ELISA and measurements were performed in triplicate.

40 monoclonal phages from the pHEN4II+LH library and 50 from the pHEN4II+HL library isolated after 2-3 rounds of panning with GST-d1 fusion protein were randomly selected and analyzed by indirect phage ELISA. 50% showed specific binding to GST-d1 but not GST. After the third round of panning against GST-d2, 20 monoclonal phages from the pHEN4II+LH library and 20 from the pHEN4II+HL library were screened by indirect phage ELISA and 48% were GST-d2 specific. Indirect phage ELISA performed with the phage clones from the library panned with GST-d3, however, gave no signal even when high concentrations (10 µg/ml) of GST-d3 fusion protein were coated on ELISA plates. To determine why there was no detection of antibody binding for the GST-d3 fusion protein, a different phage ELISA assay, capture phage ELISA, was used to analyze 40 phage clones from the pBEN4II+LH library and 40 from the pHEN4II+HL library after the third round of panning against GST-d3.

All previously isolated NS_{M} domain specific clones identified by indirect phage ELISA reacted with GST-d1, GST-d2 fusion proteins and GST alone, and the signals from fusion proteins or GST by capture phage ELISA were higher than those revealed by indirect phage ELISA.

### 3. Sequence analysis of selected phage clones

To analyze the efficiency of enrichment after panning, 68 clones were randomly selected and sequenced from panned libraries. After three rounds of panning, a significant enrichment was obtained, as shown from the sequence analyses of 68 isolated clones from the libraries panned with GST-fusion proteins. 17 different V_{H} domain and 15 different V_{L} domain gene sequences were present. However, one V_{H} domain and one V_{L} domain predominated. These results confirmed that the construction of scFv libraries was successful resulting in a diverse repertoire and that solution-phase panning enriched phage clones. 19 of 68 sequenced clones showed different DNA sequences either in their V_{H} domain or in V_{L} domain.

### 4. Mini-scale expression and characterization of isolated clones using soluble scFvs

To characterize the specificity of isolated phage clones using full-length NS_{M} protein, 19 isolated phage-scFv clones after 2^{nd} and 3^{rd} round of panning with different amino acid sequences were induced with IPTG to express soluble scFvs in *E. coli.* Secretion of soluble scFvs into the supernatants was detected by dot blot using *c-myc* tag specific monoclonal antibody 9E10.

Fifty µl of culture supernatant containing soluble scFv from each clone were immobilized on the nitrocellulose membrane and detected using anti*-c-myc* tag monoclonal antibody (9E10) (0.3 µg/ml) and goat-anti mouse polyclonal antibodies conjugated to alkaline. phosphatase (0.12 µg/ml) followed by staining with NBT/BCIP.

NS_{M}-specific clones were identified by ELISA using mini-scale expressed soluble scFvs and full-length NS_{M} protein for coating. Four clones designated N3, N5, N10 and N12 (Tables 4 and 5) reacted with NS_{M} protein with an OD_{410 nm} value higher than 0.2, were positive clones, while the pHEN4II vector control gave an OD_{410 nm} value of 0.02. These clones were further characterized for antigen-binding activity using large-scale expressed soluble scFvs in *E. coli* and subsequently cloned into plant expression vector pSSH1 for functional analyses in transient expression and stable transformation.

One clone designated G19 (Table 5), was used as non-specific control in bioassays with transgenic plants. G19 contains the most frequent sequence in the isolated clones.5. Isolation of NS_{M}-specific phage clones using full-length NS_{M} protein

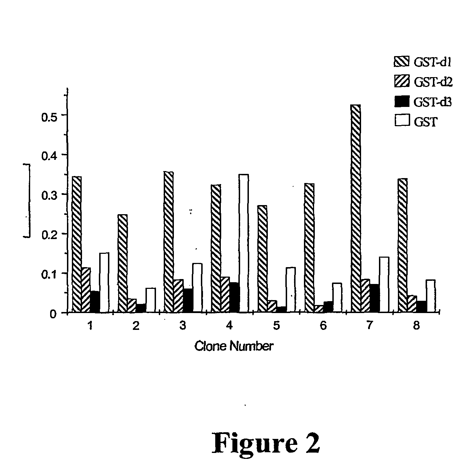

To avoid the false positives caused by carrier protein-antigen fusions during biopanning and screening, full-length NS_{M} protein without any carrier was used for phage-displayed antibody selection. Full-length NS_{M} protein was prepared using IMPACT expression system as described and used for panning the two phage libraries pHEN4II+LH and pHEN4II+HL. Because NS_{M} protein remains soluble only in SDS-containing buffers (SB), a solid-phase panning procedure using a PVDF membrane was performed to isolate NS_{M}-specific phage clones. After four rounds of panning, 80 monoclonal phage were analyzed by capture phage ELISA using GST-domain fusion proteins, in which GST-domain fusions were captured by GST polyclonal antibodies coated on the plates, followed by addition of monoclonal phage. The result showed that 82% analyzed phage clones reacted with GST-d1 fusion protein giving at least two-fold higher OD₄₁₀ₙₘ values compared to GST controls, and half of them gave three-fold higher OD₄₁₀ₙₘ values than for GST (Fig. 2).

Twenty-seven (27) clones isolated from NS_{M} panned libraries were sequenced. Five clones with different amino acid sequences in ORFs, designated P6, P7, P29, P17 and P11 (Table 2), were subcloned into the prokaryotic secretion vector pTMZ1 for large-scale expression of soluble scFvs for further characterization of antigen-binding activity. These five clones were subsequently cloned into plant expression pSSH1 vector for transient expression of the scFvs and for generation of stable transgenic plants for testing functions of the scFvs *in vivo* and *in vitro* (Table 3).

Fig. 2 displays an analysis of the reactivity of monoclonal phage from full-length NS_{M} panned library using GST fusion proteins in capture phage ELISA. The OD values at 410 nm of eight monoclonal phage isolated by panning against full-length NS_{M} protein on a PVDF membrane are shown. Capture phage ELISA was performed as described using GST-domain fusion proteins and GST control (1 µg/ml). The antigens used for ELISA are indicated.

### EXAMPLE 4: EXPRESSION AND CHARACTERIZATION OF SOLUBLE SCFVS

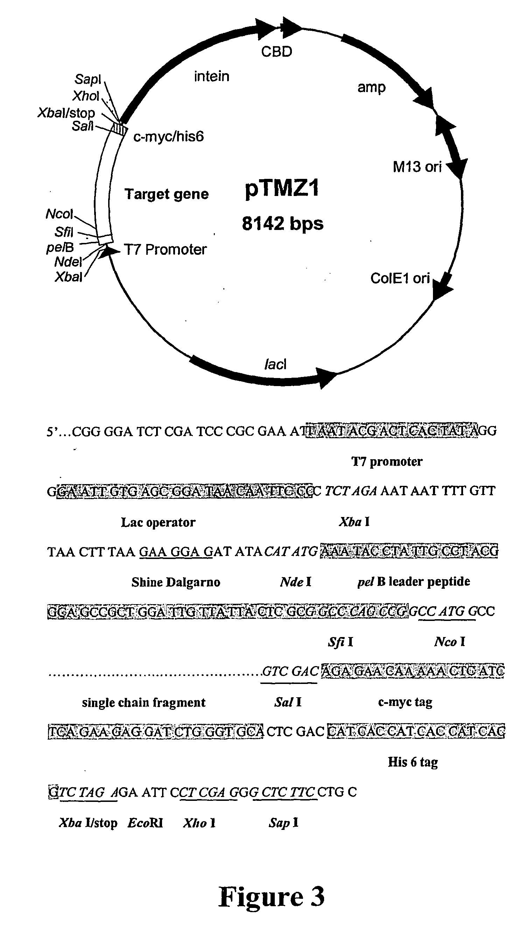

Five phage-displayed scFv clones (P6, P7, P29, P17 & P11) isolated from solid-phase panning against full-length NS_{M} protein using a PVDF membrane as a support (Table 2 and Table 5) and four phage antibody clones (N5, N10, N12, N3) isolated from solution-phase panning against GST-NS_{M} and GST-domain fusion proteins were subcloned downstream of the *pel*B leader peptide of the pTMZ1 vector (Figure 3) using *Nco*I and *Sal*I restriction sites. In addition, one phage antibody clone showing GST-binding capacities (Table 2, G19) and another two phage antibody clones showing relatively low NS_{M}-binding activities (Table 2), clones G5 and G6, were also cloned in the pTMZ1 vector for further characterization.

### A. Large-scale expression of soluble scFvs

### 1. Expression of scFv in pTMZ1 and purification of secreted ScFv by IMAC

To further characterize isolated scFvs, their respective scFv genes were subcloned into prokaryotic secretion vector pTMZ1 either between NcoI and SaII restriction sites or SfiI and SalI restriction sites if there was an internal NcoI restriction site in the scFv gene. The resulting recombinant DNA was transformed into *E. coli* strain ER2566 to express soluble scFv fragments.

A single recombinant colony was inoculated in 5 ml of LB medium containing 1% (w/v) glucose and 100µg/ml ampicillin (LBGA) and cultivated at 37°C o/n with shaking (200rpm). Two milliliters of an o/n culture was transferred into 200 ml of LBGA medium and cultivated at 37°C until the OD₆₀₀ₙₘ reached 0.4-0.5. The culture was centrifuged (4,000g/4°C/15 min) and the cell pellet resuspended in the same volume (200 ml) of LBA medium, cultivated at 37°C for 45 min followed by induction with 0.1 mM IPTG at 30°C for 16 h. The cells were separated from the culture medium by centrifugation (5,000g/4°C/10 min) and soluble scFvs in periplasmic space of *E. coli* were isolated by osmotic shock.

His-6 tagged secreted scFvs were affinity purified by IMAC. After removal of bacterial cells, the culture medium was adjusted to pH 7.4 using 10xPBS buffer supplemented with 1 M NaCl. Following centrifugation (16,000g/4°C/20 min) the culture medium was filter-sterilized (0.2 µm). A 0.5 cm (diameter) x 20 cm (length) column was packed with 2 ml of ProSep Chelating matrix, charged with 5 column volumes (CV) of 50 mM NiSO₄ and equilibrated with 10 CV of binding buffer (PBS pH 7.4, 1 M NaCl). Filtered culture medium was applied to the column at a flow rate of 2 ml/min. After sample application, the column was washed with 5 CV of binding buffer. Non specifically bound proteins were removed with binding buffer containing 25 mM imidazole. Ni-NTA bound his-6 tagged scFvs were eluted using 2 CV of binding buffer containing 125 mM imidazole. The collected fractions were immediately dialyzed against PBS (pH 7.4) to remove imidazole and salt. Purified scFv concentration was determined by Bradford assay using BSA as a standard and the scFv preparation used for characterization of scFv by immunoblot, ELISA and determination of the affinity constant using surface plasmon resonance (BIAcore).

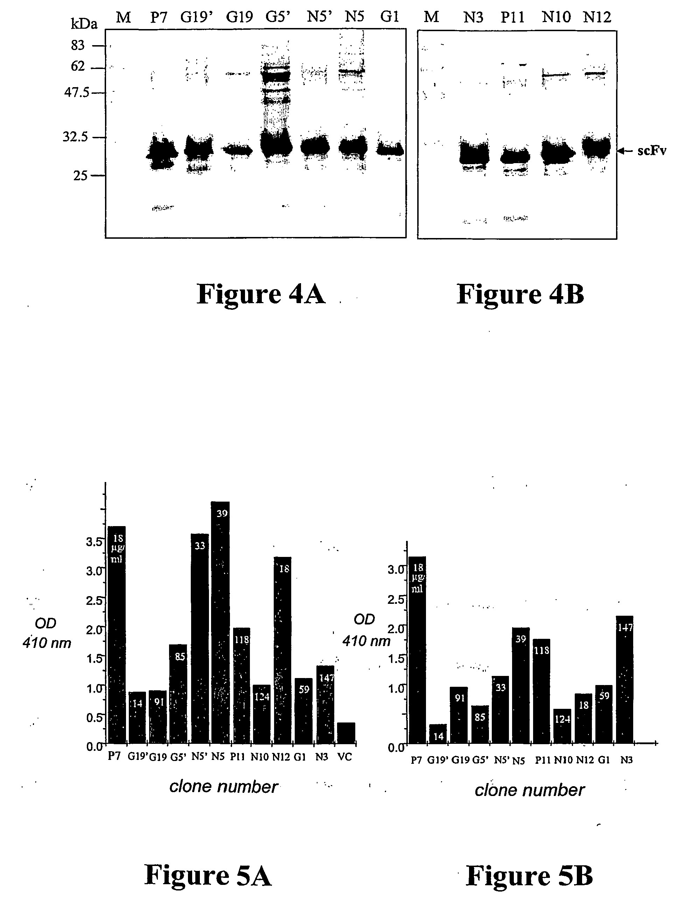

All 12 scFv antibody clones (Table 2) were expressed by induction with IPTG. His-tagged scFvs secreted into the culture medium were purified by IMAC. The concentration of affinity purified scFvs from culture medium was determined by Bradford assay using BSA as a standard. Nine scFv clones (Table 2 and Fig. 4, P7, G19, G5, N5, G1, N3, P11, N10 and N12) expressed soluble scFvs in culture medium at high levels, which ranged from 1.0 to 5.0 mg per litre culture medium. Clone G19 (Table 2) revealed the maximum yield of 5.7 mg per litre culture medium. The two clones (P6 & P17) produced very low levels of secreted scFvs, which were only detectable by immunoblot, but not by SDS-PAGE. Another two clones (P29 & G6) did not secrete scFvs into culture medium since no scFvs were detectable in the culture medium using a Ni-NTA charged sensor chip by surface plasmon resonance analysis (Johnson et al., Biotechniques 11(5): 620-7 (1991); Leidberg et al., Sensors and Actuators 4, 299-304 (1983)).

Ni-NTA bound scFvs from three clones (Fig. 4, G19, G5 & N5) could be released by 25 mM imidazole, which was actually used for removing non-specific bound proteins prior to elution with 125 mM imidazole. In one case (Table 2, G5), all bound scFvs were released by 25 mM imidazole. The yield of secreted scFvs of these three clones (Table 2, G19, G5 & N5) were relatively high compared to other clones.

**Table 2: Alignment of deduced amino acid sequence of complementarity determining regions (CDRs) of isolated clones in pTMZ1 vector.**

| | | | | | | |
|---|---|---|---|---|---|---|
| **Clone Name** | **H1** | **H2** | **H3** | **L1** | **L2** | **L3** |
| G19 | SYDMV ≈ | GIS-SGTSPNYGAAVKG ≈ | NEGADWCGHYYCSVAY--IDA ≈ | SGGSGDH----YG ≈ | NNDNRPS ≈ | GNRDSSA-----GI |
| P6 | SYGMV ≈ | GIDADGIYTNYGAAVKG ≈ | GAYGYCDSGTWCADDY--IDA ≈ | TGSSSY-----YG ≈ | ESSSRPS ≈ | GSYDGSIDAGYVGI |
| P7 | SNDMG ≈ | AIGNTGSWTGYGAAVKG ≈ | AA-GYCGSQSCGSAAY--IDA ≈ | SGASY-----YG ≈ | FDNKRPS ≈ | GSTDNTNYD----I |
| P29 | SYAMY ≈ | GIYSSGSSTYYAPAVKG ≈ | ESYSK----YY-GPGE--IDA ≈ | SGGGSSD----YG ≈ | QKNQRPS ≈ | GSTDDSATV----I |
| P17 | GYIMH ≈ | GIDAGGGVTWYGAAVQG ≈ | DGDNCCTTS---GADQ--IDA ≈ | SGGGSNSGSYYYG ≈ | DNTKRPS ≈ | GSGDSYSSV---GI |
| P11 | SYGFN ≈ | GINADGSETAYGAAVKG ≈ | SIGGSYCGSSGCYINIGTIDA ≈ | SGSSGS-----YG ≈ | TNDKRPS ≈ | GGYDYNANT---GI |
| G5 | SYEMQ ≈ | AISNDGSWTGYGAAVKG ≈ | SVYGG-CGN---AAAQ--IDA ≈ | SGTSGSY----YS ≈ | DNNKRPS ≈ | GGYDSDSARYVP-I |
| G6 | TYEMQ ≈ | GIDDDGSSTYYATAVKG ≈ | DVSDDGVCG---GAIW--IDA ≈ | SGDAW-----YG ≈ | QNDKRPS ≈ | GSGDSSAGYV--GI |
| N5 | SYDMV ≈ | GIS-SGSVPNYGAAVKG ≈ | NEGADWCGHYYCSVAY--IDA ≈ | SGGSGDH----YG ≈ | KNENRPS ≈ | GNRDSSA-----GI |
| N10 | SYSMG ≈ | GIGSSVIRTYYAPAVKG ≈ | ESGSGK---WF-SIGQ--IDA ≈ | SGGSGN-----YG ≈ | SSDKRPS ≈ | GSADSSHL----GI |
| N12 | SYDMV ≈ | GIS-SGSGPNYGAAVKG ≈ | NEGADWCGHYYCSVAY--IDA ≈ | SGGSGDH ---- YG ≈ | NNDNRPS ≈ | GNRDSSA-----GI |
| N3 | SYSMG ≈ | GIGSSVIRTYYAPAVKG ≈ | ESG---SG-KWFSIGQ--IDA ≈ | SGGSGS-----YG ≈ | SSDKRPS ≈ | GSADSSHL----GI |

Twelve isolated scFv genes were cloned into the pTMZ1 vector via the *Nco*I*-Sal*I restriction sites for large-scale expression of soluble scFvs in *E. coli.* Clone P6 and P17 were from the phage library panned against full-length NS_{M} protein on a PVDF membrane. The remaining clones (G5, N5, N10 and N12 ) were from solution-phase panned libraries against GST-NS_{M} domain fusion proteins. G19 was a non-specific clone used as control. CDRs (H1, H2, H3, L1, L2, L3) were determined according to Kabat et al. (1991). "*": the same amino acid residue as above. "≈": framework regions or linker region. "-": no amino acid residue at this position.

### B. Immunoblot analysis of purified soluble scFvs

Immunoblot analysis of eleven selected soluble scFvs was carried out using anti-*c-myc* monoclonal antibody 9E10 (Fig. 4). According to the concentration estimated with Bradford assay, 300 ng of soluble scFvs from each clone was resolved on SDS-PAGE gels and subsequently blotted onto the PVDF membrane. The result showed that all purified scFvs eluted either with 25 mM or 125 mM imidazole had the expected size of 28 kDa (Fig. 4). Occasionally, an additional band was detected by western blot, which was probably a co-purified bacterial protein of about 60 kDa.

Fig. 4 is an Immunoblot analysis of bacterially expressed soluble scFvs. Selected scFv genes were subcloned into the pTMZ1 vector and expressed in the *E. coli* strain ER2566 by induction with IPTG and secreted scFvs were purified by immobilized metal-ion affinity chromatography (IMAC) which purifies the proteins via the His6 tag. Three hundred ng of purified scFv were resolved on SDS-PAGE gels and blotted onto Hybond-C membrane. Blotted scFvs were revealed by anti*-c-myc* monoclonal antibody (9E10) (0.3 µg/ml) as primary antibody and goat-anti mouse polyclonal antibodies conjugated to alkaline phosphatase (0.12 µg/ml) as a secondary antibody followed by addition of the NBT/BCIP substrate. The names of scFv clones are indicated. Clone names highlighted with prime marker (') represent scFvs eluted with 25 mM imidazole. All other scFvs were eluted with 125 mM imidazole. M: Molecular weight standards / pre-stained protein markers.

### C. ELISA analysis of purified soluble scFvs

The antigen-binding capacity of NS_{M}-specific soluble scFvs was analyzed by indirect ELISA using NS_{M} protein from inclusion bodies as antigens and capture ELISA using the purified soluble NS_{M} obtained from IMPACT system (Fig. 5). Six clones showed high binding signals to NS_{M} protein from inclusion bodies (Fig. 5A, P7, G5, N5, P11, N12 & N3), of which, four scFvs showed high binding signals to the soluble NS_{M} protein (Fig. 5B, P7, N5, P11 and N3) as well. Of four clones (Table 2, N5, N10, N12 and N3) which showed also strong binding activities to NS_{M} protein from inclusion bodies in the indirect ELISA using mini-scale expressed soluble scFvs, two clones (N5 & N12) reacted more strongly with NS_{M} protein from inclusion bodies (Fig. 5A) than soluble NS_{M} protein (Fig. 5B) using large-scale expressed scFvs, while one clone (N3) reacted more strongly with soluble NS_{M} protein than with NS_{M} protein from inclusion bodies (Fig. 5), and the remaining one (N10) showed no significant difference in binding activity to either form of the NS_{M}. Two clones (P7 and P11) isolated from solid-phase panning on PVDF membrane using full-length NS_{M} protein showed similar binding activities to NS_{M} protein from inclusion bodies and soluble NS_{M} obtained from IMPACT system.

G19 displayed low binding signals to NS_{M} protein from inclusion bodies (Fig. 5A). Of two clones (G5 and G6) that showed low binding signals to NS_{M} protein from inclusion bodies in the phage ELISA, one clone (G5) gave a higher binding signal to NS_{M} inclusion bodies than to native NS_{M} protein (Fig. 5), the other (G6) was not characterized in this assay because of its low expression level.

Fig. 5 displays the reactivity of affinity purified soluble scFvs in indirect ELISA using NS_{M} protein from inclusion bodies (A) and capture ELISA using soluble NS_{M} protein obtained from the IMPACT system (B). NS_{M} inclusion bodies (3µg/ml) were immobilized on ELISA plates and affinity purified scFvs were added to the plate. Bound scFvs were detected by anti*-c-inyc* monoclonal antibody 9E10 (0.3 µg/ml) and revealed by goat-anti mouse, conjugated to horse radish peroxidase HRP (0.12 µg/ml) and ABTS substrate (used according to manufacturer's, Roche Molecular Biochemicals, instructions). The color development was carried out at room temperature for 30 min. The concentrations of purified scFvs (µg/ml) were indicated within the bars.

Rabbit polyclonal antibodies raised against native NS_{M} (1µg/ml) were coated on the plate. Upon the addition of soluble NS_{M} protein (0.5µg/ml) from the IMPACT system, the same scFvs as in indirect ELISA (A) were added to the plate. Bound scFvs were detected as above. The concentrations of purified scFvs (µg/ml) were indicated within the bars.

The results from ELISA analysis of bacterially expressed soluble scFvs demonstrated that all seven NS_{M}-specific scFvs (P7, G5, N5, P11, N10, N12 & N3) have binding specificities for either NS_{M} from inclusion bodies or soluble NS_{M} protein from IMPACT system, in which five clones (P7, N5, P11, N12 & N3) showed high binding signals. These seven NS_{M}-specific clones together with GST-specific clone G19 as control were selected for subsequent subcloning into the plant expression vector pSSH1 for characterization of the biological function of NS_{M}-specific scFvs *in vino.* The other four clones (P6, P29, G6 & P17) (Table 2) were also used for plant expression despite the low expression level (P6 & P17) or no detection (P7 & G6) of soluble scFvs in culture medium.

### EXAMPLE 5: GENERATION AND CHARACTERIZATION OF TRANSGENIC PLANTS

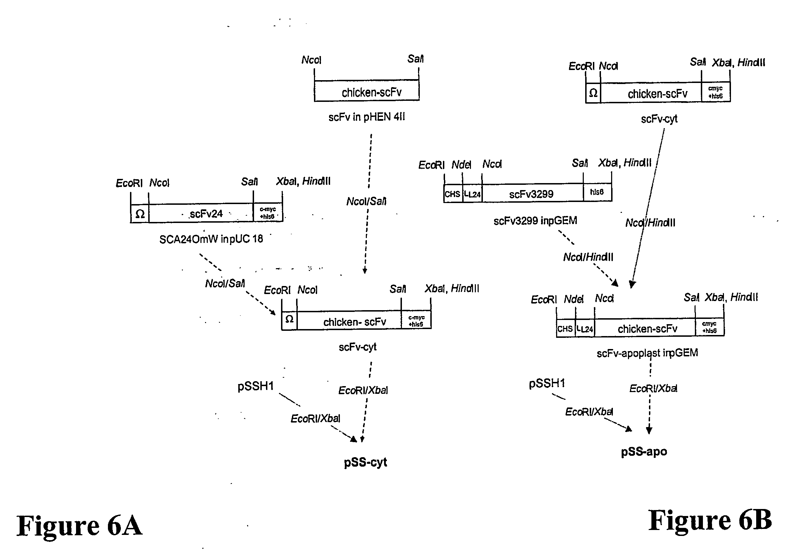

To characterize the expression level of NS_{M}-specific scFvs, 12 scFv genes were subcloned into the plant expression vector pSSH1 for expression of scFvs in the plant cytosol (Fig. 6A) or secretion of scFvs to apoplastic space (Fig. 6B). 24 constructs in total were generated and all constructs were transformed into *Agrobacteria.* Eleven cytosolic expression constructs and eleven corresponding apoplastic expression constructs were analyzed using transiently expressed scFvs in tobacco leaves to validate the plant expression constructs and to evaluate the scFv expression levels in plant cytosol. Following the transient expression, all 12 cytosolic constructs (Table 2) were stably transformed into tobacco plants by agrobacteria-mediated transformation.

Transiently expressed scFvs in tobacco leaves were analyzed by immunoblot and ELISA using crude extracts of total soluble leaf protein. Expression of scFvs in transgenic plants was analyzed by immunoblot using crude extracts of total soluble leaf protein from 20 or 25 regenerated plants for each construct.

### A. Cloning of NS_{M}-specific scFv genes into plant expression pSSH1 vector

The strategy for cloning of scFv genes into the plant expression pSSH1 vector is shown in Fig. 6. Twelve (12) scFv genes (Table 3) were cloned into two expression cassettes that allow the expression of scFvs in plant cytosol (Fig. 6A) or target scFvs for secretion into the apoplast (Fig. 6B).

NS_{M}-specific or non-specific scFv genes (Table 5) were cloned into the pSSH1 vector for the expression and targeting of scFvs to the plant cytosol (A) or secretion into the apoplastic space (B). The designations in the figure are as follows: Ω: omega sequence (5' untranslated sequence of TMV); CHS: 5' untranslated region of the chalcon synthase; L_{L24}: plant codon optimized leader peptide of the rAb24 light chain; *c-myc*: myc epitope for scFv detection; his-6: his-6 epitope for purification and detection; scFv24: TMV specific single chain antibody 24; scFv3299: HCG-specific scFv.

For cytosolic targeting, pSS-cyt constructs were generated (Fig. 6A) by digesting selected scFv genes in the pHEN4II vector with *Nco*I and *Sal*I restriction enzymes and subsequent cloning into pUC 18 which contained an omega sequence, the scFv24 and c-*myc*/his-6 tags (SCA24OmW in pUC18) digested with the same restriction enzymes, resulting in the scFv-cyt recombinant DNA. The cassette containing omega sequence, scFv gene and *c-myc*/his 6 tags in the scFv-cyt plasmid was excised using *Eco*RI and *Xba*I and cloned into the pSSH1 vector resulting in the pSS-cyt constructs.

For generation of pSS-apo constructs allowing the secretion of scFvs into apoplastic space (Fig. 6B), scFvs in scFv-cyt plasmid (Fig. 6A) were excised using *Nco*I and *Hind*III restriction enzymes and cloned into *Nco*I*-Hind*III digested pUC18 containing the 5' untranslated region of the chalcone synthase, codon-optimized leader peptide of the rAb24 light chain and *c-myc*/his6 tags (scFv3299 in pUC18) to obtain the scFv-apoplast recombinant DNA. The scFv-apoplast recombinant DNA cassette containing the 5' untranslated region of the chalcone synthase, codon-optimized leader peptide of the rAb24 light chain, scFv genes and *c-myc*/his-6 tags were excised using *Eco*RI and *Xba*I restriction enzymes and cloned into *Eco*RI*-Xba*I restricted pSSH1 vector, resulting in pSS-apo constructs. The recombinant DNA of pSS-cyt and pSS-apo constructs was transformed into *Agrobacterium* either by nitrogen transformation or by electroporation. Four independent colonies from each transformation were analyzed by PCR for the presence of the scFv DNA and *Agrobacterium* cultures were prepared from a single positive clone of each construct and used for transient expression and stable transformation of *N. tabacum.*

### B. Generation and characterization of transgenic plants

### 1. Transient assay in tobacco leaves by vacuum infiltration

*Agrobacterium tumefaciens* GV 3101 (pMP90RK Gm^{R}, Km^{R}), Rif^{R} (Koncz, and Schell, "The promoter of TL-DNA gene 5 controls the tissue specific expression of chimeric genes carried by a novel type of Agrobacterium binary vector." Mol Gen Genet 204: 383-396 (1986) incorporated herein by reference) was used for *Agrobacterium-mediated* gene transfer. *N. tabacum* L. cv. Petite Havana SR1was used for transient expression by agrobacterial vacuum infiltration, and generation of stably transformed plants. Growth of recombinant *Agrobacterium* and vacuum infiltration of tobacco leaves was performed as described Kapila et al.,"An Agrobacterium mediated transient gene expression system for intact leaves." Plant Sci. 122: 101-108 (1997) incorporated herein by reference.

### a. Preparation of Agrobacterium

A single colony of a positive *Agrobacterium* clone harboring a recombinant pSSH1 plasmid was verified by *Agrobacterium*-control PCR and inoculated in 5 ml of YEB medium and cultivated at 28°C for 2-3 days with shaking at 250 rpm. One milliliter was transferred into 100 ml of YEB-km-rif-carb medium and cultivated at 28°C o/n with shaking at 250 rpm. Ten milliliter of an o/n culture was transferred into 250 ml of induction medium in a 500-ml flask and cultivated at 28°C o/n with shaking at 250 rpm. Agrobacteria cells were centrifuged (4,000g/15-25°C/15 min) and resuspended in 50 ml of MMA solution and kept at RT for 2 h. The OD₆₀₀ₙₘ was measured after 1:10 dilution and the cell suspension was diluted to an OD₆₀₀ₙₘ of 2.4. 100 ml of the diluted cell suspension was used for infiltration.

### b. Infiltration of intact leaves

Young tobacco leaves (4 leaves for each construct) containing recombinant pSSH1 plasmid were placed in 100 ml suspension of agrobacteria normal in a "Weck" glass and a continuous vacuum (60-80 mbar) was applied for 15-20 min. The applied vacuum was released rapidly and the leaves were briefly rinsed in tap water and kept on a wet Whatman paper no. 1 with adaxial side upward. The Whatman paper was soaked in water. The plastic tray was sealed with saran wrap and placed at 22°C with a 16 h photoperiod for 60 h. Following the removal of the central leaf vein, leaves were weighed, frozen in liquid nitrogen and stored at -80°C until analysis. As a control, leaves were infiltrated with agrobacteria suspension, which did not contain the pSSH1 plasmid.

### c. Transient expression of scFvs in tobacco leaves by vacuum infiltration

Upon subcloning in the plant expression pSSH1 vector, eleven scFv clones (Table 3 & 4) were analyzed by transient expression in tobacco leaves. The eleven cytosolic constructs as well as their corresponding eleven apoplastic constructs were transformed into tobacco leaves for the secretion of recombinant proteins into the apoplast, which usually results in higher protein accumulation (Conrad and Fiedler "Compartment-specific accumulation of recombinant immunoglobulins in plant cells: an essential tool for antibody production and immunomodulation of physiological functions and pathogen activity." Plant Mol Biol 38: 101-109 (1998); Fischer et al., "Expression and characterization of bispecific single chain Fv fragments produced in transgenic plants." Eur J Biochem 262: 810-816 (1999); Schillberg et al., "Apoplastic and cytosolic expression of full-size antibodies and antibody fragments in Nicotiana tabacum." Transgenic Research 8: 255-263 (1999)). Accumulation of the scFvs in tobacco leaves was analyzed by immunoblot and ELISA 72 h after vacuum infiltration.

### d. Preparation of total soluble proteins from plant leaves

For the extraction of transiently expressed scFv in vacuum infiltrated tobacco leaves or in stably transformed tobacco plant leaves, frozen leaves were ground in liquid nitrogen to a fine powder with a mortar and pestle. Total soluble proteins were extracted using 2 ml of extraction buffer per gram leaf material. Cell debris was removed by two rounds of centrifugation (16,000g/4°C/30 min) and the supernatant used for expression analyses by immunoblot and ELISA.

| | | |
|---|---|---|
| Induction medium: | YEB medium, | pH 5.6 |
| | MES | 10 mm |

2 mM MgSO₄, 25 µg/ml Kanamycin, 100 µg/ml Rifampicin, 100 µg/ml Carbenicillin, 20 µM Acetosyringone were added after autoclaving and cooling.

| | | |
|---|---|---|
| MMA buffer: | MS-salts (Murashige & Skoog, basic salt mixture) | 0.43% (w/v) |

| | |
|---|---|
| MES pH 5.6 | 10 mM |
| Sucrose | 2% (w/v) |

200 µM Acetosyringone were added after autoclaving and cooling.

| | | |
|---|---|---|
| Extraction buffer: | Tris-HCl, pH 7.5 | 200 mM |
| | EDTA | 5 mM |
| | DTT | 0.1 mM |
| | Tween 20 | 0.1% (v/v) |

As summarized in Table 3, four out of eleven cytosolically expressed scFvs were detectable by immunoblot (P6, G19, G5 & N10), indicating that chicken derived scFvs generated by phage display can accumulate to high levels in plant cytosol. Surprisingly, only six out of eleven apoplastically expressed scFvs were detectable by immunoblot, while the remaining five were undetectable. It was found that for four scFv clones (Table 3, P7, P29, G6 & N5), the apoplastically expressed scFvs accumulated to a high level, but their cytosolic counterparts were undetectable, while for two scFv clones (Table 3, G5 & N10), the apoplastically expressed scFvs were undetectable by immunoblot, but their cytosolic counterparts accumulated to detectable levels in the cytosol. For three scFvs (Table 3, P17, P11 & N12), neither cytosolically expressed scFvs nor apoplastic counterparts were detectable although the nucleotide sequences of these three clones were confirmed as well as their functional expression in *E. coli.*

Western blot analysis demonstrated that the scFvs expressed in tobacco leaves had the same size as the bacterially expressed scFvs (28 kDa). Variations in expression level were observed among four leaves for the same construct.

### 2. Stable transformation and analysis of transgenic plants

### a. Agrobacterium-mediated stable transformation of tobacco plants

Transgenic *N*. *tabacum* cv. Petite Havana SR1 were generated by leaf disc transformation using recombinant *Agrobacteria* and transgenic T₀ plants were regenerated from transformed callus (Fraley et al., "Expression of bacterial genes in plant cells", Proc Natl Acad Sci U S A 80: 4803-4807 (1983); Horsch et al., "A simple and general method for transferring genes into plants" Science 227: 1229-1231 (1985) both incorporated herein in their entirety by reference). Briefly, wildtype plants were grown on MS medium in "Weck" glasses and the youngest leaves (length up to 6 cm) were used for transformation. The agrobacteria suspension was prepared as described and the OD₆₀₀ₙₘ was adjusted to at least 1.0 after dilution in MMA buffer. The leaves were cut into 4-6 pieces (without the central vein) and transferred into "Weck" glasses containing 50-100 ml of agrobacteria suspension (OD₆₀₀ₙₘ ≅ 1.0) and incubated at RT for 30 min. The leaf pieces were then transferred onto sterile pre-wetted Whatman filters in petri dishes, closed with saran wrap and incubated at 26-28°C in the dark for two days. Following washing with distilled water containing 100 µg/ml kanamycin, 200 µg/ml claforan and 200 µg/ml Betabactyl (Ticarcillin / Clavulanic acid, 25:1), leaf pieces were transferred onto MS II-plates and incubated at 25°C in the dark for one week and with a 16 h photoperiod for 2-3 weeks. After shooting, the shoots were removed and transferred onto MS-III-plates and incubated at 25°C with a 16 h photoperiod for 10-14 days until roots developed. The small plants were transferred into Weck glasses containing MS-III medium and incubated at 25°C in 16 h light rhythm for 2 weeks until they would be transferred into soil. The young leaves from regenerated transgenic plants were used for immunoblot analysis of expressed scFvs.

| | | |
|---|---|---|
| MS medium: | MS-salts | 0.43% (w/v) |
| | Myo-Inosite (SERVA) | 0.1% (w/v) |
| | Sucrose | 2% (w/v) |
| | Thiamin-HCl | 0.4 mg/l |
| | A. bidest | add to 1000 ml |

The pH was adjusted to 5.8 with 1 N NaOH (for preparation of solid medium, 0.8% (w/v) agar were added), autoclaved and 500 µl of vitamin solution I were added upon cooling to 55°C.

| | | |
|---|---|---|
| MS-II medium: | MS medium supplemented with: | |
| | BAP (in DMSO, from Sigma) | 1 mg/l |
| | NAA (from Sigma) | 0.1 mg/l |
| | Kanamycin | 100 mg/l |
| | Claforan | 200-500 mg/l |
| | Betabactyl | 200-250 mg/l |
| MS-III medium: | MS medium supplemented with: | |
| | Kanamycin | 100 mg/l |
| | Claforan | 200-250 mg/l |
| | Betabactyl | 200-250 mg/l |
| Vitamin solution I: | Glycine | 0.4% (w/v) |
| | Nicotinic acid | 0.1% (w/v) |
| | Pyridoxin | 0.1% (w/v) |

Filter sterilized and stored at 4°C.

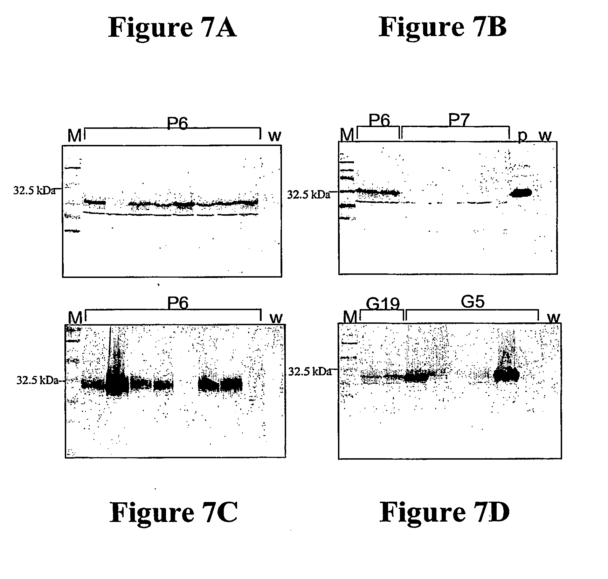

Twelve (12) cytosolic scFv constructs (Table 3) were stably transformed into *N*. *tabacum* cv. Petite Havana SR1 to target the expressed scFvs to the plant cytosol. 20 or 25 regenerated transgenic lines from each construct were analyzed by immunoblot (Fig. 7). The results showed that seven out of the 12 scFvs accumulated to detectable levels in the plant cytosol (Table 3, P6, G19, G5, N5, P17, N10 & N12), in which six scFvs (P6, G19, G5, N5, N10 & N12) accumulated to unexpectedly high levels in the cytosol of the primary transformants (0.2-0.4% of leaf total soluble proteins). The highest expression levels of four cytosolic constructs reached up to 0.3-0.4% of leaf total soluble proteins (G19, G5, N5 & N10). As shown in Fig. 7 and Table 3, variations of the expression level in the cytosol occurred in all these seven lines with an average level ranging from 0.1% to 0.3% of leaf total soluble proteins.

Fig. 7 is an immunoblot analysis of cytosolically expressed scFvs in transgenic tobacco plants. Expression levels of scFvs in 18, 5, 2 and 6 regenerated plants transformed with the cytosolic constructs derived from the clones P6, P7, G19 and G5 are shown. 20 µl of total soluble proteins extracted from transgenic plant leaves were resolved on 12% SDS-PAGE gels and blotted onto the PVDF membranes. M: Molecular weight standards / pre-stained protein markers; P: positively control (500ng of purified scFvs expressed in *E. coli*); W: total soluble proteins extracted from wild type tobacco leaves.

**Table 4: Comparison of expression levels of stably expressed scFvs in transgenic plants (To) and transiently expressed scFvs in tobacco leaves by immunoblotting.**

| **ScFv Construct** | **No. of T0 Plants producing different levels of scFvs** | | | | | **Transient Expression** | |
|---|---|---|---|---|---|---|---|
| | **Tested** | **High (+++)** | **Average (++, +)** | **Low (+/-)** | **undetected (-)** | **cytoplasm** | **apoplast** |
| P6 | 20 | 1 | 15 | 0 | 4 | + | +++ |
| P7 | 20 | 0 | 0 | 0 | 20 | - | ++ |
| P29 | 20 | 0 | 0 | 0 | 20 | - | ++ |
| P17 | 25 | 0 | 4 | 7 | 14 | - | - |
| P11 | 25 | 0 | 0 | 0 | 25 | - | - |
| G19 | 20 | 3 | 11 | 5 | 1 | ++ | ++ |
| G5 | 20 | 8 | 7 | 2 | 3 | +++ | - |
| G6 | 25 | 0 | 0 | 0 | 20 | - | ++ |
| N5 | 25 | 1 | 11 | 3 | 10 | - | ++ |
| N10 | 25 | 2 | 15 | 1 | 7 | ++ | - |
| N12 | 20 | 4 | 5 | 3 | 8 | - | - |
| N3 | 25 | 0 | 0 | 0 | 3 | nd | nd |

For each cytosolic scFv expression construct 25 plants were regenerated after stable transformation. 20 or 25 T₀ plants of each construct were analyzed by immunoblot as described (Fig. 7) 4-6 weeks after transferring into soil. The expression level in each plant was estimated based on a positive control using bacterially expressed scFv with known scFv concentration. Transiently expressed scFvs in tobacco leaves were analyzed by immunoblot and the expression level estimated. +++: 0.2-0.4% of leaf total soluble protein; ++: 0.1-0.2% of leaf total soluble protein; +: 0.05-0.1% of leaf total protein; +/-: 0.002-0.05% of leaf total soluble protein; -: undetectable; nd: not detected.

Overall, the results from stably expressed scFvs in transgenic tobacco lines were consistent with the results from transiently expressed scFvs. Three exceptions were identified with clones N5, P17 & N12 (Table 5), which were undetectable in transient assay, but were detectable by immunoblot in the cytosol of transgenic tobacco plant cells. From the plant expression data, it can be concluded that scFvs isolated by solution-phase panning procedure are more resistant to the reducing environment of plant cytosol than scFvs isolated by solid-phase panning using PVDF membrane as a support. Of eight scFvs isolated by the solution-phase panning (G19, G5, G6, N5, N10, N12, G1 & N3), five scFvs (G19, G5, N5, N10 & N12) accumulated to high levels in the cytosol (0.3-0.4%), while only two out of five scFvs isolated by solid-phase panning on PVDF membrane accumulated to an average level of 0.1-0.3% in the cytosol.

Characterization of transiently expressed scFvs in tobacco leaves and stably expressed scFvs in transgenic plants demonstrated that a variety of chicken derived NS_{M}-specific scFvs have been functionally expressed in the plant cytosol at high accumulation levels. Ten of these lines were selfed, seeds collected and seeded for the establishment of T₁ generation plants.

### b. Cytosolic accumulation levels of chicken scFv antibodies in transgenic T1 plants

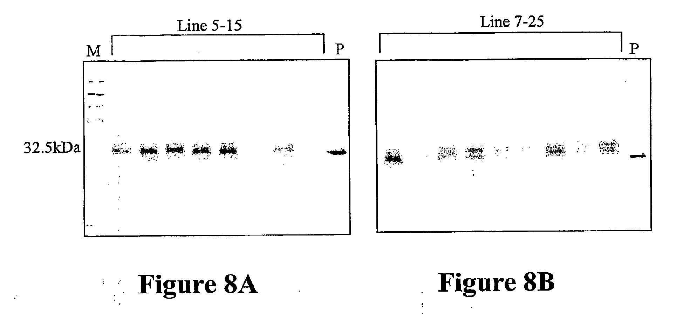

Chicken scFvs in transgenic plant leaves were determined by immunoblot using NBT/BCIP (Fig. 8) or chemo-luminescent substrate. Plant leaf total soluble proteins (TSP) were determined by Bradford assay using BSA as a standard. Expression levels were shown as the percentage of the chicken scFv in leaf total soluble protein (Table 4). ScFv fragments detectable in T0 plants were also detectable in T1 plants and scFvs not detectable in T0 plants were also undetectable in T1 plants. Accumulation levels of specific scFv fragments were similar in T0 and T1 plants. However, the T1 progeny of a parental T0 plant showed a distribution of accumulation levels with lower, similar and higher accumulation levels when compared to the parental T0 plants.

Fig. 8 is an immunoblot analysis of cytosolically expressed scFvs in transgenic T1 plants. Expression levels of scFvs in T₁ plants lines 5-15 and 7-25 transformed with the cytosolic constructs derived from the clones G5 and N5 are shown. 8 µl of total soluble proteins extracted from transgenic plant leaves were resolved on 12% SDS-PAGE gels and blotted onto the PVDF membranes. Blotted scFvs were detected as described for analysis of transiently expressed scFvs in tobacco leaves M: Molecular weight standards / pre-stained protein markers; P: positive control (500ng of purified scFvs expressed in *E. coli*).

**Table 4: Accumulation levels of stably expressed scFvs in transgenic plants (T₁).**

| **construct** | **panning antigen** | **Solution/solid phase panning** | **T₀ (%/TSP)** | **T₁ (%/TSP)** |
|---|---|---|---|---|
| P6 | full-length NS_{M} (IMPACT) | solid | 0.24 | 0.19 |
| G19 | GST-NS_{M} domains | solution | 0.4 | 5.91 |
| G5 | GST-NS_{M} domains | solution | 0.37 | 8.0 |
| N5 | GST-NS_{M} domains | solution | 0.3 | 2.62 |
| P17 | full-length NSM (IMPACT) | solid | 0.13 | 0.125 |
| N10 | GST-NS_{M} domains | solution | 0.28 | 0.24 |
| N12 | GST-NS_{M} domains | solution | 0.1 | 0.13 |

T₁ plants were analyzed by immunoblot using chemi-luminescence detection. The expression level in each plant was estimated based on a positive control using bacterially expressed scFv with known scFv concentration. The procedure for SDS-PAGE and protein transfer to the membrane is the same as for normal western blot except the alkaline phosphatase substrate is CDP star instead of NBTBCIP. Fuji LAS-1000 program was used to take the images and data was evaluated using AIDA (version 2.31) program.

### EXAMPLE 6 - CHARACTERIZATION OF CHICKEN-DERIVED SCFVS

Analysis of the deduced amino acid sequences of chicken-derived V_{L} domains, V_{H} domains of the scFv antibodies disclosed herein indicate that avian-derived antibodies share similar features with mammalian antibodies. Two cysteine residues which are highly conserved in mammalian immunoglobulins were also present in FR1 and FR3 in both V_{H} and V_{L} domains (at position +22 and +92 for V_{H}, and +23 and +88 for V_{L}) according to Kabat numbering of chicken-derived scFvs. These two cysteine residues may form intrachain disulfide bridges in both heavy and light chain variable domains. Several amino acid residues in the framework regions (FRs), and even in the complementarity determining regions (CDRs), are conserved in both mammalian and avian-derived antibodies. The CDR3 of the V_{H} domain in chicken-derived antibodies are longer than its human counterpart, while the other regions show no significant differences in length (Martin, "Accessing the Kabat Antibody Sequence Database by Computer." PROTEINS: Structure, Function and Genetics 25: 130-133 (1996)).

### A. Sequence analysis of chicken-derived scFvs

Amino acid sequences of 12 scFv clones were compared in order to find a suitable scaffold for high expression of single chain antibodies or other polypeptides in the plant cytosol.

12 scFv clones (Table 5 and 6) were divided into three classes. G19, N5, N12, N10, P6, G5 and P17 belong to the first group. They have similar framework regions, but differ in CDR regions. All these scFvs antibodies accumulated in the plant cytosol, about 0.13% to more than 1% of total soluble protein, indicating scFv antibodies comprising these framework scaffolds are stable in the cytosol and suitable for antibody engineering for intracellular immunization. P7, G6, P29, P11 and N3 (Table 5 and 6) belong to the second group. These scFvs were not detectable in the plant cytosol.

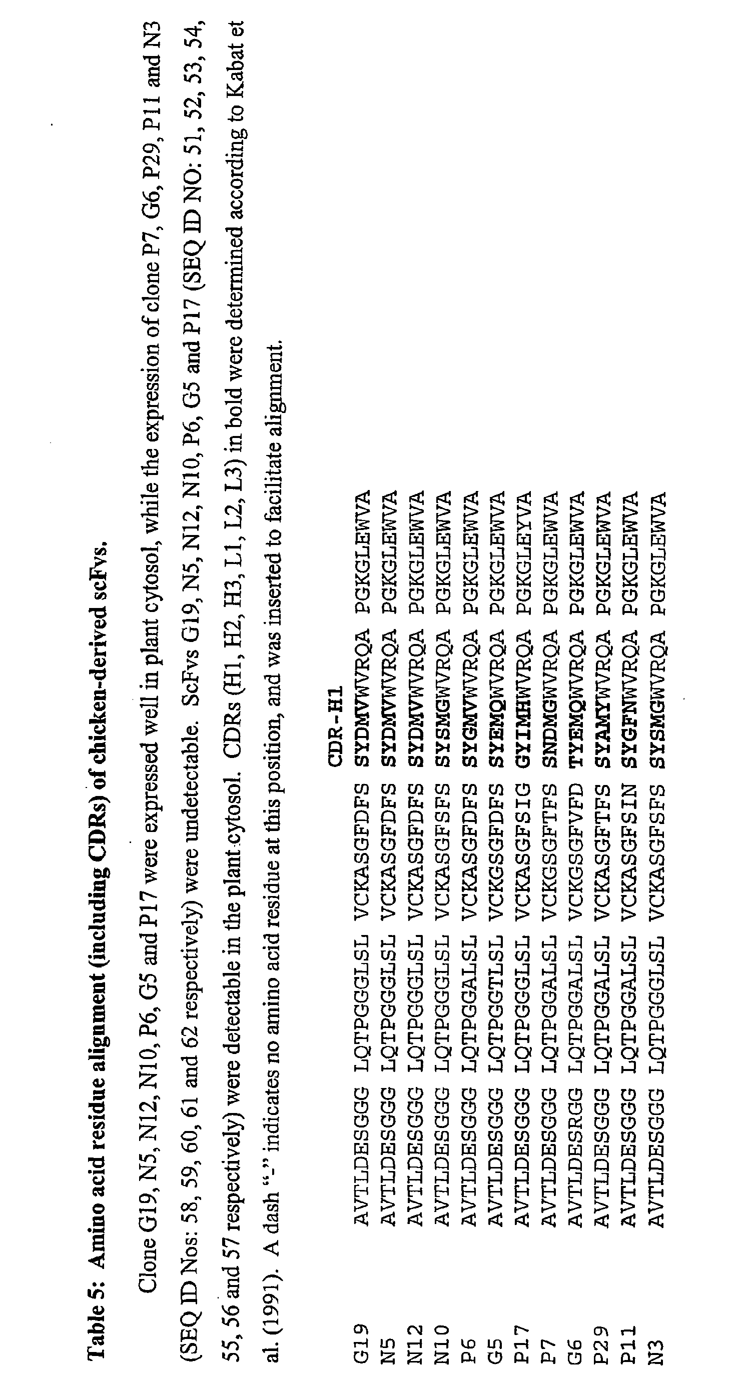

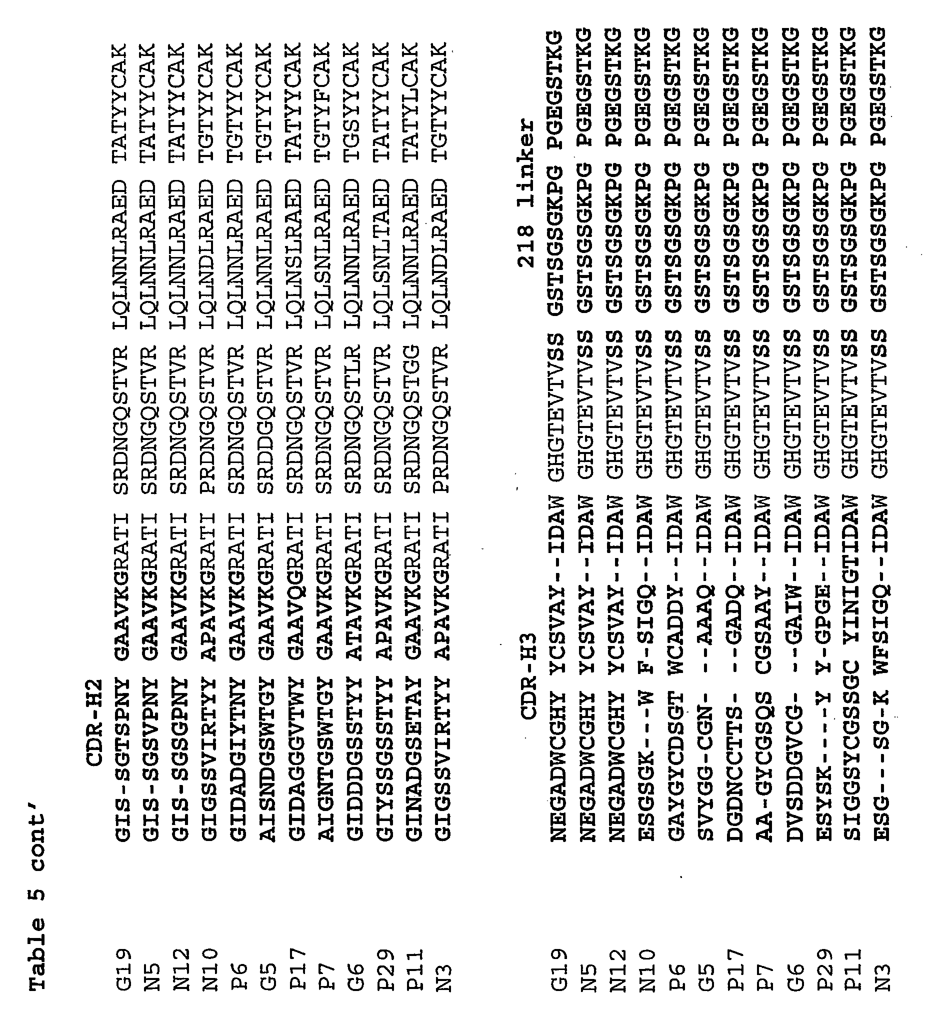

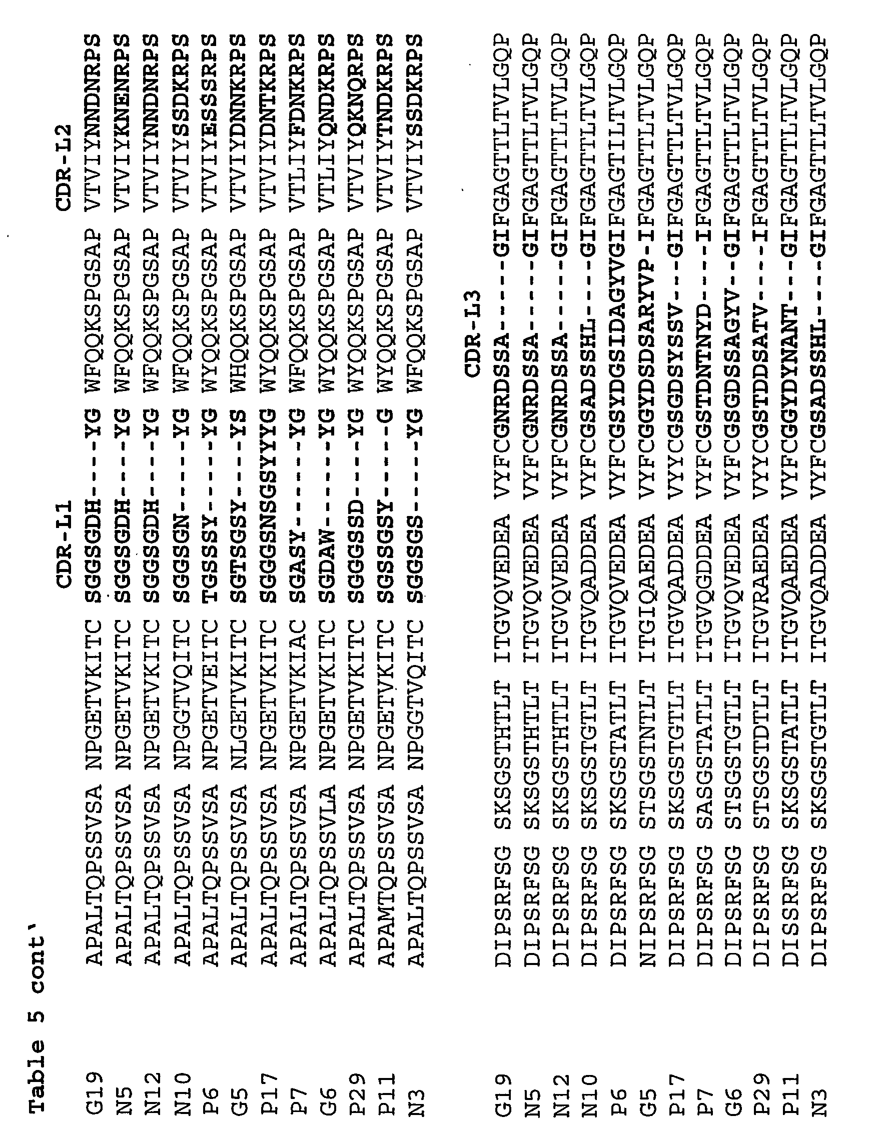

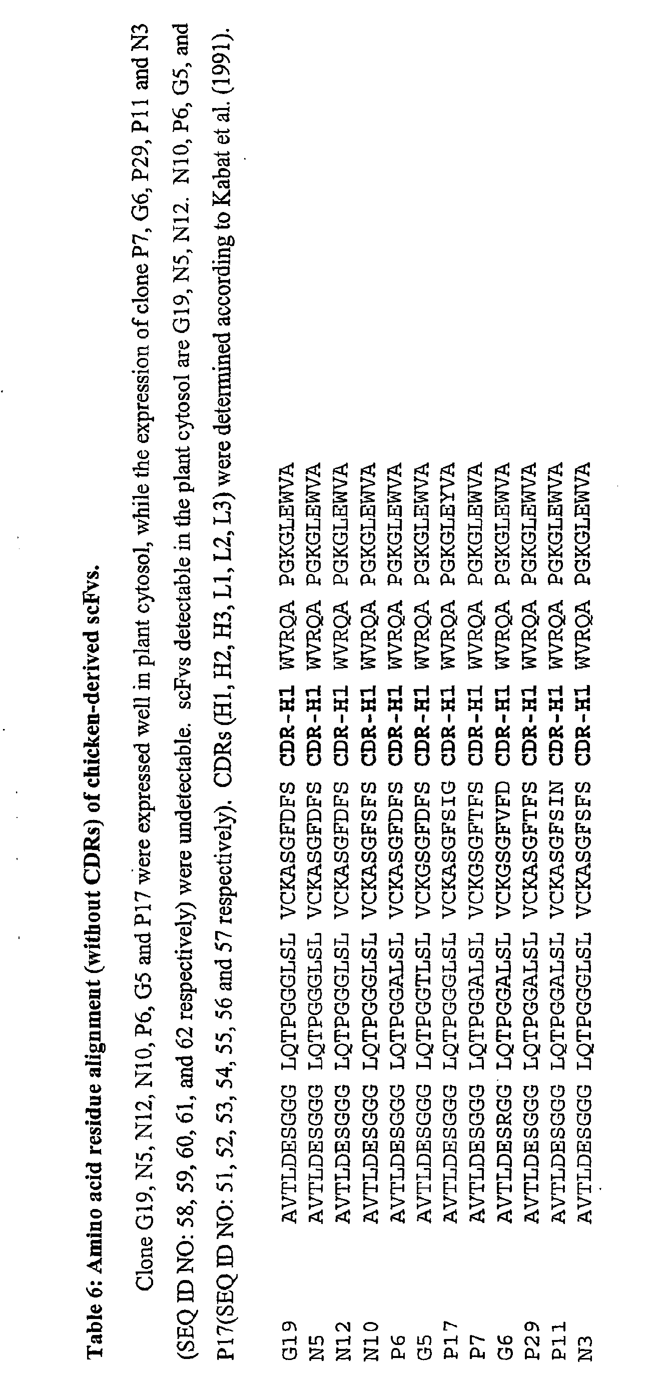

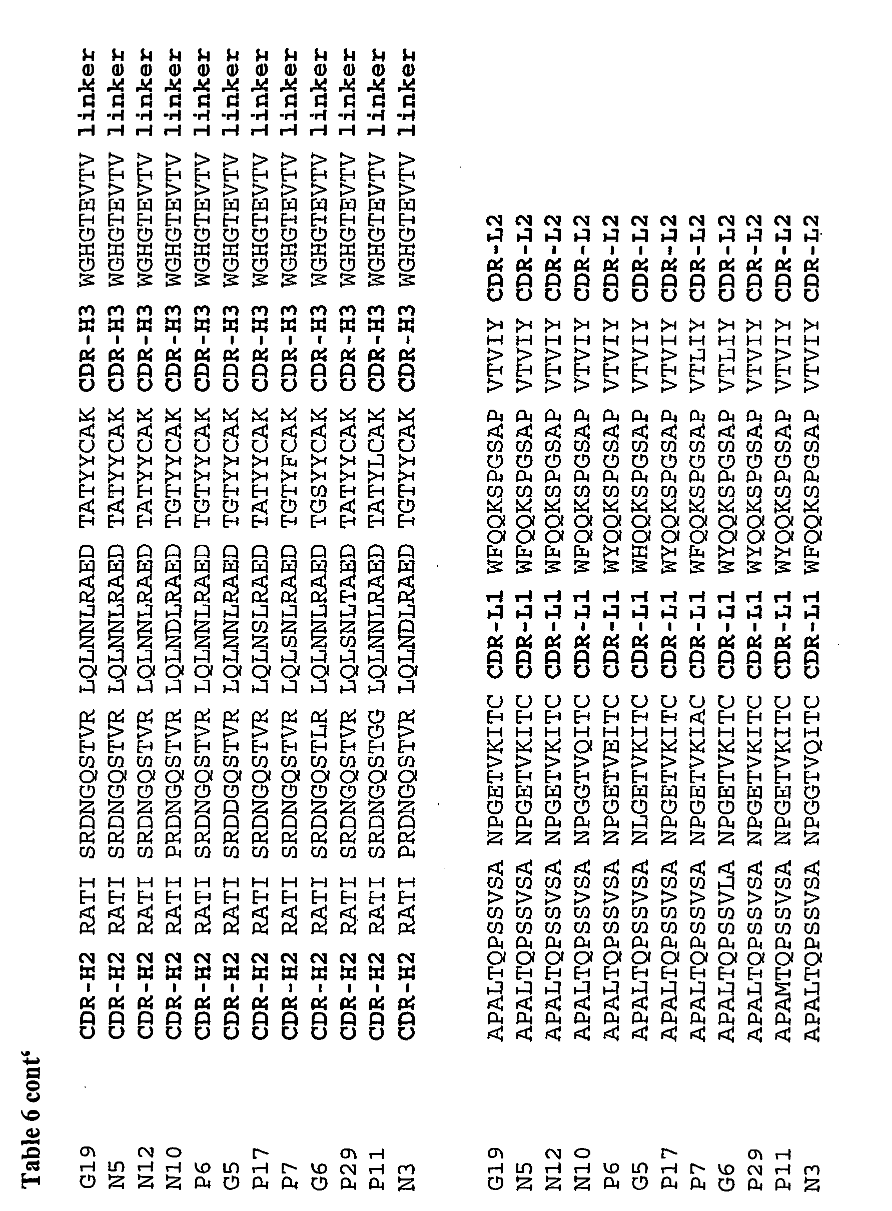

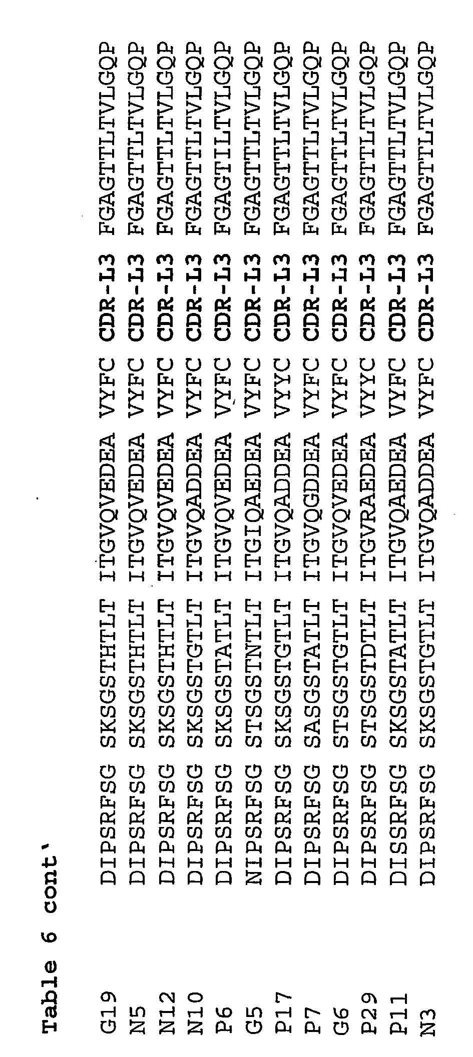

### EXAMPLE 7 - DESIGN AND CONSTRUCTION OF SEMI-SYNTHETIC CHICKEN ANTIBODY LIBRARY

Semi-synthetic chicken antibody libraries are created based on the framework regions identified herein, preferably the framework scaffold comprises all the framework regions described herein (Table 7). All six CDRs of V_{H} and V_{L} are randomized using trinucleotide cassette mutagenesis (Virnekäs et al., 1994, *supra*; Kayushin et al. Nucleic Acids Res. 24:3748-3755 (1996); Knappik et al., 2000, *supra*, incorporated herein in their entirety by reference), which leads to high-quality libraries.

To generate the master gene depicted in Table 7, comprising a framework scaffold for producing for high level accumulation, amino acid sequences from variable domains of chicken immunoglobulins from Kabat and disclosed herein were collected and analyzed. The sequences were incorporated into two databases, respectively for the V_{H} and V_{L} and aligned according to the Kabat numbering system. Sequences that were more than 70% incomplete were not included in the databases. The variability for common amino acid residue at each position was calculated according to Kabat et al. for both the V_{H} and V_{L} sequences. Within each database the sequences were grouped according to the loop length of the six CDR regions (respectively H1 H2, H3 for the VH database and L1, L2 and L3 for the VL database) to give classes of loop length. The frequency of occurrences of each loop length is calculated by dividing the number of sequences composing the loop length class of each database by the total number of sequences included in the database. Finally, HCDR and LCDR cassettes were designed such that the naturally occurring diversity in terms of amino acid residue variability and loop length was well represented.

The CDR cassettes are introduced into the master gene by PCR techniques to generate a semi-synthetic chicken antibody library based on the framework regions of the master gene depicted in Table 7.

Differences in the CDRs after randomization and selection of immunoglobulin molecules having a particular specificity may influence the range of expression yields seen with the master gene, however, the framework scaffold described herein enhances the accumulation of the immunoglobulin molecules and reduce the large imbalances in the display efficiencies.

In Table 7 the chicken heavy and light chain variable region numbering is according to Kabat numbering. The regions enclosed within boxes are six CDRs the in heavy chain variable domain and the light chain variable domain. Their length can be varied, and amino acid residue composition can be fully randomized. A "#" corresponds to the positions with or without amino acid residues, depending on the CDR length variation. Residues identified by a number followed by a capital letter are not required in the framework region. Immunoglobulin molecules comprising all, a subset or none of the residues so marked are produced at high levels. Thus these immunoglobulin molecules are also part of this invention as are the nucleic acid molecules which encode the molecules, the vectors comprising the nucleic acid molecules, the host cells and plants transformed with the vectors and nucleic acid molecules expressing the immunoglobulin molecules.

In the heavy chain framework regions (Table 7, SEQ ID NO: 15), residues at position 66, 104 and 45 are critical for antibody stability and folding. Residue 66 in the V_{H} domain must be Arginine (R); residue 104 in V_{H} domain must be Tryptophan (W); and residue 45 in V_{H} fragment can only be Leucine (Leu) or Proline (Pro). In the light chain framework regions (Table 7, SEQ ID NO: 16), residues at position 44 and 98 are critical for antibody stability and folding. Residue 98 in the V_{L} domain must be Phenylalanine (Phe) and residue 44 must be Proline or Leucine (Leu). In the linker region (Table 7, SEQ ID NO: 17), the second Proline (Pro) can be replaced with Serine (S). The amino acid residue at position 39A of the light chain variable region (Table 7) is not present in chicken antibody sequences derived from the Kabat database.

The orientation of the V_{L} and V_{H} domains can be V_{H}-linker-V_{L} as shown in Table 7, or may be V_{L}-linker-V_{H}.

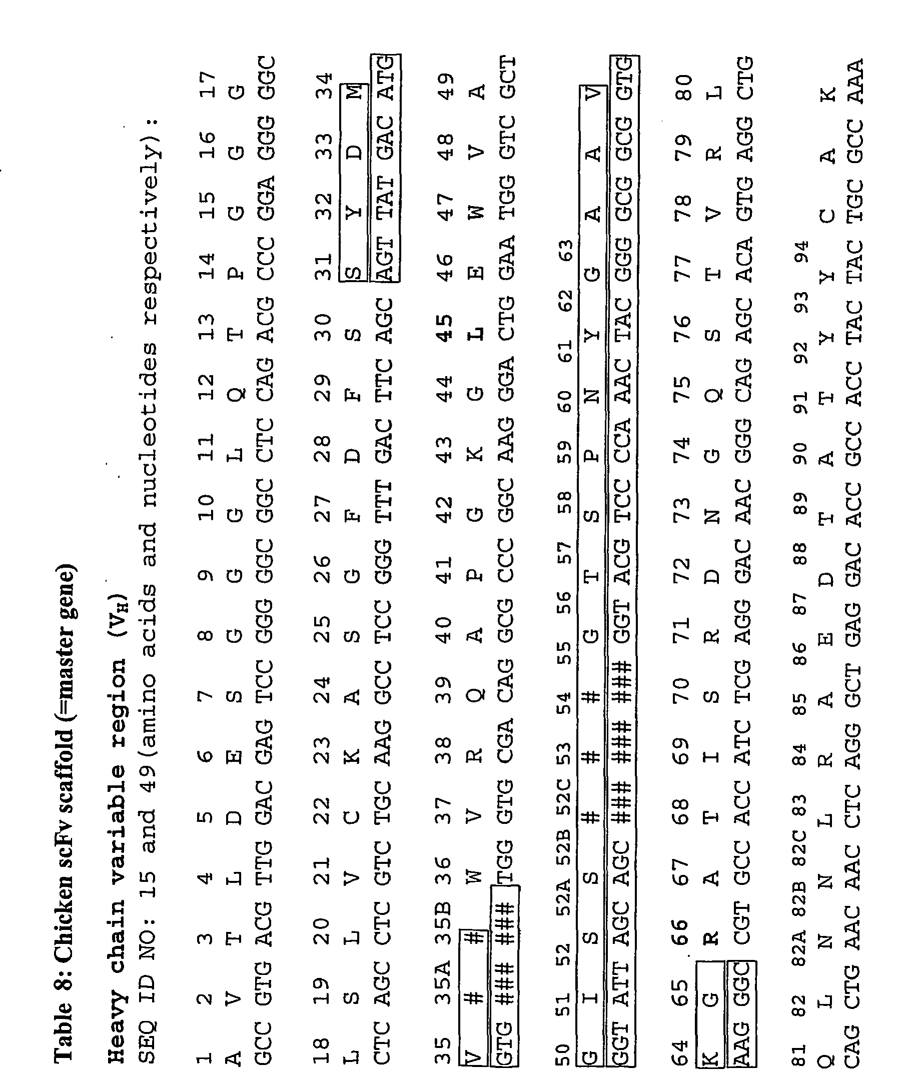

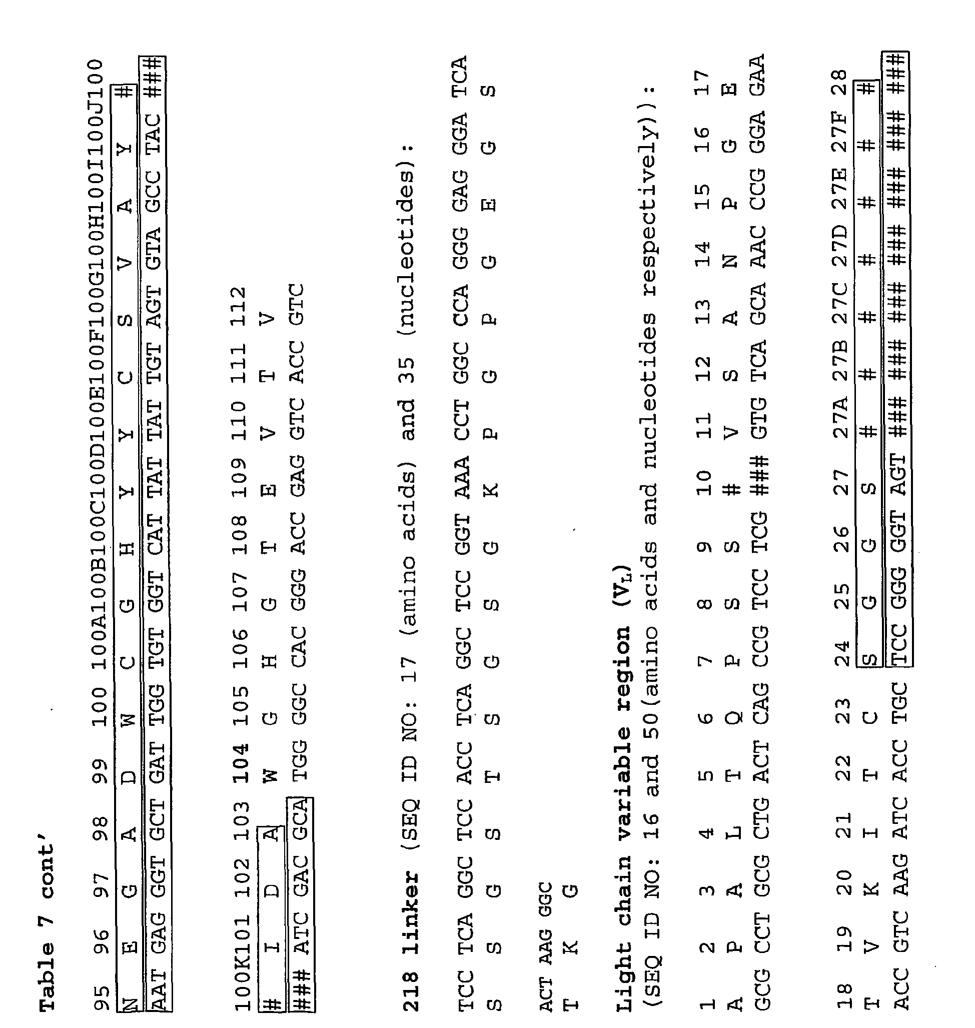

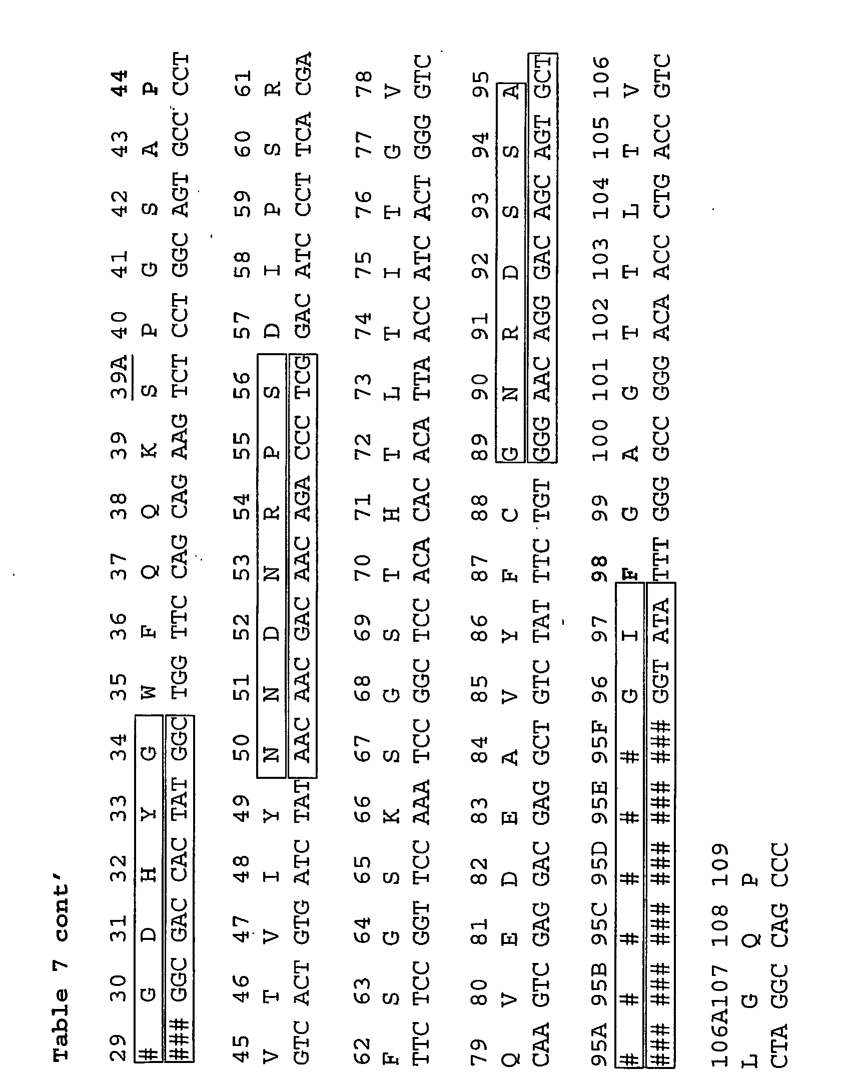

### EXAMPLE 8 - CONSTRUCTION AND CHARACTERIZATION OF AN SCFV ANTIBODY WITH CHIMERIC CHICKEN-MURINE VARIABLE DOMAINS

Described herein is the construction of an immunoglobulin molecule, particularly a scFv antibody, having chimeric variable domains which combine features of two or more parental antibodies into one immunoglobulin molecule. Preferably, the immunoglobulin molecule having the chimeric variable domains displays the antigen binding properties of one parent and has the stability of the other parent. Such molecules differ from "chimeric antibodies" wherein the variable domain of an antibody is replaced essentially in its entirety with the variable domain from another parent antibody.

When humans are treated with murine antibodies for therapeutic purposes, immunogenic responses known as the Human Anti-Mouse Antibodies (HAMA) responses are generated (Schroff, R. et al., Human anti-murine immunoglobulin responses in patients receiving monoclonal antibody therapy Cancer Research 45: 879-885 (1985); Shawler, D. L. et al., Human immune response to multiple injections of murine monoclonal IgG. J. Immunol. 135: 1530-1535 (1985)). Chimeric antibodies, wherein the murine antibody is replaced with the constant region human antibody, have been generated e.g., to humanize antibodies of murine origin (Morrison, S. L. et al., Chimeric human antibody molecules: mouse antigen-binding domains with human constant region domains. Proc Natl Acad Sci 81: 6851- 6855 (1984)). Humanization of murine antibodies is of great interest as this technique can significantly reduce or completely abolish the HAMA responses. However, in many cases, chimeric antibodies do not show a significant reduction of the HAMA responses. In fact, some amino acid residues of murine origin that are located in the variable regions are sufficient to generate an immune response in humans. Several studies showed that alternative approaches can be used to generate humanized versions of murine or other antibodies, such as those reported by Riechmann, L.M. et al., Reshaping human antibodies for therapy. Nature 332: 323- 327 (1988); Jones, P. T. et al., Replacing the complementarity-determining regions in a human antibody with those from a mouse. Nature 321: 522-525 (1986); Looney, J. E. et al., High level expression and characterization of a mouse-human chimeric CD4 antibody with therapeutic potential. Hum Antib Hybridomas 3:191-200 (1992); Winter G., and Harris W.J., Humanized antibodies Trends Pharmacol Sci 14: 139-143 (1993); Roguska MA. et al., Humanization of murine monoclonal antibodies through variable domain resurfacing Proc Natl Acad Sci 91: 969-973 (1994); Baca M. et al., Antibody Humanization Using Monovalent Phage Display J Biol Chem 272 16: 10678-10684 (1997), Nakamura et al. Dissection and optimization of immune effector functions of humanized anti-ganglioside GM2 monoclonal antibody Mol Immunol 37: 1035-1046 (2000), all incorporated herein by reference.

As a result of these research efforts, humanization of antibodies of non-human origin is now carried out by a technique known to those skilled in the art as CDR-grafting or CDR-reshaping. CDR-grafting consists essentially in the grafting of the CDRs of antibodies of mouse or other origin that show peculiar binding properties against a specific antigen, onto the variable framework regions of a human antibody selected on the basis of particular features, such as high frequency of occurrence in the human antibody repertoire or higher stability. However, the grafting of the CDRs of murine or other origin onto the variable regions of human antibodies is in many cases not sufficient to retrieve the binding properties of the parental antibody that donates the CDRs. Therefore, additional human amino acid residues located in the variable FRs have to be replaced with the corresponding amino acid of murine or other origin. The result is a recombinant antibody with chimeric variable domains.

Antibodies with chimeric variable domains can be generated to display the binding properties of pre-selected antibodies by combinatorial approaches, e.g., a technique known to those skilled in the art as CDR-randomization or CDR-shuffling.

The chicken antibody scaffold disclosed in the present invention is a suitable tool for the techniques described above, as it can be used as a donor of frameworks having desirable characteristics, such as higher protein stability in plant, bacteria, yeast, human or animal cells. Also included in this invention is the use of the chicken scFv scaffold of the present invention in its entirety or parts of the scaffold, e.g., the individual framework regions alone or in combination, short sequences or motifs thereof, to conferring desirable features suitable to the purpose disclosed above, e.g., enhanced stability in plant cells and more preferably the cytosol of plant cells, bacteria and more preferably the bacterial periplasm or cytosol, yeast, human or animal cells.

Described hereinafter is a method to generate immunoglobulin molecules with chimeric variable domains, preferably scFv antibodies with chimeric variable domains. Preferably the chimeric variable domain is a chicken-murine or chicken-human variable domain. The CDRs of the chimeric variable domain are those of a "donor" immunoglobulin molecule and the framework regions are those of a chicken immunoglobulin and wherein particular amino acid residues in the framework regions are replaced such that the immunoglobulin molecule has the binding and affinity of the CDRs of the "donor." The assignment of an amino acid residue to a particular CDR or framework regions, as well as the position of amino acid residues within those regions, as described herein are in accordance with Kabat (Kabat et al., Sequences of Proteins of Immunological Interest 5th Edition NID (1991); Martin, "Accessing the Kabat Antibody Sequence Database by Computer." PROTEINS: Structure, Function and Genetics 25: 130-133(1996). The immunoglobulin molecule with a chimeric variable domain is derived from two partners:
an avian partner, which in this example is a chicken scFv scaffold, preferably the scFvG19 (SEQ ID NO: 51) described in the present invention that has been shown to accumulate to very high level in the cytosol of plant cells (see Tables 4 and 5), and a
a donor partner, which may be a preselected immunoglobulin molecule from another species, e.g., mouse, rat, horse, cow, sheep, pig, monkey or human. In the particular example which follows the donor partner is a murine scFv antibody scFv29, which is described by Schillberg et al., Apoplastic and cytosolic expression of full-size antibodies and antibody fragments in Nicotiana tabacum. Transgenic Res 8: 255-263 (1999) incorporated herein by reference. The scFv29, generated from the parental murine monoclonal antibody 29 (rAb29), specifically binds to monomers of tobacco mosaic virus (TMV) coat protein but as reported by Schillberg et al. (1999), is poorly expressed in the cytosol of tobacco plants.

The scFv antibody with chimeric chicken-murine variable domains, in the following example is hereinafter referred to as the scFvG19-29, and is generated through CDR-grafting approach enriched with information on antibody structure derived from studies of antibody engineering. One or more amino acid residues located in the framework regions of the chicken scaffold, scFvG19, are replaced with the amino acid residue present in the corresponding position in the murine scFv29 to preserve loop conformation and binding properties of the parental murine CDRs.

### A. Sequence analysis of the murine scFv29 and the chicken scaffold scFvG19

To construct a scFv with a chimeric variable domain, such as scFvG19-29 of this invention, the amino acid sequences of the chicken scFv antibody and the preselected donor scFv, e.g., an avian, piscean, mammalian, e.g., camelid, murine or human, scFv antibody donor are aligned and compared. The analysis of the amino acid sequences of the two proteins identifies the major differences between the chicken scFv antibody scaffold and the donor scFv. In particular, for a murine or human donor this comparison focuses on positions that are occupied by highly conserved amino acid residues in human and mouse antibodies. Moreover, attention is devoted to amino acid residues that play a key role in the classification of the CDR loop length and in the assignment to loop conformations known to those skilled in the art as canonical structures. The amino acid residues that comprise the "Vernier zone" (Foote J, Winter G. Antibody framework residues affecting the conformation of the hypervariable loops. J Mol Biol 224: 487-499 (1992) incorporated herein by reference) and those located at the interface of the variable Heavy and Light chain are also compared. The amino acid residues which are at positions within chicken and donor FRs which play a key role in maintaining antibody structure and biological function are compared. If the amino acid residues in the chicken and donor antibodies differ, they are analyzed to determine if the amino acid residue present in the chicken antibody should be replaced by the amino acid residue in the corresponding position in the murine antibody. Below is an example of this method wherein scFvG19 is the chicken scFv and scFv29 is the murine donor scFv used to design scFv's having chimeric variable domains which bind the antigen bound by the parental scFv29.

### 1. Amino acid sequence alignment of the scFv29 and the scFvG19

The variable domains of the heavy and light chain amino acid sequences are aligned to identify main differences between scFv29 (29) (SEQ ID NO: 63 and 64) and scFvG19 (G19)(amino acid residues 1-127 of SEQ ID NO: 51 and amino acid residues 145-250 of SEQ ID NO: 51 respectively). In bold and underlined are the CDRs:

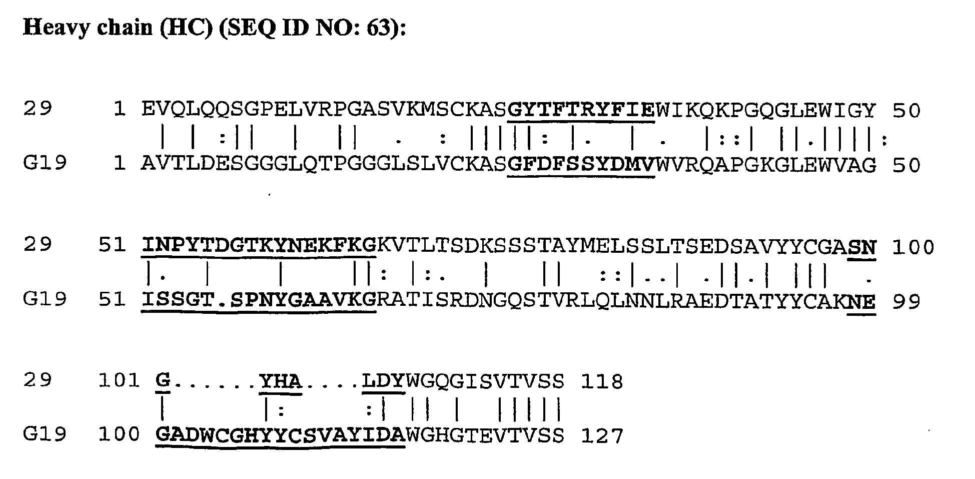

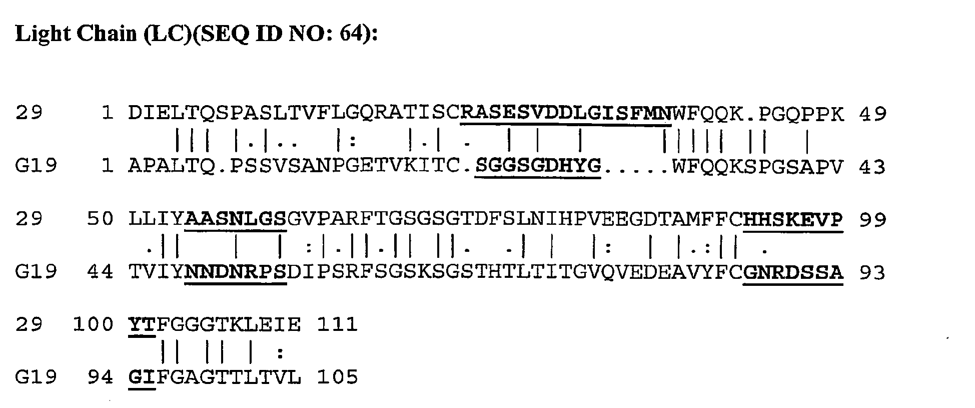

The alignment of the scFv29 heavy and light chain variable domains and the scFvG19 heavy and light chain variable domain shows the following results (Table A):

| **Table A** | | | | | |
|---|---|---|---|---|---|
| Identity HC | Identity LC | Identity HC+LC | Identity FR-HC | Identity FR-LC | Identity FR-HC+LC |
| 42.7% | 40.3% | 41.5% | 47.1% | 46.8% | 46.9% |

The HC of the scFvG19 has a higher identity with the scFv29 than the LC. The scFvG19 has a λ light chain whereas the scFv29 has a κ light chain.

### 2. Analysis of highly conserved amino acid residues

The amino acid residues in Table B are those in the FRs of the HC and LC, that are identified by Kabat et al. (1991) *supra* and by Chothia et al. (Structural determininats in the sequences of immunoglobulin variable domain, J. Mol. Biol. 278:457-470 (1998) incorporated herein by reference) as highly conserved, or more precisely as invariant amino acid residues, i.e. with a 95% or higher frequency of occurrence in the available antibody repertoire of variable human and mouse antibodies (in the following tables the Kabat numbering system is used):

| **Table B** | | | | | | | | |
|---|---|---|---|---|---|---|---|---|
| Position | Conserved residue | scFv29 | scFvG19 | | Position | Conserved residue | scFv29 | scFvG19 |
| H22 | C | C | C | | L23 | C | C | C |
| H25 | S | S | S | | L35 | W | W | W |
| H36 | W | W | W | | L49 | Y | Y | Y |
| H49 | G | G | **A** | | L57 | G | G | **D** |
| H66 | R | **K** | R | | L88 | C | C | C |
| H92 | C | C | C | | L98 | F | F | F |
| H103 | W | W | W | | L99 | G | G | G |

The highly conserved amino acid residues in human and murine antibodies are all found in scFv29 with the exception of a Lys instead of an Arg at position H66 (highlighted in gray). The majority of the amino acid residues highly conserved in human and mouse antibodies are also present in the FRs of the chicken scFvG19 with the exception of Gly/Ala in H49 and Gly/Asp in L57. The differences between the scFv29 and the scFvG19 are in H49, H66 and L57 (bold and underlined amino acid residues). In H49 and H66, Ala and Arg are highly conserved in chicken antibodies. The substitutions Gly/Ala and Lys/Arg are conservative substitutions in proteins and the positions H46 and H66 in the chicken framework are not included in the set of positions wherein the amino acid residue is replaced by the corresponding amino acid residue of the murine antibody to generate the chimeric variable domain.

At L57 Asp and Asn are frequently encountered in chicken antibodies whereas Gly (present in the murine scFv29) occurs only at a very low frequency (ca. 2.5%). Therefore, substitution of the Asp at position L57 with the murine Gly is not made to produce the chimeras.

### 3. Classification of the CDR loops

To identify all the amino acid residues that are implicated in the function of the six CDR loops in the scFv antibodies we used the CDR classification based on the Kabat numbering system, as well as the classification proposed by Chothia and the Contact classifications (described *infra*). Chothia and the Contact classification identify differences between amino acid residues which according to Kabat are assigned to the framework regions, but are also implicated in the function of the CDR loops.

### CHOTHIA'S NUMBERING SYSTEM

The Chothia numbering system (Chothia, C., Lesk, A.M.,. Canonical structure for the hypervariable regions of immunoglobulins. J Mol Biol 196, 901-917 (1987); Chothia, et al., Conformations of immunoglobulin hypervariable regions. Nature 342, 877-883 (1989); Al-Lazikani B. et al., Standard conformations for the canonical structures of immunoglobulins J Mol Biol 273: 927-948 (1997) all incorporated by reference) is very similar to the Kabat's numbering system. The main differences between the two numbering systems are in the CDR-H1 and -L1 loops. Chothia assigns the amino acid residue insertions to the correct structural position based on solved antibody 3D-structures. In addition, the CDR-H1 loop proposed by Chothia is larger than the one described by Kabat. Therefore, the amino acid residues at the following positions in Table C are compared:

| **Table C** | | | |
|---|---|---|---|
| CDR | Position (Kabat) | scFv29 | scFvG19 |
| H1 | H26 | G | G |
| | H27 | **Y** | **F** |
| | H28 | **T** | **D** |
| | H29 | F | F |
| | H30 | **T** | **S** |

### Contact:

The contact numbering system proposed by MacCallum, et al., Antibody-antigen interactions: Contact analysis and binding site topography. J Mol Biol 262: 732-745 (1996), incorporated herein by reference, considers amino acid residues (see Table D) assigned to the in the framework regions by the Kabat classification that have been frequently reported to make contact with the antigen:

| **Table D** | | | |
|---|---|---|---|
| CDR | Position (Kabat) | scFv29 | scFvG19 |
| H1 | H30 | **T** | **S** |
| H2 | H47 | W | W |
| | H48 | **I** | **V** |
| | H49 | **G** | **A** |
| H3 | H93 | **G** | **A** |
| | H94 | **A** | **K** |
| L1 | L35 | W | W |
| | L36 | F | F |
| L2 | L46 | **L** | **T** |
| | L47 | **L** | **V** |

The analysis of the CDR loops with Kabat, Chothia and the Contact methods of loop classification identifies amino acid residues of the chicken FRs (bold and underlined in Tables C and D) which differ from the amino acid residue in the corresponding position in the murine antibody. These amino acid residues are included in the set that are replaced by the corresponding amino acid residue in the murine antibody in generating the chimera. While the foregoing analysis compared the chicken framework to a murine derived scFv, the same analysis may be applied to any immunoglobulin molecule that serves a donor of the CDRs to the chicken scFv.

The Chothia numbering system includes the amino acid residues in position H28-H30 in the CDR-H1 loop. The Contact classification indicates that the amino acid residues in these positions are structurally important. Therefore, these two positions, which are in the framework regions according to Kabat's system, are included among the one or more positions in the chicken frameworks that are replaced by the amino acid residue in the corresponding position in the murine scFv.

The Contact classification indicates that the amino acid residue in H48 and H49 (which are in the framework region according to Kabat, but are part of the CDR-H2 loop according to the Contact classification) can also influence the biological properties of the scFv having the chimeric variable domains. These amino acid residues as well as those at H93 (Gly/Ala) and H94 (Ala/Lys), which are involved in the conformation of the CDR-H3 loop, are included in the one or more amino acid residues that are replaced by the amino acid residue in the corresponding position in the murine scFv.

In the light chain differences occur in L46 (Leu/Thr) and L47 (Leu/Val) of the CDR-L2 loop (Contact classification). In L46, Thr is highly conserved in chicken antibodies (96% frequency of occurrence) whereas Leu never found. In L47, Val has a 25% frequency of occurrence in chicken antibodies, moreover the substitution Leu/Val is considered to be conservative in proteins. L46 and L47 are involved in the contact to the antigen and thus these positions are also included in the one or more amino acid residues in the chicken framework regions (in accordance with the Kabat classification system) that are replaced with the amino acid residue in the corresponding position in the murine scFv.

### 4. Canonical class assignment

### Heavy Chain:

### CDR-H1:

According to Kabat the length of the CDR-H1 of the scFv29 and scFvG19 is 5 amino acid residues. This corresponds the canonical class 5-1. The key residues for the canonical class 5-1 are summarized in Table E where amino acid residues located in the FRs are highlighted in grey (H24, H26, H27, H29, H94):

| **Table E** | | | |
|---|---|---|---|
| Position (Kabat) | Allowed amino acid | scFv29 | scFvG19 |
| H24 | A,V,G | A | A |
| H26 | G | G | G |
| H27 | F,Y | **Y** | **F** |
| H29 | F | F | F |
| H34 | M,W,I | **I** | **M** |
| H94 | R,K | **A** | **K** |

scFv29 cannot be assigned to the canonical class 5-1 due to the presence of an Ala in position H94 instead of Arg or Lys. In contrast, scFvG19 contains a CDR-H1 loop that belongs to the canonical class 5-1.

The amino acid residues (bold and underlined) in position H27 (Tyr/Phe) and H94 (Ala/Lys) have to be of murine origin, i.e., Y and A respectively to preserve the loop conformation of scFv29 in the scFvG19-29. If Tyr and Ala are present at positions H27 and H94 in scFvG19-29, as in the donor scFv29, scFvG19-29 will not fit into any described canonical structure for the CDR-H1 loop.

### CDR-H2:

According to Kabat the CDR- H2 loop of the scFv29 has a length of 17 amino acid residues and is assigned to the canonical classes (CC) 17-2 or 17-3. scFvG19 has a loop of 16 amino acid residues and is assigned to the canonical class 16-1.

Key residues for the canonical class 17-2, 17-3 and 16-1 are summarized in Table F where amino acid residues in the FRs are highlighted in grey:

| **Table F** | | | | | |
|---|---|---|---|---|---|
| Position (Kabat) | Allowed amino acid amino for the CC 17-2 | Allowed acid for the CC 17-3 | scFv29 | Allowed amino acid for the CC 16-1 | scFvG19 |
| H52 | P,T,A | | N | | S |
| H54 | | G,S,N | T | | G |
| H55 | G,S | | D | G,D | T |
| H71 | A,T,L | R | **S** | V,K,R | **R** |

The CDR-H2 loop of scFv29 and scFvG19 lack key amino acid residues and thus can not be assigned to the canonical class 17-2 or 17-3, or the canonical class 16-1, respectively. Since the CDRs in the chimeric variable domains of the scFv are of murine origin, the main differences between the scFv29 and the scFvG19 is in H71 where Ser is present in the former and Arg in the latter (in bold and underlined). Thus H71 is included in the set of positions that are replaced by the amino acid residue in the corresponding position in the murine antibody. As Arg in H71 is highly conserved in chicken antibodies, an alternative to the replacement of the amino acid residue is to generate a nucleic acid molecule encoding the chimera wherein a degenerate codon is inserted to encode Ser/Arg at H71 to produce a mix of chimeras having either Ser or Arg at position H71.

### CDR-H3:

The classification of the CDR-H3 is very complex. However, to some extent the residues in Table G influence loop conformation (Morea V. et al., Conformations of the third hypervariable region in the VH domain of immunoglobulins. J Mol Biol 275(2): 269-294 (1998); Shirai H. et al., Structural classification of CDR-H3 in antibodies FEBS Letters 399: 1-8 (1996); Shirai H. et al., H3-rules: Identification of CDR-H3 structures in antibodies FEBS Letters 455: 188-197 (1999)) (in grey, H94 and H103, are residues in the FRs: Table G):

| **Table G** | | |
|---|---|---|
| Position (Kabat) | scFv29 | scFvG19 |
| H94 | **A** | **K** |
| H100K or equivalent | L | I |
| H101 | D | D |
| H103 | W | W |

The scFv29 contains a CDR-H3 loop of 9 amino acid residues. According to the rules on loop conformation of Shirai et al. and Morea et al., this CDR loop should be torso bulged or kinked since the Asp in H101 and the Trp in H103 should form a hydrogen bond. ScFvG19 shows a large 19 amino acid residue CDR-H3 loop. The presence of Lys in H94 and Asp in H101 should be sufficient to favor the formation of a torso-bulged or kinked loop structure.

Another difference between the scFv29 and the scFvG19 is in H94 (Ala/Lys bold and underlined). H94 can influence the conformation of the CDR-H3 and CDR-H1 loops and therefore the amino acid residue at H94 is included in the set of amino acid residues that are replaced with the amino acid residue in the corresponding position in the murine antibody.

In conclusion the scFv29 shows a quite atypical loop conformation for the three heavy chain CDRs. These atypical CDR conformations are maintained in the chimeras generated by the methods of this invention, as summarized in the following Table H:

| **Table H** | | |
|---|---|---|
| CDR | Canonical Class assignment | |
| | scFv29 | scFvG19-29 |
| H1 | Non classificable | Non classificable |
| H2 | Non classificable | Non classificable |
| H3 | putative torso-bulged or kinked | putative torso-bulged or kinked |

### Light Chain:

### CDR-L1:

According to Kabat scFv29 has a 15 amino acid residue CDR-L1 loop whilst scFvG19 has a CDR-L1 loop of 9 amino acid residues. ScFv29 is assigned to the canonical class 15-4 whereas no canonical structures are described so far for a CDR-L1 loop of 9 amino acid residues. The scFvG19-29 has the CDR-L1 loop of scFv29 and thus a putative canonical class 15-4.

The key residues for the assignment to the canonical class 15-4 are summarized in Table I where amino acid residues located in the FRs are highlighted in grey (L2 and L71):

| **Table I** | | | |
|---|---|---|---|
| Position (Kabat) | Allowed amino acid | scFv29 | scFvG19 |
| L2 | I | I | **P** |
| L25 | A | A | G |
| L27B | V | V | - |
| L33 | M | M | Y |
| L71 | F | F | **H** |

The CDR-L1 of the scFv29 can be assigned to the canonical class 15-4. The main differences between the scFvG19 and the scFv29 are in L2 (Pro/Ile) and L71 (His/Phe). The differences in L2 and L71 (bold and underlined) are important to maintain the CDR-L1 loop conformation of the scFv29. Thus the amino acid residues at positions L2 and L71 are included in the set of amino acid residues that are substituted by the amino acid residue in the corresponding position in the murine antibody to preserve the CDR-L1 loop.

### CDR-L2:

According to Kabat the scFv29 and the scFvG19 show a CDR-L2 length of 7 amino acid residues and can be assigned to the canonical class 7-1. Key residues for the canonical class 7-1 are summarized in the following Table J where amino acid residues within the FRs are highlighted in grey (L48 and L64):

| **Table J** | | | |
|---|---|---|---|
| Position (Kabat) | Allowed amino acid | scFv29 | scFvG19 |
| L48 | I | I | I |
| L51 | A,T | A | **N** |
| L52 | S,T | S | **D** |
| L64 | G | G | G |

The CDR-L2 of scFv29 is assigned to the canonical class 7-1, whereas scFvG19 shows significant amino acid residue differences in L51 and L52 (bold and underlined) that do not allow the assignment to the canonical class 7-1. However, the chimeric variable domain of scFvG19-29 has the loop conformation of the parental scFv29 as L51 and L52 are derived from the scFv29 and no differences between scFv29 and scFvG19 occur at L48 and L64.

### CDR-L3:

According to Kabat the scFv29 and the scFvG19 show a CDR-L3 loop of 9 amino acid residues and can be assigned to the canonical class 9-1 or 9-2. Key amino acid residues for the canonical class 9-1 and 9-2 are summarized in Table K:

| **Table K** | | | | |
|---|---|---|---|---|
| Position (Kabat) | Allowed amino acid for the CC 9-1 | Allowed amino acid for the CC 9-2 | scFv29 | G19 |
| L90 | Q,N,H | Q | H | N |
| L94 | | P | V | **S** |
| L95 | P | | P | **A** |

ScFv29 shows a CDR-L3 loop of the canonical class 9-1 as His and Pro are present in L90 and L95, respectively. Although scFvG19 shows no canonical structure for the CDR-L3 loop (bold and underlined differences in L94 and L95), scFvG19-29 has the canonical class 9-1 as the key amino acid residues for this canonical class are located within the CDR-L3 that is entirely derived from the scFv29.

In summary the canonical class assignment for the light chain of scFv29 and scFvG19-29 are depicted in Table L:

| **Table L** | | |
|---|---|---|
| CDR | Canonical Class assignment | |
| | scFv29 | scFvG19-29 |
| L1 | 15-4 | Non classificable |
| L2 | 7-1 | 7-1 |
| L3 | 9-1 | 9-1 |

### 5. Analysis of the residues at the VH/VL interface

The amino acid residues in Table M have been reported to play an important role in the interaction between Vh and Vl and are located at the interface of the heavy and light chain of the folded scFv antibody molecule. The analysis of the amino acid residues located at the Vh-Vl interface of the scFv29, scFvG19 and scFvG19-29 showed the following results (Table M) :

| **Table M** | | | | |
|---|---|---|---|---|
| Position | Conserved amino acid at the VH/VL interface | scFv29 | scFvG19 | scFvG19-29 |
| H35 | H,N,S,E,G,A | E | V | E |
| H37 | V,I,L | I | V | V |
| H39 | Q,K,R | Q | Q | Q |
| H45 | L,P | L | L | L |
| H47 | W,L,Y | W | W | W |
| H91 | Y,F | Y | Y | Y |
| H93 | A,T,V | **G** | A | **A** |
| H95 | Y,D,G,S,R,E | S | N | S |
| H100K | F,M,L | L | I | L |
| H103 | W | W | W | W |
| L34 | A,H,N,Y,E,D | **N** | **G** | **N** |
| L36 | Y,F,L | F | F | F |
| L38 | Q,H | Q | Q | Q |
| L44 | P,V,I,L | P | P | P |
| L46 | L,R,P,V | **L** | T | **T** |
| L87 | Y,F | F | F | F |
| L89 | Q,L,F,M | H | G | H |
| L91 | W,S,Y,G,H,R,A | S | R | S |
| L96 | L,Y,W,R,F,I | Y | G | Y |
| L98 | F | F | F | F |

The six core residues at the Vh/Vl interface are highlighted in grey (H45, H100K, H103, L44, L96 and L98). In the scFvG19-29 these residues do not differ from those highly conserved at the Vh/Vl interface in human and mouse antibodies. The core residues of the scFv29 and the scFvG19 differ in position H100K (Leu/Ile) and L96 (Tyr/Gly). However, H100K and L96 are within the CDR-H3 and CDR-L3 loops, respectively. As the CDR loops of scFvG19-29 are entirely derived from scFv29, no difference will occur in these positions.

Few additional differences between the scFv29 and the scFvG19-29 are encountered at the Vh/Vl interface (bold and underlined):
- In H37 Ile is present in the chicken framework while Val is present in the murine antibody, however both residues are highly conserved in mouse and human antibodies and should be mutually exchangeable with minor structural effects.
- In H93 Gly is not included in the list of the conserved residues at the VH-VL interface in human and mouse antibodies. Therefore, H93 is included in the set of amino acid residues that are replaced with the amino acid residue in the corresponding position in the murine antibody.
- In L46 Thr is found in the murine antibody and Leu is found in the chicken antibody. Thr may replace Leu in the chimeras of scFvG19 and scFv29. Leu is one of the four conserved residues in mouse and human antibodies. Moreover L46 is one of the contact residues. In chicken antibodies, L46 is never occupied by Leu whereas Thr is highly conserved. Therefore L46 is included in the set of amino acid residues that are replaced by the amino acid residue in the corresponding position in the murine antibody. Alternatively a nucleic acid molecule may be generated that encodes the chimera, wherein a degenerate codon for L/T at L46 may be incorporated into the nucleic acid molecule to encode a mix of antibodies having either Leu or Thr at position L46.

Additional differences between scFv29 and scFvG19 such as those in H35, H95, L34, L89, and L91 are all located in the CDR loops that are donated by scFv29. Thus, in these postions there are no differences between scFvG19-29 and scFv29.

### 6. Residues at the Vernier zone

At the "Vernier zone" (Foote J, Winter G. Antibody framework residues affecting the conformation of the hypervariable loops. J Mol Biol 224: 487-499 (1992)) are amino acid residues that can influence the conformation of the CDR loops. The following alignment of the light and heavy chain shows differences at the Vernier zones of scFv29 and scFvG19:

| **Table N** | | | | | | |
|---|---|---|---|---|---|---|
| Vernier zone | scFv29 | scFvG19 | | Vernier zone | scFv29 | scFvG19 |
| H2 | V | V | | L2 | **I** | **P** |
| H27 | Y | F | | L4 | L | L |
| H28 | **T** | **D** | | L35 | W | W |
| H29 | F | F | | L36 | F | F |
| H30 | **T** | **S** | | L46 | **L** | **T** |
| H47 | W | W | | L47 | **L** | **V** |
| H48 | **I** | **V** | | L48 | I | I |
| H49 | **G** | **A** | | L49 | Y | Y |
| H67 | **V** | **A** | | L64 | G | G |
| H69 | **L** | **I** | | L66 | **G** | **K** |
| H71 | **S** | **R** | | L68 | G | G |
| H73 | **K** | **N** | | L69 | **T** | **S** |
| H78 | **A** | **V** | | L71 | **F** | **H** |
| H93 | **G** | **A** | | L98 | F | F |
| H94 | **A** | **K** | | | | |
| H103 | W | W | | | | |

The amino acid residues comprising the Vernier zone are located in the FRs. Some of the amino acid residues in the Vernier zones of scFv29 and G19 differ and thus some are included in the set of amino acid residues that are replaced by the amino acid residue in the corresponding position in the murine antibody (amino acid residues in bold and underlined) to produce the scFvs having chimeric variable domains.

In the HC the differences in H28, H29, H30, H48, H49, are important. The residues H28 to H30 are part of the CDR-H1 according to the Chothia's numbering system and H48, H49 are involved in the contact to the antigen. Therefore, these position are included in the set of positions wherein the amino acid residue is replaced by the amino acid residue in the corresponding position in the murine antibody. In H69, Ile and Leu, respectively in the scFv29 and in the scFvG19, represent conservative substitutions with minor effects on structure. Therefore, H69 is not included in the set of positions for replacement.

The amino acid residues in H67 (scFv29), as well as in H73 and H78, play a minor structural role and thus these positions are not included in the set of amino acid residues for replacement.

In H71, Arg is a highly conserved residue in chicken antibodies. Thus this position is included in the set of positions wherein the amino acid residue is replaced with the amino acid residue in the corresponding position in the murine antibody. Alternatively, a nucleic acid molecule may be generated encoding the chimera wherein the codon encoding the amino acid residue at H71 may be a degenerate codon encoding S/R (see also the canonical class assignment for the CDR-H2).

H93 and H94 are also included in the set of positions wherein the amino acid residue is replaced with the amino acid residue in the corresponding position in the murine antibody. The amino acid residues in these positions are also important for the conformation of the CDR-H1 and CDR-H3 loop (see the Canonical class assignment for the CDR-H1 and CDR-H3).

In the light chain Pro L2 in the chicken is Ile in the murine antibody. Since the amino acid residue in L2 plays an important structural role in the conformation of the CDR-L1 loop (Petri S et al., N-terminal mutations in the anti-estradiol Fab 57-2 modify its hapten binding properties Prot Sci 9: 2547-2556 (2000)) this position is included in the set of positions wherein the amino acid residue is replaced with the amino acid residue in the corresponding murine antibody.

The amino acid residues in positions L46, L66, L69 and L71 were analyzed to determine if the amino acid residue should be replaced by the amino acid residue in the murine antibody. The results follow:
L46: Thr should be replaced by Leu since L46 is one of the contact residues
L66: Gly is rarely encountered in chicken antibodies and thus L66 is not included in the positions wherein the amino acid residue is replaced with the corresponding murine amino acid. However, in one embodiment, a nucleic acid molecule may be generated with a degenerate codon encoding K/G at L66 to produce an scFv antibody of this invention wherein either a Lys or a Gly occurs at position L66. .
L69: Thr is rarely encountered in chicken antibodies whereas Ser is highly conserved. Thus L69 is not included in the set of amino acid residue positions for replacement.
L71: Phe is never encountered in chicken antibodies. However, L71 is important for the conformation of the CDR-L1 loop and shows a quite high variability in chicken antibodies. Therefore, L71 is included in the set of positions wherein the amino acid residue is replaced by the amino acid residue in the corresponding position in the murine antibody. Alternatively a nucleic acid molecule encoding the chimera may be generated wherein a degenerate codon for H/F is inserted to encode a His or Phe at position L71.

### 7. Overlay of amino acid residues in selected positions and assignment

To design the scFvG19-29 with chimeric chicken-murine variable domains, two complimentary strategies can be pursued:
- semi-conservative: the CDRs of the scFv29 as defined by the three different numbering systems (Kabat, Chothia, Contact) are grafted in their entirety onto the chicken FRs, whereas those amino acid residues of the chicken FRs located at key positions are partially exchanged with murine ones. Preferably, one or more amino acid residues at positions from H27 to H30, H48, H49, H93, H94 of the variable heavy chain and L2, L46, L47, L71 of the variable light chain of the chicken scFvG19 FRs are exchanged with the corresponding amino acid residues of the CDR-donor antibody, e.g., an avian, piscean or mammalian, e.g., camelid, murine (e.g., the scFv29 of the present invention) or human antibody donor. Optionally one or more amino acid residues in position from H66 to H69, H78 of the variable heavy chain and L57, from L66 to L69, L87 of the variable light chain in the chicken scFvG19 FRs are exchanged with the corresponding amino acid residues of the CDR-donor antibody, e.g., an avian, piscean or mammalian, e.g., camelid, murine (e.g., the scFv29 of the present invention) or human antibody donor. The semi-conservative strategy represents a compromise between the need to restore the binding properties of the parental scFv29 on the one hand and the attempt to alter as little as possible the FRs of chicken origin on the other hand.
- combinatorial: the residues identified by the analysis above are exchanged in a combinatorial fashion, i.e. singularly or in combinations. The combinatorial strategy is less conservative in respect to the amino acid composition of the chicken FRs as the full set of amino acid residues that the analyses above identified as structurally determinant, are replaced by the amino acid residues in the corresponding positions in the donor antibody. E.g., one or more amino acid residues at positions from H27 to H30, H37, H48, H49, from H66 to H69, H71, H73, H78, H93, H94 of the variable heavy chain and L2, L46, L47, L57, from L66 to L69, L87, L71 of the variable light chain in the chicken scFvG19 FRs may be substituted by the corresponding amino acid residues in the CDR-donor antibody, e.g., an avian, piscean or mammalian, e.g., camelid, murine (e.g., the scFv29 of the present invention) or human antibody donor. Ultimately, the combinatorial strategy generates a small library of scFvs having chimeric variable domains. The scFvs of the library are employed in screening assays, known to those skilled in the art, with the antigen recognized by the parental antibody that donates the CDRs. The aim of these assays is to select one or more scFvs with variable chimeric domains that show binding affinities comparable to or higher than the one shown by the CDR-donor antibodies

In the example hereinafter, a scFv antibody with a chimeric chicken-murine variable region is generated according to the semi-conservative strategy. This scFv is referred to as scFvG19-29.

The amino acid residues of the FRs located in positions that strongly influence antibody structure and function are first overlaid in two lists, one for the heavy chain and one for the light chain (Table O):

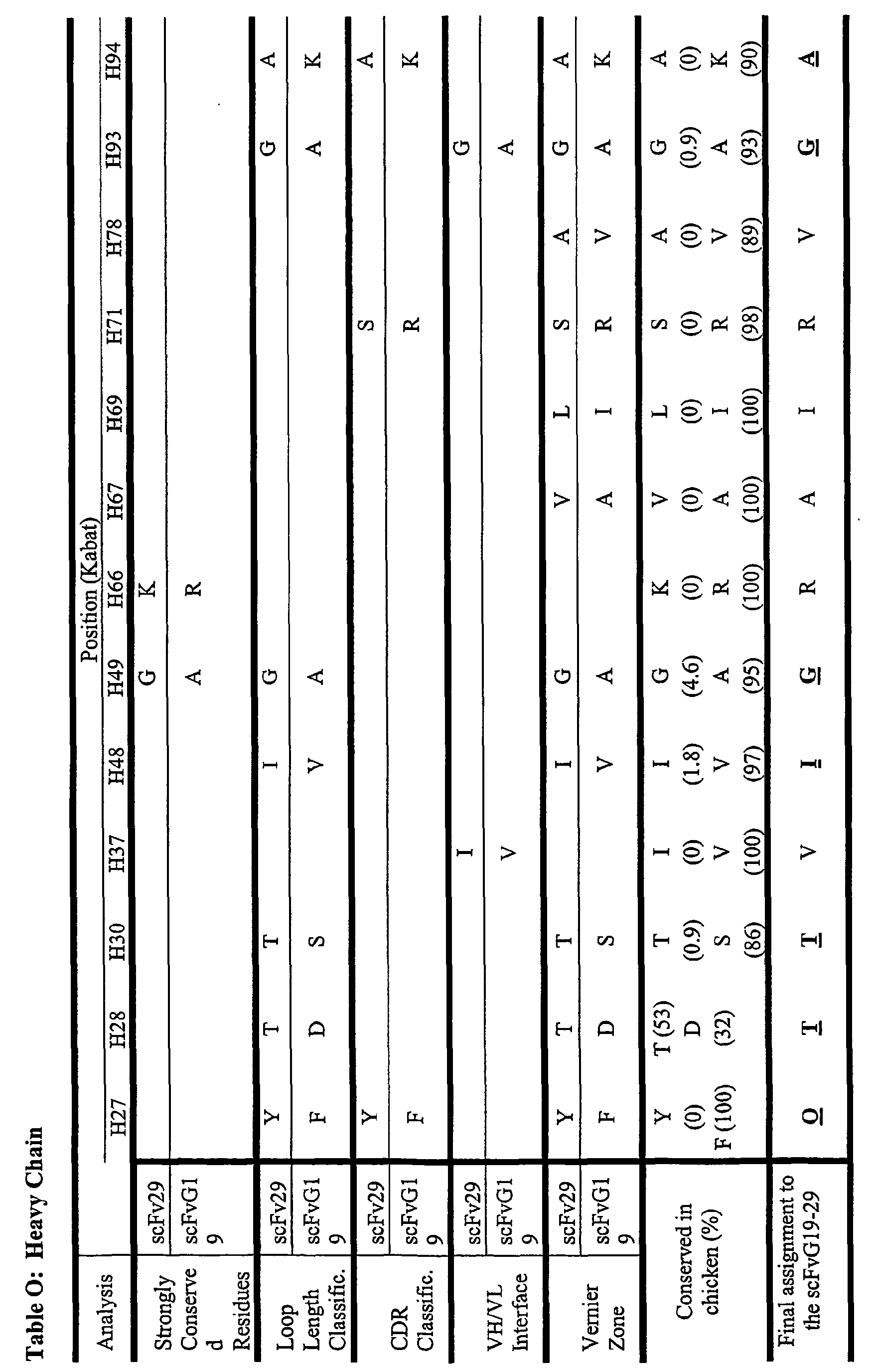

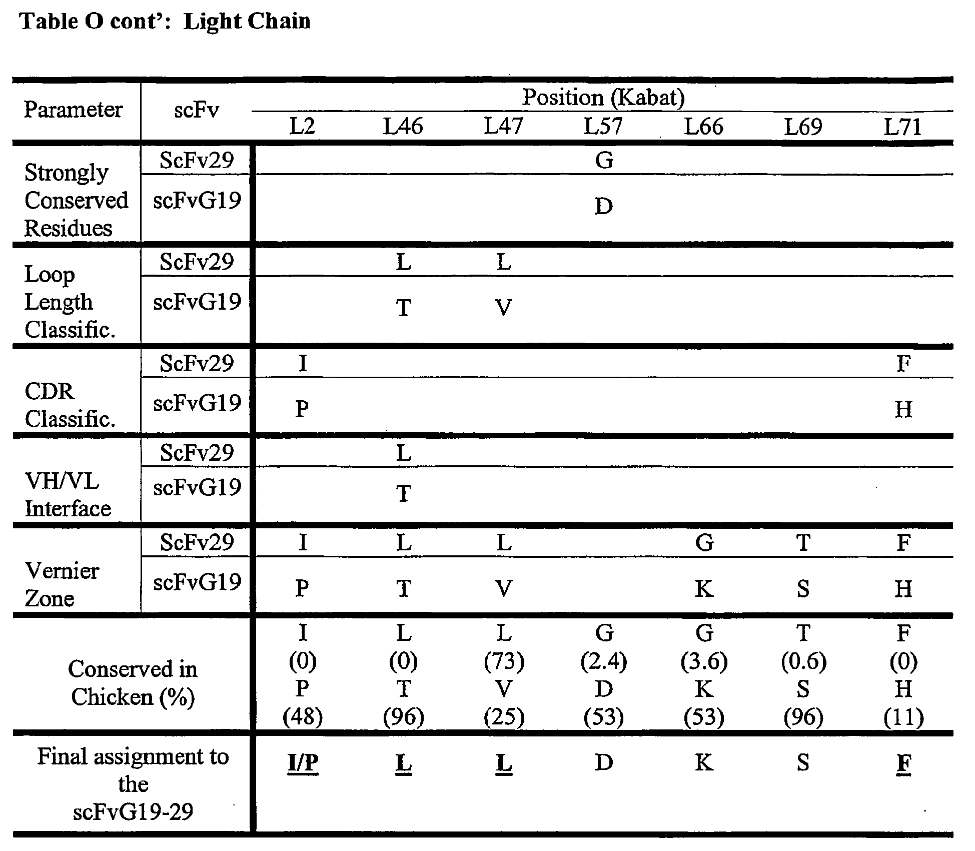

The positions in the chicken framework regions wherein the amino acid residue is replaced with the amino acid residue in the corresponding position in the murine antibody are underlined and in bold.

To assign murine or chicken amino acid residues at the key positions the following criteria are used:
a) Priority: priority is given to the results of the alternative loop length classifications of the CDRs followed by the result of the canonical class assignment, since both analysis put emphasis on loop conformation.
b) Frequency: the decision to replace an amino acid residue in the chicken framework with the corresponding amino acid residue in the murine antibody is made by taking into account how often amino acid residues occurring in a particular position are indicated to be crucial to structure by the different comparisons.
c) Occurrence in chicken Abs: it is considered to what extent a particular amino acid residue in a crucial position is conserved in chicken antibodies. If the amino acid residue in the chicken antibody is highly conserved while the corresponding amino acid residue in the murine antibody is not conserved in chicken antibodies, the chicken amino acid residue is not replaced. However, exceptions may be made for those amino acid residues which by the criteria of priority and frequency should be replaced.

### B. Generation of the dummy DNA sequence of scFvG19-29

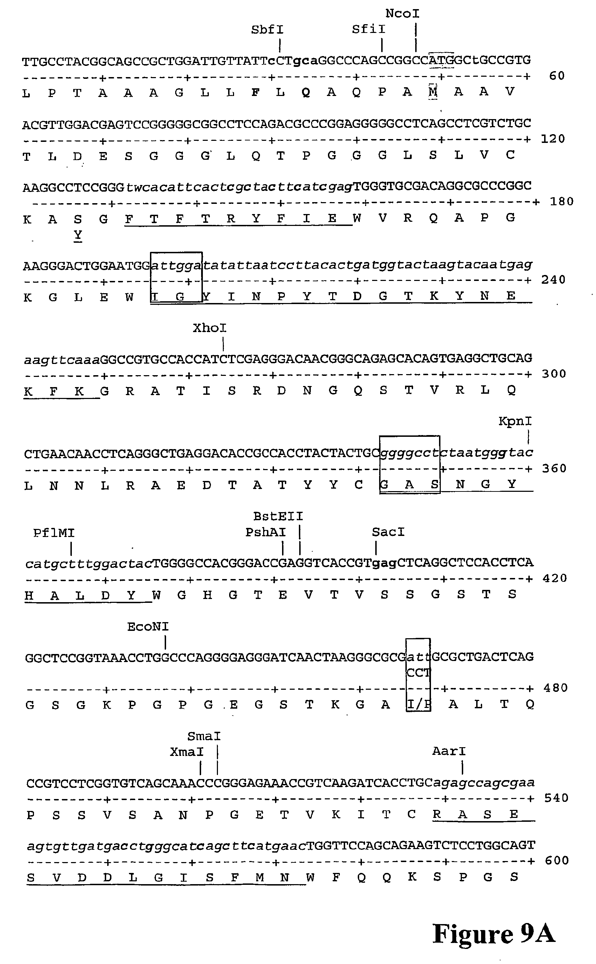

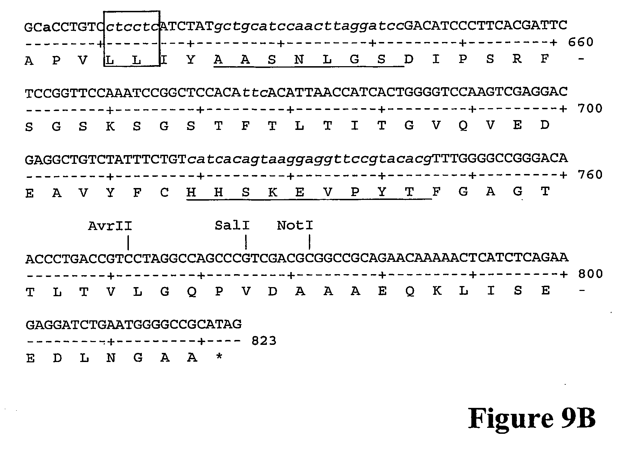

The nucleotide sequence in Figure 9 (SEQ ID NO:66) is the DNA sequence encoding a scFvG19-29 having chimeric variable domains, which is generated pursuing the semi-conservative strategy in accordance with the results of the comparative analysis of amino acid residues encountered in key positions as described above.

The DNA sequence represents the basis for designing primers to splice together nucleic acid molecules encoding the murine CDRs of the scFv29 and nucleic acid molecules encoding the FRs of the scFvG19. The nucleotide sequences encoding the murine CDRs are in small italic letters and the corresponding amino acid sequence is underlined. In small italic letters are also single amino acid residues of mouse origin located in the FRs. Some additional silent mutations to delete or insert restriction sites for sub-cloning steps may be made. Moreover, in order to increase the GC content and the melting temperature of some primers, modifications are made within the regions of primer annealing. These modifications are indicated in small letters and bold. The codons which encode the amino acid residues replaced by the amino acid residues of the murine antibody are indicated by boxes.

### C. Primer design

To assemble the scFvG19-29 with chimeric variable domains a set of oligonucleotide primers are designed that encode for the murine CDRs and part of the chicken FRs. The scFvG19-29 is semi-synthetically assembled, i.e. part of the chicken FRs are derived from the DNA of the chicken scaffold of the present invention used as template in the PCRs.

The primer regions that anneal to or encode the FRs of the scFvG19 DNA are indicated in capital letters in Figure 9, whereas the sequence regions of murine origin are in small italic characters. In small bold characters are nucleotides that encode for silent mutations and that are introduced in order to generate or delete restriction sites for subsequent cloning steps.

### Heavy Chain

To assemble the chimeric heavy chain eight primers are required:
- ch19-29-p1-1 (SEQ ID NO: 67)
5' GGATTGTTATT**c**CT**gca**GGCCCAGCCGGCCATGGCtGCCGTGACGTTGGACGAG 3'

This primer contains an SbfI restriction site (underlined) inserted for sub-cloning purposes.
- ch19-29-p1-2 (SEQ ID NO: 68)
5' CGCACCC*A**c**tc**g**at**g**aa**g**ta**g**c**g**agtGAAtgtgwa*CCCGGAGGCCTTGCAGACGAGG 3'

This primer contains a single wobble (highlighted) encoding Phe or Tyr.
- ch19-29-p12-3 (SEQ ID NO: 69)
5' ATCCAATCCATTCCAGTCCCTTGCCGGGCGCCTGTCGCACCCA*ctcgatgaagtagc* 3' - 19-29-p2-4 (SEQ ID NO: 70)
5' *ctcattgtacttagtaccatcagtgtaaggattaatatatccaat*CCATTCCAGTCCC 3'
- ch19-29-p2-5 (SEQ ID NO: 71)
5' CCGTTGTCCCTCGAGATGGTGGCACGGCC*tttgaacttctcattgtacttagtacc* 3'
- ch19-29-p3s-6 (SEQ ID NO: 72)
5' GGCCGTGCCACCATCTCGAGGGACAACGG 3'
- ch19-29-p3-7 (SEQ ID NO: 73)
5' GGCCCCA*tgagtccaaagcat***g***gtacccattagaggcccc*GCAGTAGTAGGTGGCGGTGTCC 3'
- ch19-29-p3-8 (SEQ ID NO: 74)
5' GAGGTGGAGCCT**gagctc**ACGGTGACCTCGGTCCCGTGGCCCCA*gtagtccaaagcatgg* 3'

### Light Chain

To assemble the light chain ten primers are required.
- ch19-29-le1-1 (SEQ ID NO: 75)
5' *ggggcctctaatgggtaccatgctttgg* 3'
- ch19-29-le1-2 (SEQ ID NO: 76)
5' TTGCTGACACCGAGGACGGCTGAGTCAGCGC*aat*CGCGCCCTTAGTTGATCCC 3'
- ch19-29-le1-3 (SEQ ID NO: 77)
5' *ggctct*GCAGGTGATCTTGACGGTTTCTCCCGGGTTTGCTGACACCGAGGACGG 3'
- ch19-29-le1-4 (SEQ ID NO: 78)
5' C*atgaagctgatgcccaggtcatcaacactttcgctggctct*GCAGGTGATCTTG 3'
- ch19-29-sle1-5 (SEQ ID NO: 79)
5' GCACTGCCAGGAGACTTCTGCTGGAACCA*gttcatgaagctgatgcccaggtc* 3'
- ch19-29-sle21-6 (SEQ ID NO: 80)
5' CCAGCAGAAGTCTCCTGGCAGTGCaCCTGTC*ctcctc*ATCTAT*gctgcatccaac* 3'
- ch19-29-sle23-7 (SEQ ID NO: 81)
5' GGAACCGGAGAATCGTGAAGGGATGTC*ggatcctaagttggatgcagc*ATAGAT*gagg* 3'
- ch19-29-sle32-8 (SEQ ID NO: 82)
5' CCCTTCACGATTCTCCGGTTCCAAATCCGGCTCCACA*ttc*ACATTAACCATCACTGGGGTC 3'
- ch19-29-le3-9 (SEQ ID NO: 83)
5' GTCCCGGCCCCAAA*cgtgtacggaacctccttactgtgatg*ACAGAAATAGACAGCCTCG 3'
- ch19-29-le3-10 (SEQ ID NO: 84)
5' TGCGGCCGCGTCGACGGGCTGGCCTAGGACGGTCAGGGTTGTCCCGGCCCCAAA*cgtgtacgg* 3'

### D. PCR mediated CDR splicing of the scFvG19-29 and cloning strategy

The splicing of the nucleic acid molecules encoding the murine CDRs and the FRs of the chicken scaffold of the present invention is carried out through PCR using the primers listed above.

Some of the PCRs make use of the template DNA of the scFvG19 in the pHEN4II vector.

### Heavy chain:

The splicing of the variable chimeric heavy chain DNA is carried out in three steps:

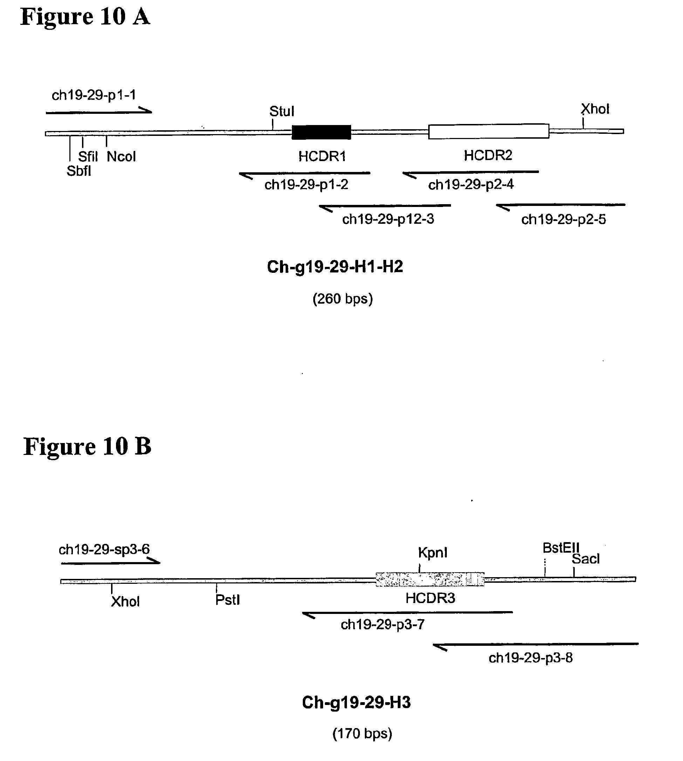

In the first step the nucleic acid molecules encoding CDR-H1 and CDR-H2 of the murine scFv29 are spliced with the nucleic acid molecules encoding the HFR1 and HFR2 of the scFvG19 by four PCRs. The first PCR is carried out using the scFvG19 template DNA. The first splicing step is summarized in Figure 10A.

Figure 10A shows that the primers ch19-29-p1-2 and ch19-20-p12-3 encode for the murine CDR-H1, whereas the primers ch19-29-p-12-3, ch19-29-p2-4 and ch19-29-p2-5 encode for the murine CDR-H2. The primer ch19-29-p1-1 and ch19-29-p1-2 are employed in combination with the scFvG19 template DNA. The DNA fragment obtained by the first PCR is purified and used in three subsequent PCRs where ch19-29-p1-1 is used as the sense primer and ch19-29-p12-3, ch19-29-p2-4 and ch19-29-p2-5 are used in independent reactions as the anti-sense primers. The product of the four PCRs of the first step is the fragment Ch-g19-29-H1-H2.

In the second step the murine CDR-H3 is spliced with the HFR3 and HFR4 of the chicken scaffold by two PCRs The first PCR is carried out using the scFvG19 template DNA in combination with the primers ch19-29-sp3-6 and ch19-29-p3-7. The DNA fragment obtained by the first PCR is purified and used in a second PCR where ch19-29-sp3-6 and ch19-29-p3-8 are employed as the sense and anti-sense primer, respectively. The PCRs of the second step are summarized in Figure 10B.

Figure 10B shows that the primer ch19-29-p3-7 and ch19-29-p3-8 encode for the murine CDR-H3. The product of the two PCRs of the second step is the fragment Ch-g19-29-H3.

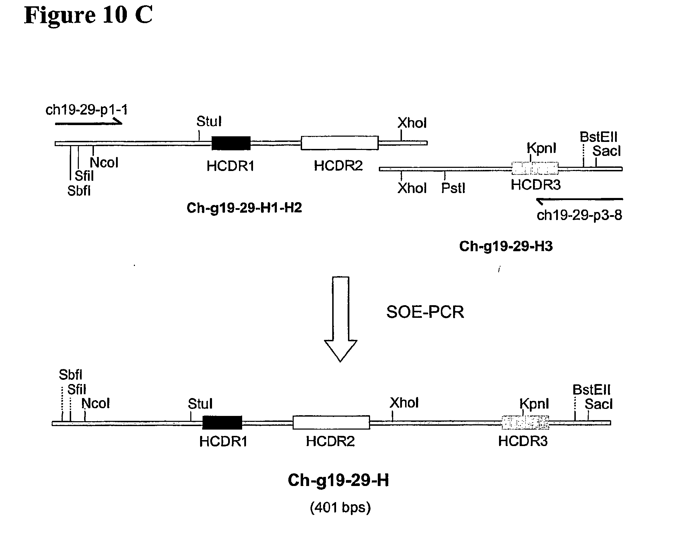

In the third step the fragments Ch-g19-29-H1-H2 and Ch-g19-29-H3 are spliced by SOE-PCR using ch19-29-p1-1 and ch19-29-p3-8 respectively, as the sense and anti-sense primers as summarized in Figure 10C.

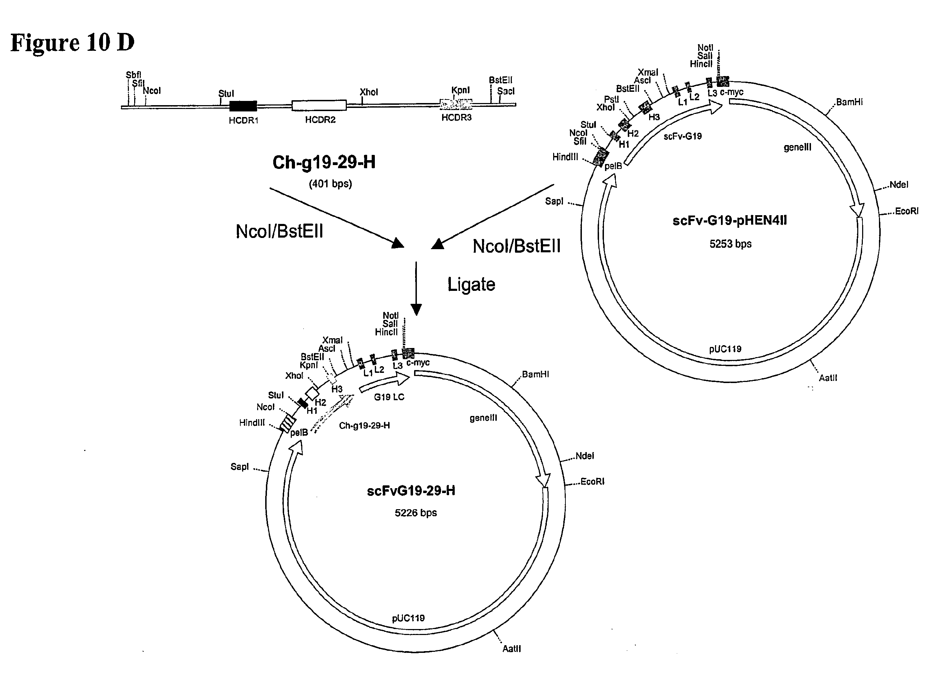

The fragment Ch-g19-29-H that encodes for the heavy chain variable domain is cloned via NcoI/BstEII into the template scFvG19 to replace the chicken heavy chain variable domain fragment as indicated Figure 10D.

### Light chain:

The splicing of the variable chimeric light chain DNA is carried out in five steps:

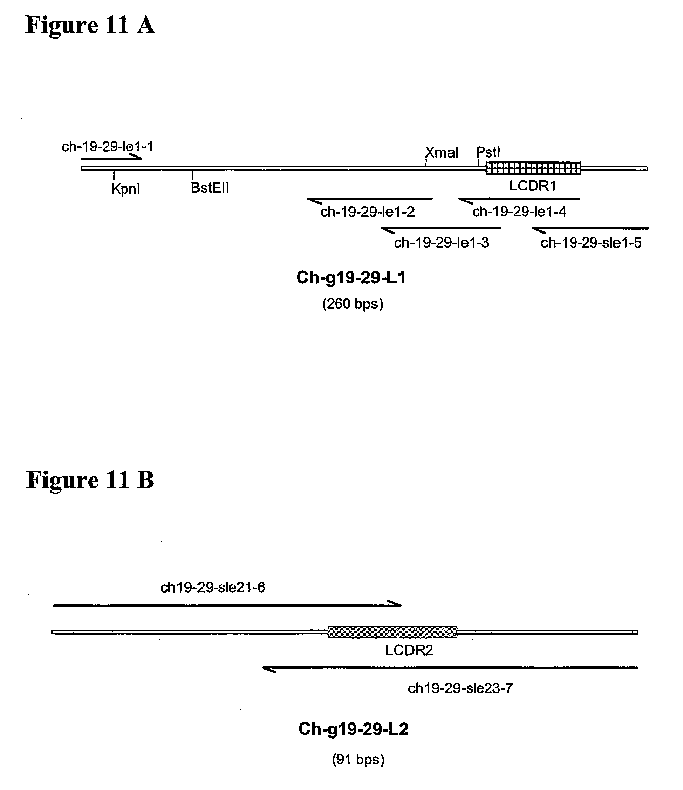

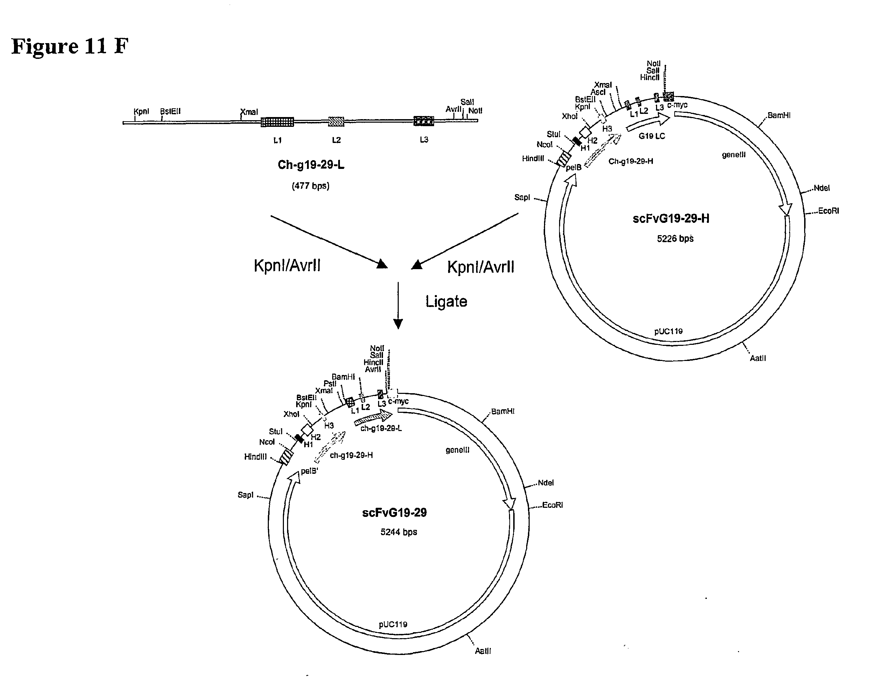

In the first step the CDR-L1 of the murine scFv29 is spliced to the LFR1 and LFR2 of the chicken scFvG19 by five PCRs. The first PCR is carried with the scFvG19-29 H as template DNA. The PCRs of the first step are carried out according to the Figure 11A:

Figure 11A depicts that the primers ch19-29-le1-3, ch19-29-le1-4 and ch19-29-le1-5 encode the murine CDR-L1. The primer ch19-29-le1-1 and ch19-29-le1-2 are used in combination with the scFvG19-29-H template DNA. The DNA fragment obtained by the first PCR is purified and used in three subsequent PCRs where ch19-29-le1-1 is the sense primer and ch19-29-le1-3, ch19-29-le1-4 and ch19-29-le1-5 are used in independent reactions as the anti-sense primers. The product of the four PCRs of the first step is the fragment Ch-g19-29-L1.

In the second step the murine CDR-L2 is spliced with the LFR2 and LFR3 of the chicken scaffold by joining the primers ch19-29-sle21-6 and ch19-29-sle23-7 in a single PCR as summarized in the following scheme:

Figure 11 B shows that the primer ch19-29-sle21-6 and ch19-29-sle23-7 encode for the murine CDR-L2 and part of the chicken LFR2 and LFR3, respectively.

The product of the PCR of the second step is the fragment Ch-g19-29-L2.

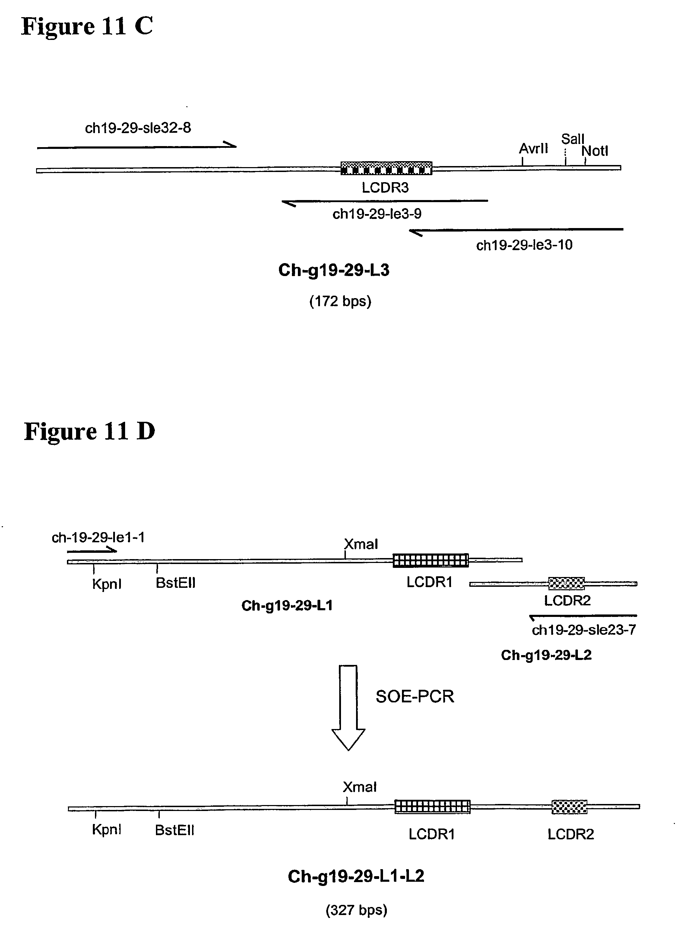

In the third step the murine CDR-L3 is spliced with the LFR3 and LFR4 of the chicken scaffold by two PCRs. The first PCR is carried out using the scFvG19 template DNA in combination with the primers ch19-29-sle32-8 and ch19-29-le3-9. The DNA fragment obtained by the first PCR is purified and employed in a second PCR where ch19-29-sle32-8 and ch19-29-le3-10 are the sense and anti-sense primer, respectively. The PCRs of the third step are summarized in Figure 11C.

Figure 11C shows that the primer ch19-29-le3-9 and ch19-29-le3-10 encode for the murine CDR-L3. The product of the two PCRs of the third step is the fragment Ch-g19-29-L3.

In the fourth PCR step the fragments Ch-g19-29-L1 and Ch-g19-29-L2 are spliced by SOE-PCR using ch19-29-le1-1 and ch19-29-sle3-7 respectively, as the sense and anti-sense primers as summarized in Figure 11D.

The product of the fourth PCR step is the fragment Ch-g19-29-L1-L2

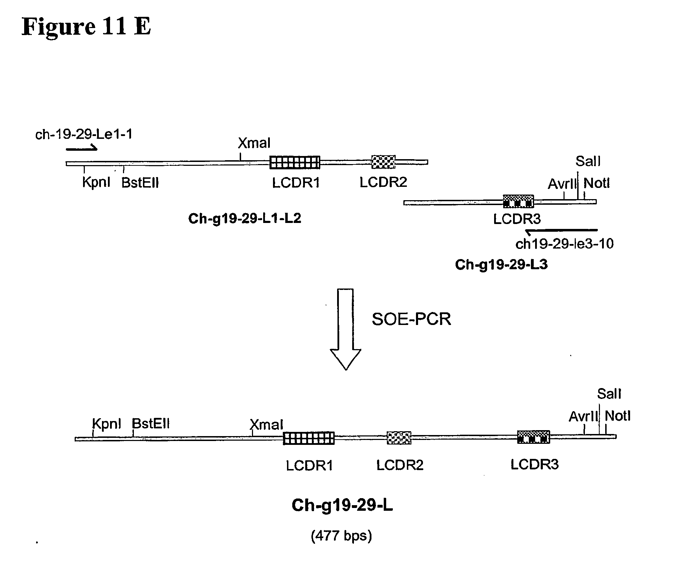

In the fifth PCR step the fragments Ch-g19-29-L1-L2 and Ch-g19-29-L3 are spliced by SOE-PCR using ch19-29-le1-1 and ch19-29-sle3-10 respectively, as the sense and anti-sense primers as summarized in Figure 11E.

The product of the SOE-PCR of the fifth step, is Ch-g19-29-L and encodes for the chimeric light chain variable domain of the scFvG19-29.

Ch-g19-29-L can be obtained by an alternative procedure. Firstly, the fragments Ch-g19-29-L2 to the Ch-g19-29-L3 are spliced together and then the resulting DNA fragment is employed in a second SOE-PCR to be joined to the fragment Ch-g19-29-L1.

The light chain variable domain fragment Ch-g19-29-L is cloned via KpnI/AvrII into the scFvG19-29-H to replace the chicken light chain DNA as depicted in Figure 10 F.

The resulting product is the nucleic acid molecule encoding scFvG19-29 with chimeric variable domains that is sub-cloned into the pTMZ1 bacterial expression vector or the pSSH vector for expression in plant cells. Alternatively the nucleic acid molecule encoding scFvG19-29 may be cloned into vectors such as those disclosed *supra* for expression in other systems, e.g., bacterial (e.g., E. coli), yeast, insect, avian, piscean or mammalian, e.g., murine or human, systems. The vectors may be transformed into a suitable host cell and expressed. The host cell may be assayed by methods known in the art for the accumulation of the scFv with the chimeric variable domains..

### E. Analysis of protein expression and biological activity of scFvG19-29

The expression of the scFvG19-29 in bacteria may be assayed in *E. coli* cultures grown in batch system using standard protocols for protein expression. Moreover, the expression of scFvG19-29 having chimeric variable domains in plants may be assayed by the transient expression system of vacuum infiltrated tobacco leaves as described previously. The expression of the scFv antibodies of this invention may be assayed in other systems using any appropriate method known in the art. The accumulated levels of the scFv antibodies of this invention is compared to the accumulated level of the parental counterparts using immunoblot analysis and ELISA.

The biological activity of scFvG19-29 having the chimeric chicken-murine variable domains is evaluated in appropriate ELISA formats, e.g., conventional solid phase ELISA and competition ELISA. The binding activity of scFvG19-29 which has the chimeric variable domains is compared with that of the parental murine monoclonal Ab29 and scFv29 to estimate the binding affinity of scFvG19-29 to the TMV coat protein as compared to the binding affinity of the parental antibody.

The expression levels and/or the binding properties of a scFv having chimeric variable domains may be altered further by replacing additional amino acid residues to bias the protein composition of the scFv with chimeric variable domains toward the chicken or the murine counterpart.

## Claims

1. An immunoglobulin molecule which accumulates in host cells to a concentration of at least about 0.2 % to about 30 % of the total soluble protein, wherein the molecule comprises the structure:
(a) HFR1--CDR-H1--HFR2--CDR-H2--HFR3--CDR-H3--HFR4 and
(b) LFR1--CDR-L1--LFR2--GDR-L2--LFR3--CDR-L3--LFR4,
wherein,
(i) HFR1 is a first heavy chain framework region consisting of a sequence of about 30 amino acid residues and comprising SEQ ID NO: 1;
(ii) HFR2 is a second heavy chain framework region consisting of a sequence of about 14 amino acid residues and comprising SEQ ID NO: 2;
(iii) HFR3 is a third heavy chain framework region consisting of a sequence of about 29 to about 32 amino acid residues and comprising SEQ ID NO: 3, wherein the amino acid residues at positions 18, 19 and/or 20 in SEQ ID NO: 3 are optionally absent and not substituted by another amino acid;
(iv) HFR4 is a fourth heavy chain framework region consisting of a sequence of about 9 amino acid residues and comprising SEQ ID NO: 4;
(v) CDR-H1 is a first heavy chain complementary determining region;
(vi) CDR-H2 is a second heavy chain complementary determining region;
(vii) CDR-H3 is a third heavy chain complementary determining region;
(viii) LFR1 is a first light chain framework region consisting of a sequence of about 22 to about 23 amino acid residues and comprising SEQ ID NO: 5;
(ix) LFR2 is a second light chain framework region comprising SEQ ID NO: 6, wherein the amino acid residue at position 6 is optionally absent and not substituted by another amino acid;
(x) LFR3 is a third light chain framework region consisting of a sequence of about 32 amino acid residues and comprising SEQ ID NO: 7;
(xi) LFR4 is a fourth light chain framework region consisting of a sequence of about 12 to about 13 amino acid residues, and comprising SEQ ID NO: 8 wherein the amino acid residue at position 10 is optionally absent and not substituted by another amino acid;
(xii) CDR-L1 is a first light chain complementary determining region;
(xiii) CDR-L2 is a second light chain complementary determining region;
(xiv) CDR-L3 is a third light chain complementary determining region,
or
variants of (a) and (b) having conservative substitutions whereby one or more amino acid residues is substituted by an equivalent amino acid,
provided that the first residue in HFR4 is Trp, and that a Pro or Leu is at position 10 of LFR2 if the sequence is 15 amino acid residues long or position 11 if the sequence is 16 amino acid residues long, and that the first residue in LFR4 is Phe,
wherein arginine, lysine and histidine are equivalent amino acids; alanine, glycine and serine are equivalent amino acids; and phenylalanine, tryptophan and tyrosine are equivalent amino acids,
and wherein the length of the CDRs and the framework regions and positions of the amino acid residues in the CDRs and the framework regions are in accordance with the Kabat numbering system..

2. The immunoglobulin molecule of claim 1, wherein the first residue in HFR3 is Arg and the tenth residue in HFR3 is Gln.

3. The immunoglobulin molecule of claim 1 or claim 2, wherein the amino acid residue at position 6 of LFR2 is present and is preferably Ser.

4. The immunoglobulin molecule or any of the preceding claims, wherein the heavy chain framework regions comprise one or more of SEQ ID NO: 1 (HFR1), SEQ ID NO: 2 (HFR2), SEQ ID NO: 3 (HFR3) and SEQ ID NO: 4 (HFR4).

5. The immunoglobulin molecule of any of the preceding claims, wherein the light chain framework regions comprise one or more of SEQ ID NO: 5 (LFR1), SEQ ID NO: 6 (LFR2), SEQ ID NO: 7 (LFR3) and SEQ ID NO: 8 (LFR4).

6. The immunoglobulin molecule of any of the preceding claims, wherein the immunoglobulin accumulates in plant cells to a concentration of at least about 0.2 % to about 30 % of the total soluble protein.

7. The immunoglobuin molecule of claim 1, wherein:
HFR1 comprises SEQ ID NO: 1;
HFR2 comprises SEQ ID NO: 2;
HFR3 comprises SEQ ID NO: 3;
HFR4 comprises SEQ ID NO: 4;
LFR1 comprises SEQ ID NO: 5;
LFR2 comprises SEQ ID NO: 6;
LFR3 comprises SEQ ID NO: 7, and;
LFR4 comprises SEQ ID NO: 8.

8. The immunoglobulin of any one of the preceding claims wherein:
CDR-L1 is 5-14 amino acid residues in length,
CDR-L2 is 5-7 amino acid residues in length,
CDR-L3 is 5-15 amino acid residues in length,
CDR-H1 is 5-7 amino acid residues in length,
CDR-H2 is 16-18 amino acid residues in length, and
CDR-H3 is 9-21 amino acid residues in length.

9. The isolated immunoglobulin molecule of claim 8 wherein:
CDR-H1 consists of 5 amino acid residues,
CDR-H2 consists of 17 amino acid residues,
CDR-H3 consists of 9 to 19 amino acid residues,
CDR-L1 consists of 8, 9, 10 or 13 amino acid residues,
CDR-L2 consists of 7 amino acid residues and
CDR-L3 consists of 8 to 12 amino acid residues.

10. The immunoglobulin molecule of claim 9 wherein CDR-H3 consists of 14 to 19 amino acid residues.

11. The immunoglobulin molecule of any one of the preceding claims further comprising a linker which joins (a) to (b).

12. The immunoglobulin molecule of any one of the preceding claims further comprising a cellular targeting signal and/or a tag.

13. The immunoglobulin molecule of claim 12 wherein said cellular targeting signal is selected from the group consisting of apoplastic targeting peptide, an endoplasmic reticulum targeting peptide, a vacuole targeting peptide, protein body targeting peptide and a chloroplast targeting peptide.

14. An isolated nucleic acid molecule encoding the immunoglobulin molecule of any one of claims 1-13.

15. The isolated nucleic acid molecule of claim 14 comprising a nucleotide sequence selected from the group consisting of SEQ ID NO: 9, SEQ ID NO: 10, SEQ ID NO: 11, SEQ ID NO: 12, SEQ ID NO; 13 and SEQ ID NO; 14.

16. A recombinant library comprising one or more isolated nucleic acid molecule according to claim 14 or claim 15.

17. A vector comprising an isolated nucleic acid molecule of claim 14 or claim 15 in operable linkage with a promoter.

18. The vector of claim 17 comprising a nucleotide sequence encoding a cellular targeting peptide.

19. A host cell comprising a nucleic acid molecule of claim 14 or claim 15, or the vector of claim 16 or claim 17.

20. The host cell of claim 19 wherein said host is bacterial cell, a yeast cell, an algae cell, an insect cell, a mammalian cell or a plant cell.

21. A method for preparing a recombinant library expressing immunoglobulin molecules of any one of claims 1 to 13, wherein said method comprises preparing one or more nucleic acid molecules encoding the immunoglobulin molecules, or domains thereof, and expressing said nucleic acid molecules in an appropriate host cell wherein expression of said nucleic acid produces a recombinant library expressing the immunoglobulin molecules or the domains thereof.

22. The method of claim 21, wherein the immunoglobulin molecules are selected from the group consisting of scFv, diabodies, triabodies and tetrabodies.

23. The method of claim 21, wherein the immunoglobulin molecules comprise randomized CDRs.

24. A method for identifying an immunoglobulin molecule from a recombinant library expressing immunoglobulin molecules of any one of claims 1 to 13, wherein the immunoglobulin molecule binds to a predetermined antigen, comprising contacting the immunoglobulin molecules with the predetermined antigen and assaying for binding therebetween.

25. The method of claim 24 further comprising identifying the nucleic acid molecule that encodes the immunoglobulin molecule or domain thereof identified in claim 24.

26. Use of the immunoglobulin molecule of any one of claims 1 to 13 in *in vitro* diagnostic assays.

27. The use of claim 26, wherein the assay is an *in vitro* screening assay for detecting molecules which bind to said immunoglobulin molecule.

28. The use of claim 26 or claim 27 wherein the assays are selected from the group consisting of ELISA assays, phage display assays and protein chips.

29. The use of claim 26 or claim 27 wherein the immunoglobulin molecule may be a scFv, a bispecific scFv or a multivalent scFv.

30. A method for producing an immunoglobulin molecule having a chimeric variable domain, wherein the immunoglobulin molecule accumulates in a host cell to at least 0.15 % total soluble protein, comprising:
(a) determining amino acid sequence of an avian immunoglobulin molecule comprising a variable domain, wherein said variable domain contains framework regions, and complementarity determining regions (CDRs), and determining amino acid sequence of a preselected immunoglobulin molecule, which is specific for an antigen, said preselected immunoglobulin comprising a variable domain which contains framework regions and CDRs, wherein the framework regions and CDRs of the immunoglobulin molecules are in accordance with Kabat's numbering system,
(b) comparing the amino acid sequences of the variable domains of the avian immunoglobulin and the preselected immunoglobulin to identify differences in amino acid residues at corresponding positions in the avian and preselected antibody framework regions and CDRs that are necessary for maintaining conformation of the CDRs,
(c) preparing a nucleic acid molecule encoding an immunoglobulin molecule comprising a variable domain where the variable domain CDRs are the CDRs of the preselected immunoglobulin molecule and wherein the variable domain framework regions are the avian framework regions with the proviso that one or more of the amino acid residue positions identified in (b) as having different amino acid residues in the avian immunoglobulin molecule variable domain as compared to the preselected immunoglobulin molecule variable domain, contain the amino acid residue present in the preselected immunoglobulin variable domain, and
(d) expressing the nucleic acid molecule of (c) to produce an immunoglobulin molecule having a chimeric variable domain.

31. The method of claim 30 wherein the avian immunoglobulin amino acid sequence comprises SEQ ID NO: 51.

32. The method of claim 30 wherein the amino acid residue position which are necessary for maintaining conformation of the CDRs of the preselected immunoglobulin molecule are determined by the methods of Kabat, Chothia and the contact method.

33. The method of claim 30 wherein the immunoglobulin having a chimeric variable domain is a V_{L}, V_{H}, scFv, diabody, triabody or tetrabody.

34. A transgenic plant, or a part thereof, expressing an immunoglobulin molecule according to any of claims 1 to 13.

35. A transgenic plant or part thereof according to claim 34, wherein the immunoglobulin molecule is specific for a plant pathogen.

## Patentansprüche

1. Immunglobulinmolekül, das in Wirtszellen bis zu einer Konzentration von zumindest etwa 0,2 % bis etwa 30 % des gesamten löslichen Proteins akkumuliert, worin das Molekül folgende Struktur umfasst:
(a) HFR1--CDR-H1--HFR2--CDR-H2--HFR3--CDR-H3--HFR4
und
(b) LFR1--CDR-L1--LFR2--CDR-L2--LFR3--CDR-L3--LFR4,
worin,
(i) HFR1 eine erste Schwerketten-Gerüstregion ist, die aus einer Sequenz von etwa 30 Aminosäureresten besteht und die Seq.-ID Nr. 1 umfasst;
(ii) HFR2 eine zweite Schwerketten-Gerüstregion ist, die aus einer Sequenz von etwa 14 Aminosäureresten besteht und die Seq.-ID Nr. 2 umfasst;
(iii) HFR3 eine dritte Schwerketten-Gerüstregion ist, die aus einer Sequenz von etwa 29 bis etwa 32 Aminosäureresten besteht und Seq.-ID Nr. 3 umfasst, worin die Aminosäurereste an Positionen 18, 19 und/oder 20 in Seq.-ID Nr. 3 gegebenenfalls fehlen und nicht durch eine andere Aminosäure substituiert sind;
(iv) HFR4 eine vierte Schwerketten-Gerüstregion ist, die aus einer Sequenz von etwa 9 Aminosäureresten besteht und die Seq.-ID Nr. 4 umfasst;
(v) CDR-H1 eine erste komplementaritätsbestimmende Schwerketten-Region ist;
(vi) CDR-H2 eine zweite komplementaritätsbestimmende Schwerketten-Region ist;
(vii) CDR-H3 eine dritte komplementaritätsbestimmende Schwerketten-Region ist;
(viii) LFR1 eine erste Leichtketten-Gerüstregion ist, die aus einer Sequenz von etwa 22 bis etwa 23 Aminosäureresten besteht und Seq.-ID Nr. 5 umfasst;
(ix) LFR2 eine zweite Leichtketten-Gerüstregion ist, die Seq.-ID Nr. 6 umfasst, worin der Aminosäurerest an Position 6 gegebenenfalls fehlt und nicht durch eine andere Aminosäure substituiert ist;
(x) LFR3 eine dritte Leichtketten-Gerüstregion ist, die aus einer Sequenz von etwa 32 Aminosäureresten besteht und Seq.-ID Nr. 7 umfasst;
(xi) LFR4 eine vierte Leichtketten-Gerüstregion ist, die aus einer Sequenz von etwa 12 bis etwa 13 Aminosäureresten besteht und Seq.-ID Nr. 8 umfasst, worin der Aminosäurerest an Position 10 gegebenenfalls fehlt und nicht durch eine andere Aminosäure substituiert ist;
(xii) CDR-L1 eine erste komplementaritätsbestimmende Leichtketten-Region ist;
(xiii) CDR-L2 eine zweite komplementaritätsbestimmende Leichtketten-Region ist;
(xiv) CDR-L3 eine dritte komplementaritätsbestimmende Leichtketten-Region ist,
oder
Varianten von (a) und (b) mit konservativen Substitutionen, wobei ein oder mehrere Aminosäurereste durch eine äquivalente Aminsäure substituiert ist,
vorausgesetzt, dass der erste Rest in HFR4 Trp ist und dass ein Pro oder Leu an Position 10 von LFR2 ist, wenn die Sequenz 15 Aminosäurereste lang ist, oder an Position 11 ist, wenn die Sequenz 16 Aminosäurereste lang ist, und dass der erste Rest in LFR4 Phe ist,
worin Arginin, Lysin und Histidin äquivalente Aminosäuren sind; Alanin, Glycin und Serin äquivalente Aminosäuren sind; und Phenylalanin, Tryptophan und Tyrosin äquivalente Aminosäuren sind
und worin die Länge der CDRs und die Gerüstregionen und Positionen der Aminosäurereste in den CDRs und die Gerüstregionen in Übereinstimmung mit dem Kabat-Nummerierungssystem sind.

2. Immunglobulinmolekül nach Anspruch 1, worin der erste Rest in HFR3 Arg und der zehnte Rest in HFR3 Gln ist.

3. Immunglobulinmolekül nach Anspruch 1 oder 2, worin der Aminosäurerest an Position 6 von LFR2 vorhanden und vorzugsweise Ser ist.

4. Immunglobulinmolekül nach einem der vorangegangenen Ansprüche, worin die Schwerketten-Gerüstregionen eine oder mehrere von Seq.-ID Nr. 1 (HFR1), Seq.-ID Nr. 2 (HFR2), Seq.-ID Nr. 3 (HFR3) und Seq.-ID Nr. 4 (HFR4) umfassen.

5. Immunglobulinmolekül nach einem der vorangegangenen Ansprüche, worin die Leichtketten-Gerüstregionen eine oder mehrere von Seq.-ID Nr. 5 (LFR1), Seq.-ID Nr. 6 (LFR2), Seq.-ID Nr. 7 (LFR3) und Seq.-ID Nr. 8 (LFR4) umfassen.

6. Immunglobulinmolekül nach einem der vorangegangenen Ansprüche, worin das Immunglobulin in Pflanzenzellen bis zu einer Konzentration von zumindest etwa 0,2 % bis etwa 30 % des gesamten löslichen Proteins akkumuliert.

7. Immunglobulinmolekül nach Anspruch 1, worin:
HFR1 Seq.-ID Nr. 1 umfasst;
HFR2 Seq.-ID Nr. 2 umfasst;
HFR3 Seq.-ID Nr. 3 umfasst;
HFR4 Seq.-ID Nr. 4 umfasst;
LFR1 Seq.-ID Nr. 5 umfasst;
LFR2 Seq.-ID Nr. 6 umfasst;
LFR3 Seq.-ID Nr. 7 umfasst; und
LFR4 Seq.-ID Nr. 8 umfasst.

8. Immunglobulin nach einem der vorangegangenen Ansprüche, worin:
CDR-L1 eine Länge von 5-14 Aminosäuren aufweist,
CDR-L2 eine Länge von 5-7 Aminosäuren aufweist,
CDR-L3 eine Länge von 5-15 Aminosäuren aufweist,
CDR-H1 eine Länge von 5-7 Aminosäuren aufweist,
CDR-H2 eine Länge von 16-18 Aminosäuren aufweist,
CDR-H3 eine Länge von 9-21 Aminosäuren aufweist,

9. Isoliertes Immunglobulinmolekül nach Anspruch 8, worin:
CDR-H1 aus 5 Aminosäureresten besteht,
CDR-H2 aus 17 Aminosäureresten besteht,
CDR-H3 aus 9 bis 19 Aminosäureresten besteht,
CDR-L1 aus 8, 9, 10 oder 13 Aminosäureresten besteht,
CDR-L2 aus 7 Aminosäureresten besteht,
CDR-L3 aus 8 bis 12 Aminosäureresten besteht.

10. Immunglobulinmolekül nach Anspruch 9, worin CDR-H3 aus 14 bis 19 Aminosäureresten besteht.

11. Immunglobulinmolekül nach einem der vorangegangenen Ansprüche, das weiters einen Linker umfasst, der (a) an (b) bindet.

12. Immunglobulinmolekül nach einem der vorangegangenen Ansprüche, das weiters ein zelluläres Targetingsignal und/oder eine Markierung umfasst.

13. Immunglobulinmolekül nach Anspruch 12, worin das zelluläre Targetingsignal aus der Gruppe ausgewählt ist, die aus apoplastischem Targetingpeptid, einem endoplasmisches-Retikulum-Targetingpeptid, einem Vakuolen-Targetingpeptid, Proteinkörper-Targetingpeptid und einem Chloroplasten-Targetingpeptid besteht.

14. Isoliertes Nucleinsäuremolekül, das für das Immunglobulinmolekül nach einem der Ansprüche 1-13 kodiert.

15. Isoliertes Nucleinsäuremolekül nach Anspruch 14, das eine Nucleotidsequenz umfasst, die aus der Gruppe ausgewählt ist, die aus Seq.-ID Nr. 9, Seq.-ID Nr. 10, Seq.-ID Nr. 11, Seq.-ID Nr. 12, Seq.-ID Nr. 13 und Seq.-ID Nr. 14 besteht.

16. Rekombinante Bibliothek, die eine oder mehrere isolierte Nucleinsäuremoleküle nach Anspruch 14 oder Anspruch 15 umfasst.

17. Vektor, der ein isoliertes Nucleinsäuremolekül nach Anspruch 14 oder Anspruch 15 in einer operablen Verbindung mit einem Promotor umfasst.

18. Vektor nach Anspruch 17, der eine Nucleotidsequenz umfasst, die für ein zelluläres Targetingpeptid kodiert.

19. Wirtszelle, die ein Nucleinsäuremolekül nach Anspruch 14 oder Anspruch 15 oder den Vektor nach Anspruch 16 oder Anspruch 17 umfasst.

20. Wirtszelle nach Anspruch 19, worin die Wirtszelle eine bakterielle Zelle, eine Hefezelle, eine Algenzelle, eine Insektenzelle, eine Säugetierzelle oder eine Pflanzenzelle ist.

21. Verfahren zur Herstellung einer rekombinanten Bibliothek, die Immunglobulinmoleküle nach einem der Ansprüche 1 bis 13 exprimiert, worin das Verfahren das Herstellen von einem oder von mehreren Nucleinsäuremolekülen, die für die Immunglobulinmoleküle oder Domänen davon kodieren, und Expression der Nucleinsäuremoleküle in einer geeigneten Wirtszelle umfasst, worin die Expression der Nucleinsäure eine rekombinante Bibliothek erzeugt, welche die Immunglobulinmoleküle oder die Domänen davon exprimiert.

22. Verfahren nach Anspruch 21, worin die Immunglobulinmoleküle aus der Gruppe ausgewählt sind, die aus scFv, Diakörper, Triakörper und Tetrakörper besteht.

23. Verfahren nach Anspruch 21, worin die Immunglobulinmoleküle randomisierte CDRs umfassen.

24. Verfahren zur Identifizierung eines Immunglobulinmoleküls aus einer rekombinanten Bibliothek, die Immunglobulinmoleküle nach einem der Ansprüche 1 bis 13 exprimiert, worin das Immunglobulinmolekül an ein vorherbestimmtes Antigen bindet, welches das Kontaktieren der Immunglobulinmoleküle mit dem vorherbestimmten Antigen und das Testen auf die Bindung dazwischen umfasst.

25. Verfahren nach Anspruch 24, das weiters das Identifizieren des Nucleinsäuremoleküls umfasst, das für das Immunglobulinmolekül oder die Domäne davon, identifiziert in Anspruch 24, kodiert.

26. Verwendung des Immunglobulinmoleküls nach einem der Ansprüche 1 bis 13 in In-vitro-Diagnoseverfahren.

27. Verwendung nach Anspruch 26, worin der Test ein In-vitro-Screeningtest zum Nachweis von Molekülen ist, die an das Immunglobulinmolekül binden.

28. Verwendung nach Anspruch 26 oder Anspruch 27, worin die Tests aus der Gruppe ausgewählt sind, die aus ELISA-Tests, Phagendisplaytests und Proteinchips besteht.

29. Verwendung nach Anspruch 26 oder Anspruch 27, worin das Immunglobulinmolekül ein scFv, ein bispezifisches scFv oder ein multivalentes scFv sein kann.

30. Verfahren zur Herstellung eines Immunglobulinmoleküls mit einer chimären variablen Domäne, worin das Immunglobulinmolekül in eine Wirtszelle bis zu einem Prozentsatz von zumindest 0,15 % des gesamten löslichen Proteins akkumuliert, umfassend:
(a) Bestimmung der Aminosäuresequenz eines von einem Vogel stammenden Immunglobulinmoleküls, das eine variable Domäne umfasst, worin die variable Domäne Gerüstregionen und komplementaritätsbestimmende Regionen (CDRs) enthält, und Bestimmung der Aminosäuresequenz eines vorselektierten Immunglobulinmoleküls, das für ein Antigen spezifisch ist, wobei das vorselektierte Immunglobulin eine variable Domäne umfasst, die Gerüstregionen und CDRs enthält, worin die Gerüstregionen und CDRs der Immunglobulinmoleküle in Übereinstimmung mit Kabats Nummerierungssystem sind,
(b) Vergleich der Aminosäuresequenzen der variablen Domänen des von einem Vogel stammenden Immunglobulins und des vorselektierten Immunglobulins zur Identifizierung von Unterschieden in Aminosäureresten bei entsprechenden Positionen in den von einem Vogel stammenden und vorselektierten Antikörpergerüstregionen und CDRs, die zur Erhaltung der Konformation der CDRs notwendig sind,
(c) Herstellen eines Nucleinsäuremoleküls, das für ein Immunglobulinmolekül kodiert, das eine variable Domäne umfasst, wo die CDRs der variablen Domänen jene CDRs des vorselektierten Immunglobulinmoleküls sind und worin die variablen Domänen-Gerüstregionen die von einem Vogel stammenden Gerüstregionen sind, mit der Maßgabe, dass eine oder mehrere der Aminosäurerestpositionen in (b) so identifiziert wurden, dass sie unterschiedliche Aminosäurereste in der variablen Domäne des vom Vogel stammenden Immunglobulinmoleküls im Vergleich zur variablen Domäne des vorselektierten Immunglobulinmoleküls aufweisen, den Aminosäurerest enthalten, der in der variablen Domäne des vorselektierten Immunglobulins vorhanden ist, und
(d) Expression des Nucleinsäuremoleküls von (c), um ein Immunglobulinmolekül herzustellen, das eine chimäre variable Domäne aufweist.

31. Verfahren nach Anspruch 30, worin die vom Vogel stammende Immunglobulin-Aminosäuresequenz Seq.-ID Nr. 51 umfasst.

32. Verfahren nach Anspruch 30, worin die Amionsäurerestpositionen, die zur Erhaltung der Konformation der CDRs des vorselektierten Immunglobulinmoleküls notwendig sind, mit Hilfe der Verfahren von Kabat, Chothia und mit Hilfe des Kontaktierungsverfahrens bestimmt werden.

33. Verfahren nach Anspruch 30, worin das Immunglobulin, das eine chimäre variable Domäne aufweist, ein V_{L}, V_{H}, scFv, Diakörper, Triakörper oder Tetrakörper ist.

34. Transgene Pflanze oder ein Teil davon, die/der ein Immunglobulinmolekül nach einem der Ansprüche 1 bis 13 exprimiert.

35. Transgene Pflanze oder ein Teil davon nach Anspruch 34, worin das Immunglobulinmolekül für ein Pflanzenpathogen spezifisch ist.

## Revendications

1. Molécule d'immunoglobuline qui s'accumule dans des cellules hôtes à une concentration d'au moins environ 0,2 % à environ 30 % de la protéine soluble totale, où la molécule comprend la structure:
(a) HFR1--CDR-H1--HFR2--CDR-H2--HFR3--CDR-H3--HFR4
et
(b) LFR1--CDR-L1--LFR2--CDR-L2--LFR3--CDR-L3--LFR4, où,
(i) HFR1 est une première région charpente de chaîne lourde consistant en une séquence d'environ 30 résidus d'acides aminés et comprenant SEQ ID NO: 1;
(ii) HFR2 est une deuxième région charpente de chaîne lourde consistant en une séquence d'environ 14 résidus d'acides aminés et comprenant SEQ ID NO: 2;
(iii) HFR3 est une troisième région charpente de chaîne lourde consistant en une séquence d'environ 29 à environ 32 résidus d'acides aminés et comprenant SEQ ID NO: 3, où les résidus d'acides aminés aux positions 18, 19 et/ou 20 dans SEQ ID NO: 3 sont facultativement absents et non substitués par un autre acide aminé;
(iv) HFR4 est une quatrième région charpente de chaîne lourde consistant en une séquence d'environ 9 résidus d'acides aminés et comprenant SEQ ID NO: 4;
(v) CDR-H1 est une première région déterminant la complémentarité de chaîne lourde;
(vi) CDR-H2 est une deuxième région déterminant la complémentarité de chaîne lourde;
(vii) CDR-H3 est une troisième région déterminant la complémentarité de chaîne lourde;
(viii)LFR1 est une première région charpente de chaîne légère consistant en une séquence d'environ 22 à environ 23 résidus d'acides aminés et comprenant SEQ ID NO: 5;
(ix) LFR2 est une deuxième région charpente de chaîne légère comprenant SEQ ID NO: 6, dans laquelle le résidu d'acide aminé à la position 6 est facultativement absent et non substitué par un autre acide aminé;
(x) LFR3 est une troisième région charpente de chaîne légère consistant en une séquence d'environ 32 résidus d'acides aminés et comprenant SEQ ID NO: 7;
(xi) LFR4 est une quatrième région charpente de chaîne légère consistant en une séquence d'environ 12 à environ 13 résidus d'acides aminés, et comprenant SEQ ID NO: 8, dans laquelle le résidu d'acide aminé à la position 10 est facultativement absent et non substitué par un autre acide aminé;
(xii) CDR-L1 est une première région déterminant la complémentarité de chaîne légère;
(xiii)CDR-L2 est une deuxième région déterminant la complémentarité de chaîne légère;
(xiv) CDR-L3 est une troisième région déterminant la complémentarité de chaîne légère;
ou
des variants de (a) et (b) ayant des substitutions conservatrices, moyennant quoi un ou plusieurs résidus d'acides aminés est/sont substitué(s) par un acide aminé équivalent,
à condition que le premier résidu dans HFR4 soit Trp, et que une Pro ou Leu soit à la position 10 de LFR2 si la séquence a une longueur de 15 résidus d'acides aminés, ou à la position 11 si la séquence a une longueur de 16 résidus d'acides aminés, et que le premier résidu dans LFR4 soit Phe,
où l'arginine, la lysine et l'histidine sont des acides aminés équivalents; l'alanine, la glycine et la sérine sont des acides aminés équivalents; et la phénylalanine, le tryptophane et la tyrosine sont des acides aminés équivalents,
et où la longueur des CDR et les régions charpentes et les positions des résidus d'acides aminés dans les CDR et les régions charpentes sont selon le système de numérotation de Kabat.

2. Molécule d'immunoglobuline selon la revendication 1, dans laquelle le premier résidu dans HFR3 est Arg et le dixième résidu dans HFR3 est Gln.

3. Molécule d'immunoglobuline selon la revendication 1 ou la revendication 2, dans laquelle le résidu d'acide aminé à la position 6 de LFR2 est présent et est de préférence Ser.

4. Molécule d'immunoglobuline selon l'une quelconque des revendications précédentes, dans laquelle les régions charpentes de chaîne lourde comprennent une ou plusieurs de SEQ ID NO: 1 (HFR1), SEQ ID NO: 2 (HFR2), SEQ ID NO: 3 (HFR3) et SEQ ID NO: 4 (HFR4).

5. Molécule d'immunoglobuline selon l'une quelconque des revendications précédentes, dans laquelle les régions charpentes de chaîne légère comprennent une ou plusieurs de SEQ ID NO: 5 (LFR1), SEQ ID NO: 6 (LFR2), SEQ ID NO: 7 (LFR3) et SEQ ID NO: 8 (LFR4).

6. Molécule d'immunoglobuline selon l'une quelconque des revendications précédentes, où l'immunoglobuline s'accumule dans des cellules végétales à une concentration d'au moins environ 0,2 % à environ 30 % de la protéine soluble totale.

7. Molécule d'immunoglobuline selon la revendication 1, dans laquelle:
HFR1 comprend SEQ ID NO: 1;
HFR2 comprend SEQ ID NO: 2;
HFR3 comprend SEQ ID NO: 3;
HFR4 comprend SEQ ID NO: 4;
LFR1 comprend SEQ ID NO: 5;
LFR2 comprend SEQ ID NO: 6;
LFR3 comprend SEQ ID NO: 7, et;
LFR4 comprend SEQ ID NO: 8.

8. Immunoglobuline selon l'une quelconque des revendications précédentes, dans laquelle:
CDR-L1 a une longueur de 5-14 résidus d'acides aminés,
CDR-L2 a une longueur de 5-7 résidus d'acides aminés,
CDR-L3 a une longueur de 5-15 résidus d'acides aminés,
CDR-H1 a une longueur de 5-7 résidus d'acides aminés,
CDR-H2 a une longueur de 16-18 résidus d'acides aminés, et
CDR-H3 a une longueur de 9-21 résidus d'acides aminés.

9. Molécule d'immunoglobuline isolée selon la revendication 8, dans laquelle:
CDR-H1 consiste en 5 résidus d'acides aminés,
CDR-H2 consiste en 17 résidus d'acides aminés,
CDR-H3 consiste en 9 à 19 résidus d'acides aminés,
CDR-L1 consiste en 8, 9, 10 ou 13 résidus d'acides aminés,
CDR-L2 consiste en 7 résidus d'acides aminés et
CDR-L3 consiste en 8 à 12 résidus d'acides aminés.

10. Molécule d'immunoglobuline selon la revendication 9, dans laquelle CDR-H3 consiste en 14 à 19 résidus d'acides aminés.

11. Molécule d'immunoglobuline selon l'une quelconque des revendications précédentes, comprenant en outre un linker qui joint (a) à (b).

12. Molécule d'immunoglobuline selon l'une quelconque des revendications précédentes, comprenant en outre un signal de ciblage cellulaire et/ou un marqueur.

13. Molécule d'immunoglobuline selon la revendication 12, dans laquelle ledit signal de ciblage cellulaire est sélectionné dans le groupe consistant en un peptide de ciblage apoplastique, un peptide de ciblage du réticulum endoplasmique, un peptide de ciblage d'une vacuole, un peptide de ciblage d'un corps protéique et un peptide de ciblage d'un chloroplaste.

14. Molécule d'acide nucléique isolée codant pour la molécule d'immunoglobuline selon l'une quelconque des revendications 1 à 13.

15. Molécule d'acide nucléique isolée selon la revendication 14, comprenant une séquence de nucléotides sélectionnée dans le groupe consistant en SEQ ID NO: 9, SEQ ID NO: 10, SEQ ID NO: 11, SEQ ID NO: 12, SEQ ID NO: 13 et SEQ ID NO: 14.

16. Banque recombinante comprenant une ou plusieurs molécules d'acide nucléique isolées selon la revendication 14 ou la revendication 15.

17. Vecteur comprenant une molécule d'acide nucléique isolée selon la revendication 14 ou la revendication 15, en liaison fonctionnelle avec un promoteur.

18. Vecteur selon la revendication 17, comprenant une séquence de nucléotides codant pour un peptide de ciblage cellulaire.

19. Cellule hôte comprenant une molécule d'acide nucléique selon la revendication 14 ou la revendication 15, ou vecteur selon la revendication 16 ou la revendication 17.

20. Cellule hôte selon la revendication 19, où ledit hôte est une cellule bactérienne, une cellule de levure, une cellule d'algue, une cellule d'insecte, une cellule mammalienne ou une cellule végétale.

21. Procédé pour préparer une banque recombinante exprimant des molécules d'immunoglobuline selon l'une quelconque des revendications 1 à 13, où ledit procédé consiste à préparer une ou plusieurs molécules d'acide nucléique codant pour les molécules d'immunoglobuline, ou des domaines de celles-ci, et à exprimer lesdites molécules d'acide nucléique dans une cellule hôte appropriée, où l'expression dudit acide nucléique produit une banque recombinante exprimant les molécules d'immunoglobuline ou les domaines de celles-ci.

22. Procédé selon la revendication 21, dans lequel les molécules d'immunoglobuline sont sélectionnées dans le groupe consistant en scFv, des diabodies, des triabodies et des tétrabodies.

23. Procédé selon la revendication 21, dans lequel les molécules d'immunoglobuline comprennent des CDR randomisées.

24. Procédé pour identifier une molécule d'immunoglobuline à partir d'une banque recombinante exprimant des molécules d'immunoglobuline selon l'une quelconque des revendications 1 à 13, où la molécule d'immunoglobuline se lie à un antigène prédéterminé, consistant à mettre en contact les molécules d'immunoglobuline avec l'antigène prédéterminé et à tester la liaison entre ceux-ci.

25. Procédé selon la revendication 24, consistant en outre à identifier la molécule d'acide nucléique qui code pour la molécule d'immunoglobuline ou un domaine de celle-ci identifié(e) dans la revendication 24.

26. Utilisation de la molécule d'immunoglobuline selon l'une quelconque des revendications 1 à 13 dans des essais diagnostiques *in vitro.*

27. Utilisation selon la revendication 26, dans laquelle l'essai est un essai de criblage *in vitro* pour détecter des molécules qui se lient à ladite molécule d'immunoglobuline.

28. Utilisation selon la revendication 26 ou la revendication 27, dans laquelle les essais sont sélectionnés dans le groupe consistant en des essais ELISA, des essais de présentation sur phage et des puces protéiques.

29. Utilisation selon la revendication 26 ou la revendication 27, dans laquelle la molécule d'immunoglobuline peut être un scFv, un scFv bispécifique ou un scFv multivalent.

30. Procédé pour produire une molécule d'immunoglobuline ayant un domaine variable chimérique, où la molécule d'immunoglobuline s'accumule dans une cellule hôte à au moins 0,15 % de la protéine soluble totale, consistant à:
(a) déterminer une séquence d'acides aminés d'une molécule d'immunoglobuline aviaire comprenant un domaine variable, où ledit domaine variable contient des régions charpentes, et des régions déterminant la complémentarité (CDR), et à déterminer une séquence d'acides aminés d'une molécule d'immunoglobuline présélectionnée, qui est spécifique pour un antigène, ladite immunoglobuline présélectionnée comprenant un domaine variable qui contient des régions charpentes et des CDR, où les régions charpentes et les CDR des molécules d'immunoglobuline sont selon le système de numérotation de Kabat,
(b) comparer les séquences d'acides aminés des domaines variables de l'immunoglobuline aviaire et de l'immunoglobuline présélectionnée afin d'identifier des différences de résidus d'acides aminés à des positions correspondantes dans les régions charpentes et les CDR des anticorps aviaires et présélectionnés qui sont nécessaires pour maintenir une conformation des CDR,
(c) préparer une molécule d'acide nucléique codant pour une molécule d'immunoglobuline comprenant un domaine variable, où les CDR du domaine variable sont les CDR de la molécule d'immunoglobuline présélectionnée et où les régions charpentes du domaine variable sont les régions charpentes aviaires à condition qu'une ou plusieurs des positions de résidus d'acides aminés, identifiées en (b) comme ayant des résidus d'acides aminés différents dans le domaine variable de la molécule d'immunoglobuline aviaire par comparaison au domaine variable de la molécule d'immunoglobuline présélectionnée, contiennent le résidu d'acide aminé présent dans le domaine variable de l'immunoglobuline présélectionnée, et
(d) exprimer la molécule d'acide nucléique de (c) afin de produire une molécule d'immunoglobuline ayant un domaine variable chimérique.

31. Procédé selon la revendication 30, dans lequel la séquence d'acides aminés d'immunoglobuline aviaire comprend SEQ ID NO: 51.

32. Procédé selon la revendication 30, dans lequel les positions de résidus d'acides aminés qui sont nécessaires pour maintenir une conformation des CDR de la molécule d'immunoglobuline présélectionnée sont déterminées par les procédés de Kabat, Chothia et le procédé par contact.

33. Procédé selon la revendication 30, dans lequel l'immunoglobuline ayant un domaine variable chimérique est un V_{L}, V_{H}, scFv, diabody, triabody ou tétrabody.

34. Plante transgénique, ou partie de celle-ci, exprimant une molécule d'immunoglobuline selon l'une quelconque des revendications 1 à 13.

35. Plante transgénique ou partie de celle-ci selon la revendication 34, dans laquelle la molécule d'immunoglobuline est spécifique pour un agent pathogène végétal.


### SEQUENCE LISTING

<110> Fraunhofer -Gesselschaft zur Foredung der angewandten Forschung E. V
<120> Immonuglobulin having Particular Framework Scaffold and Methods of Making and Using
<130> SMW/FP6217152
<140> EP 02759649.3
   <141> 2002-09-13
<150> PCT/US02/29003
   <151> 2002-09-14
<160> 84
<170> PatentIn version 3.3
<210> 1
   <211> 30
   <212> PRT
   <213> Artificial sequence
<220>
   <223> This is a heavy chain fremawork region 1 (HFR1)
<400> 1
   Ala Val Thr Leu Asp Glu Ser Gly Gly Gly Leu Gln Thr Pro Gly Gly
   1               5                   10                  15
   Gly Leu Ser Leu Val Cys Lys Ala Ser Gly Phe Asp Phe Ser
               20                  25                  30
<210> 2
   <211> 14
   <212> PRT
   <213> Artificial sequence
<220>
   <223> This is a heavy chain fremawork region 1 (HFR2)
<400> 2
   Trp Val Arg Gln Ala Pro Gly Lys Gly Leu Glu Trp Val Ala
   1               5                   10
<210> 3
   <211> 32
   <212> PRT
   <213> Artificial sequence
<220>
   <223> This is a heavy chain fremawork region 1 (HFR3)
<400> 3
   Arg Ala Thr Ile Ser Arg Asp Asn Gly Gln Ser Thr Val Arg Leu Gln
   1               5                   10                  15
   Leu Asn Asn Leu Arg Ala Glu Asp Thr Ala Thr Tyr Tyr Cys Ala Lys
               20                  25                  30
<210> 4
   <211> 9
   <212> PRT
<213> Artificial sequence
<220>
   <223> This is a heavy chain fremawork region 1 (HFR4)
<400> 4
   Trp Gly His Gly Thr Glu Val Thr Val
   1 5
<210> 5
   <211> 23
   <212> PRT
   <213> Artificial sequence
<220>
   <223> This is a heavy chain fremawork region 1 (LRF1)
<220>
   <221> MISC_FEATURE
   <222> (10) . . (10)
   <223> The amino acid at position 10 may be absent or may be any amino acid.
<400> 5
   Ala Pro Ala Leu Thr Gln Pro Ser Ser Xaa Val Ser Ala Asn Pro Gly
   1 5 10 15
   Glu Thr Val Lys Ile Thr Cys
   20
<210> 6
   <211> 16
   <212> PRT
   <213> Artificial sequence
<220>
   <223> This is a heavy chain fremawork region 2 (LRF2)
<400> 6
   Trp Phe Gln Gln Lys Ser Pro Gly Ser Ala Pro Val Thr Val Ile Tyr
   1 5 10 15
<210> 7
   <211> 32
   <212> PRT
   <213> Artificial sequence
<220>
   <223> This is a heavy chain fremawork region 3 (LRF3)
<400> 7
   Asp Ile Pro Ser Arg Phe Ser Gly Ser Lys Ser Gly Ser Thr His Thr
   1               5                   10                  15
   Leu Thr Ile Thr Gly Val Gln Val Glu Asp Glu Ala Val Tyr Phe Cys
               20                  25                  30
<210> 8
   <211> 13
   <212> PRT
   <213> Artificial sequence
<220>
   <223> This is a heavy chain fremawork region 4 (LRF3)
<400> 8
   Phe Gly Ala Gly Thr Thr Leu Thr Val Leu Gly Gln Pro
   1               5                   10
<210> 9
   <211> 22
   <212> DNA
   <213> Artificial sequence
<220>
   <223> A VH cDNA primer
<400> 9
   aggggtggag gacctgcacc tc 22
<210> 10
   <211> 22
   <212> DNA
   <213> Artificial sequence
<220>
   <223> A VL cDNA primer
<400> 10
   cggtggggga catctgagtg gg 22
<210> 11
   <211> 45
   <212> DNA
   <213> Artificial sequence
<220>
   <223> A chick VH 5' primer
<400> 11
   ccttggcca gccggccatg gctgccgtga cgttggacga gtccc 45
<210> 12
   <211> 34
   <212> DNA
   <213> Artificial sequence
<220>
   <223> A chick VH 3' primer
<400> 12
   tggaggtgac ctcggtcccg tggtccatg cgtc 34
<210> 13
   <211> 37
   <212> DNA
   <213> Artificial sequence
<220>
   <223> A chick VL 5' primer
<400> 13
   tccaggcgcg cctgcgctga ctcagccgtc ctcggtg 37
<210> 14
   <211> 40
   <212> DNA
   <213> Artificial sequence
<220>
   <223> A chick VL 3' primer
<400> 14
   tcgaggatgc gcggccgcgt cgacgggctg gcctaggacg 40
<210> 15
   <211> 134
   <212> PRT
   <213> Artificial sequence
<220>
   <223> Heavy chain scaffold
<220>
   <221>MISC_FEATURE
   <222> (36) .. (36)
   <223> The amino acid at position 36 may be any amino acid
<220>
   <221>MISC_FEATURE
   <222> (37) .. (37)
   <223> The amino acid at position 37 may be any amino acid
<220>
   <221>MISC_FEATURE
   <222> (56) .. (56)
   <223> The amino acid at position 56 may be any amino acid
<220>
   <221>MISC_FEATURE
   <222> (57) .. (57)
   <223> The amino acid at position 57 may be any amino acid
<220>
   <221>MISC_FEATURE
   <222> (58) .. (58)
   <223> The amino acid at position 58 may be any amino acid
<220>
   <221>MISC_FEATURE
   <222> (119) .. (119)
   <223> The amino acid at position 119 may be any amino acid
<220>
   <221>MISC_FEATURE
   <222> (120) .. (120)
   <223> The amino acid at position 120 may be any amino acid
<400> 15
   Ala Val Thr Leu Asp Glu Ser Gly Gly Gly Leu Gln Thr Pro Gly Gly
   1               5                   10                  15
   Gly Leu Ser Leu Val Cys Lys Ala Ser Gly Phe Asp Phe Ser Ser Tyr
               20                  25                  30
   Asp Met Val Xaa Xaa Trp Val Arg Gln Ala Pro Gly Lys Gly Leu Glu
           35                  40                  45
   Trp Val Ala Gly Ile Ser Ser Xaa Xaa Xaa Gly Thr Ser Pro Asn Tyr
       50                  55                  60
   Gly Ala Ala Val Lys Gly Arg Ala Thr Ile Ser Arg Asp Asn Gly Gln
   65                  70                  75                  80
   Ser Thr Val Arg Leu Gln Leu Asn Asn Leu Arg Ala Glu Asp Thr Ala
                   85                  90                  95
   Thr Tyr Tyr Cys Ala Lys Asn Glu Gly Ala Asp Trp Cys Gly His Tyr
               100                 105                 110
   Tyr Cys Ser Val Ala Tyr Xaa Xaa Ile Asp Ala Trp Gly His Gly Thr
           115                 120                 125
   Glu Val Thr Val Ser Ser
       130
<210> 16
   <211> 123
   <212> PRT
   <213> Artificial sequence
<220>
   <223> Heavy chain scaffold
<220>
   <221>MISC_FEATURE
   <222> (10) .. (10)
   <223> The amino acid at position 10 may be any amino acid
<220>
   <223> Heavy chain scaffold
<220>
   <221>MISC_FEATURE
   <222> (28) .. (28)
   <223> The amino acid at position 28may be any amino acid
<220>
   <221>MISC_FEATURE
   <222> (29) .. (29)
   <223> The amino acid at position 29 may be any amino acid
<220>
   <221>MISC_FEATURE
   <222> (30) .. (30)
   <223> The amino acid at position 30 may be any amino acid
<220>
   <221>MISC_FEATURE
   <222> (31) .. (31)
   <223> The amino acid at position 31 may be any amino acid
<220>
   <221>MISC_FEATURE
   <222> (32) .. (32)
   <223> The amino acid at position 32 may be any amino acid
<220>
   <221>MISC_FEATURE
   <222> (33) .. (33)
   <223> The amino acid at position 33 may be any amino acid
<220>
   <221>MISC_FEATURE
   <222> (34) .. (34)
   <223> The amino acid at position 34 may be any amino acid
<220>
   <221>MISC_FEATURE
   <222> (35) .. (35)
   <223> The amino acid at position 35 may be any amino acid
<220>
   <221>MISC_FEATURE
   <222> (103) .. (103)
   <223> The amino acid at position 103 may be any amino acid
<220>
   <221>MISC_FEATURE
   <222> (104) .. (104)
   <223> The amino acid at position 104 may be any amino acid
<220>
   <221>MISC_FEATURE
   <222> (105) .. (105)
   <223> The amino acid at position 105 may be any amino acid
<220>
   <221>MISC_FEATURE
   <222> (106) .. (106)
   <223> The amino acid at position 106 may be any amino acid
<220>
   <221>MISC_FEATURE
   <222> (107) .. (107)
   <223> The amino acid at position 107 may be any amino acid
<220>
   <221>MISC_FEATURE
   <222> (108) .. (108)
   <223> The amino acid at position 108 may be any amino acid
<400> 16
   Ala Pro Ala Leu Thr Gln Pro Ser Ser Xaa Val Ser Ala Asn Pro Gly
   1               5                   10                  15
   Glu Thr Val Lys Ile Thr Cys Ser Gly Gly Ser Xaa Xaa Xaa Xaa Xaa
               20                  25                  30
   Xaa Xaa Xaa Gly Asp His Tyr Gly Trp Phe Gln Gln Lys Ser Pro Gly
           35                  40                  45
   Ser Ala Pro Val Thr Val Ile Tyr Asn Asn Asp Asn Arg Pro Ser Asp
       50                  55                  60
   Ile Pro Ser Arg Phe Ser Gly Ser Lys Ser Gly Ser Thr His Thr Leu
   65                  70                  75                  80
   Thr Ile Thr Gly Val Gln Val Glu Asp Glu Ala Val Tyr Phe Cys Gly
                   85                  90                  95
   Asn Arg Asp Ser Ser Ala Xaa Xaa Xaa Xaa Xaa Xaa Gly Ile Phe Gly
               100                 105                 110
   Ala Gly Thr Thr Leu Thr Val Leu Gly Gln Pro
           115                 120
<210> 17
   <211> 18
   <212> PRT
   <213> Artificial sequence
<220>
   <223> Synthetic linker
<400> 17
   Gly Ser Thr Ser Gly Ser Gly Lys Pro Gly Pro Gly Glu Gly Ser Thr
   1               5                   10                  15
   Lys Gly
<210> 18
   <211> 57
   <212> DNA
   <213> Artificial sequence
<220>
   <223> Murine heavy chain antibody 24 codon optimiezd for expression in pea, wheat and tobacco
<400> 18
   atggagtgga gctggatcctt tctctttctc ctctcaggaa ctgcaggtgt tcactcc 57
<210> 19
   <211> 66
   <212> DNA
   <213> Artificial sequence
<220>
   <223> Murine heavy chain antibody 24 codon optimiezd for expression in pea, wheat and tobacco
<400> 19
   atggactttc aagtgcagat tttcagcttc ctcctcatca gcgcctcagt tatcatctct      60
   agggga                                                                66
<210> 20
   <211> 177
   <212> DNA
   <213> Artificial sequence
<220>
   <223> pTMZ1 flanking sequence
<400> 20
   cggggatctc gatcccgcga aattaatacg actcactata ggggaattgt gagcggataa      60
   caattcccct gtagaaataa ttttgtttaa ctttaagaag gagatataca tatgaaatac 120
   ctattgccta cggcagccgc tggattgtta ttactcgcgg cccagccggc catggcc 177
<210> 21
   <211> 100
   <212> DNA
   <213> Artificial sequence
<220>
   <223> pTMZ1 subsequence
<400> 21
   gtcgacagag aacaaaaact catctcagaa gaggatctgg gtgcactcga ccatcaccat 60
   caccatcacg tctagagaat tcctcgaggg ctcttcctgc 100
<210> 22
   <211> 90
   <212> DNA
   <213> Artificial sequence
<220>
   <223> Sequence encoding HFR1 SEQ ID NO: 1
<400> 22
   gccgtgacgt tggacgagag cgggggcggc ctccagacgc cgggtggggg cctcagcctc 60
   gtctgcaagg ccagcgggtt tgacttcagc 90
<210> 23
   <211> 42
   <212> DNA
   <213> Artificial sequence
<220>
   <223> Sequence encoding HFR2 SEQ ID NO: 2
<400> 23
   tgggtgcgcc aggcgccggg caagggcctg gaatgggtcg ct 42
<210> 24
   <211> 96
   <212> DNA
   <213> Artificial sequence
<220>
   <223> Sequence encoding HFR3 SEQ ID NO: 3
<400> 24
   cgtgccacca tcagccgcga caacgggcag agcaccgtgc gcctgcagct gaacaacctc 60
   cgtgctgagg acaccgccac ctactactgc gccaaa 96
<210> 25
   <211> 27
   <212> DNA
   <213> Artificial sequence
<220>
   <223> Sequence encoding HFR4 SEQ ID NO: 4
<400> 25
   tggggccatg ggaccgaggt caccgtc 27
<210> 26
   <211> 54
   <212> DNA
   <213> Artificial sequence
<220>
   <223> Linker sequence
<400> 26
   ggcagcacca gcggcagcgg taaaccgggc ccgggggagg gtagcaccaa gggc 54
<210> 27
   <211> 66
   <212> DNA
   <213> Artificial sequence
<220>
   <223> Sequence encoding LFR1 SEQ ID NO: 5
<400> 27
   gcgccggcgc tgacccagcc gagctcggtg agcgcaaacc cgggtgaaac cgtcaagatc 60
   acctgc 66
<210> 28
   <211> 48
   <212> DNA
   <213> Artificial sequence
<220>
   <223> Sequence encoding LFR2 SEQ ID NO: 6
<400> 28
   tggttccagc agaagagccc gggcagcgcc ccggtcaccg tgatctat 48
<210> 29
   <211> 96
   <212> DNA
   <213> Artificial sequence
<220>
   <223> Sequence encoding LFR3 SEQ ID NO: 7
<400> 29
   gacatcccga gccgcttcag cggttccaaa agcggctcca cccatacgtt aaccatcacg 60
   ggggtccaag tcgaggacga ggctgtctat ttctgt 96
<210> 30
   <211> 39
   <212> DNA
   <213> Artificial sequence
<220>
   <223> Sequence encoding LFR3 SEQ ID NO: 8
<400> 30
   tttggggccg ggacgaccct gaccgtcctt ggccagccg 39
<210> 31
   <211> 90
   <212> DNA
   <213> Artificial sequence
<220>
   <223> Sequence encoding HFR1 SEQ ID NO: 1
<400> 31
   gccgtgacgt tggacgagtc cgggggcggc ctccagacgc ccggaggggg cctcagcctc 60
   gtctgcaagg cctccgggtt tactttctct 90
<210> 32
   <211> 42
   <212> DNA
   <213> Artificial sequence
<220>
   <223> Sequence encoding HFR2 SEQ ID NO: 2
<400> 32
   tgggtgcgac aggcgccggg taagggtcta gaatgggtcg ct 42
<210> 33
   <211> 96
   <212> DNA
   <213> Artificial sequence
<220>
   <223> Sequence encoding HFR3 SEQ ID NO: 3
<400> 33
   cgtgccacta tctcgagaga caacggtcag tctactgtga ggctgcagct gaacaacctg 60
   cgtgctgagg acactgctac ctactactgc gccaaa 96
<210> 34
   <211> 27
   <212> DNA
   <213> Artificial sequence
<220>
   <223> Sequence encoding HFR4 SEQ ID NO: 4
<400> 34
   tggggtcacg gtactgaggt caccgtc 27
<210> 35
   <211> 60
   <212> DNA
   <213> Artificial sequence
<220>
   <223> Linker sequence
<400> 35
   tcctcaggvt ccacctcagg ctccggtaaa cctggcccag gggagggatc aactaagggc 60
<210> 36
   <211> 66
   <212> DNA
   <213> Artificial sequence
<220>
   <223> Sequence encoding LFR1 SEQ ID NO: 5
<400> 36
   gcgcctgcgc tgactcagcc gtcctcggtg tctgcaaacc cgggtgaaac tgtcaagatc 60
   acttgc 66
<210> 37
   <211> 48
   <212> DNA
   <213> Artificial sequence
<220>
   <223> Sequence encoding LFR2 SEQ ID NO: 6
<400> 38
   tggttccagc agaagtctcc gagctctgcc ccggtcactg tgatctat 48
<210> 38
   <211> 96
   <212> DNA
   <213> Artificial sequence
<220>
   <223> Sequence encoding LFR3 SEQ ID NO: 7
<400> 38
   gacatccctt cacgattctc cggttccaaa tctggttcta ctcacactct gactatcact 60
   agtgtccaag tcgaggacga ggctgtctac ttctgc 96
<210> 39
   <211> 39
   <212> DNA
   <213> Artificial sequence
<220>
   <223> Sequence encoding LFR4 SEQ ID NO: 8
<400> 39
   tttggtgccg gtactactct gactgtcctg ggtcagccg 39
<210> 40
   <211> 90
   <212> DNA
   <213> Artificial sequence
<220>
   <223> Sequence encoding HFR1 SEQ ID NO: 1
<400> 40
   gccgtgactt tggacgagtc cggaggcgga cttcagactc ctggaggagg cctttcactt 60
   gtttgcaagg cctcaggatt cgatttctca 90
<210> 41
   <211> 42
   <212> DNA
   <213> Artificial sequence
<220>
   <223> Sequence encoding HFR2 SEQ ID NO: 2
<400> 41
   tgggttcgtc aagccccagg aaagggactt gagtgggttg cc 42
<210> 42
   <211> 96
   <212> DNA
   <213> Artificial sequence
<220>
   <223> Sequence encoding HFR3 SEQ ID NO: 3
<400> 42
   cgtgccacta tctcacgtga taacggacaa tcaactgttc gtcttcaact taacaacctt 60
   cgtgccgagg atactgccac ttactactgc gccaag 96
<210> 43
   <211> 27
   <212> DNA
   <213> Artificial sequence
<220>
   <223> Sequence encoding HFR4 SEQ ID NO: 4
<400> 43
   tggggacacg gaactaggt tactgtt 27
<210> 44
   <211> 60
   <212> DNA
   <213> Artificial sequence
<220>
   <223> Linker sequence
<400> 44
   tcctcaggat caacttcagg atcaggaaag cctggcccag gagagggatc aactaagggc 60
<210> 45
   <211> 66
   <212> DNA
   <213> Artificial sequence
<220>
   <223> Sequence encoding LFR1 SEQ ID NO: 5
<400> 45
   gcccctgccc ttactcaacc atcatcagtt tcagccaacc ctggagagac tgttaagatc 60
   acttgc 66
<210> 46
   <211> 48
   <212> DNA
   <213> Artificial sequence
<220>
   <223> Sequence encoding LFR2 SEQ ID NO: 6
<400> 46
   tggttccaac aaaagtcacc tggatcagcc cctgttactg ttatctac 48
<210> 47
   <211> 96
   <212> DNA
   <213> Artificial sequence
<220>
   <223> Sequence encoding LFR3 SEQ ID NO: 7
<400> 47
   gatatccctt cacgtttctc aggatcaaag tcaggctcaa ctcacactct tactatcact 60
   ggagttcaag ttgaggatga ggccgtttac ttctgc 96
<210> 48
   <211> 39
   <212> DNA
   <213> Artificial sequence
<220>
   <223> Sequence encoding LFR4 SEQ ID NO: 8
<400> 48
   ttcggagccg gaactaccct tactgttctt ggacaacca 39
<210> 49
   <211> 402
   <212> DNA
   <213> Artificial sequence
<220>
   <223> Chicken scFv scaffold sequence encoding jeavy chain variable region SEQ ID NO: 15
<220>
   <221>misc_feature
   <222> (106) .. (106)
   <223> The residue at position 106 may be absent or any nucleotide
<220>
   <221>misc_feature
   <222> (107) .. (107)
   <223> The residue at position 107 may be absent or any nucleotide
<220>
   <221>misc_feature
   <222> (108) .. (108)
   <223> The residue at position 108 may be absent or any nucleotide
<220>
   <221>misc_feature
   <222> (109) .. (109)
   <223> The residue at position 109 may be absent or any nucleotide
<220>
   <221>misc_feature
   <222> (110) .. (110)
   <223> The residue at position 110 may be absent or any nucleotide
<220>
   <221>misc_feature
   <222> (111) .. (111)
   <223> The residue at position 111 may be absent or any nucleotide
<220>
   <221>misc_feature
   <222> (166) .. (166)
   <223> The residue at position 166 may be absent or any nucleotide
<220>
   <221>misc_feature
   <222> (167) .. (167)
   <223> The residue at position 167 may be absent or any nucleotide
<220>
   <221>misc_feature
   <222> (168) .. (168)
   <223> The residue at position 168 may be absent or any nucleotide
<220>
   <221>misc_feature
   <222> (169) .. (169)
   <223> The residue at position 169 may be absent or any nucleotide
<220>
   <221>misc_feature
   <222> (170) .. (170)
   <223> The residue at position 170 may be absent or any nucleotide
<220>
   <221>misc_feature
   <222> (171) .. (171)
   <223> The residue at position 171 may be absent or any nucleotide
<220>
   <221>misc_feature
   <222> (172) .. (172)
   <223> The residue at position 172 may be absent or any nucleotide
<220>
   <221>misc_feature
   <222> (173) .. (173)
   <223> The residue at position 173 may be absent or any nucleotide
<220>
   <221>misc_feature
   <222> (174) .. (174)
   <223> The residue at position 174 may be absent or any nucleotide
<220>
   <221>misc_feature
   <222> (355) .. (355)
   <223> The residue at position 355 may be absent or any nucleotide
<220>
   <221>misc_feature
   <222> (356) .. (356)
   <223> The residue at position 356 may be absent or any nucleotide
<220>
   <221>misc_feature
   <222> (357) .. (357)
   <223> The residue at position 357may be absent or any nucleotide
<220>
   <221>misc_feature
   <222> (358) .. (358)
   <223> The residue at position 358may be absent or any nucleotide
<220>
   <221>misc_feature
   <222> (359) .. (359)
   <223> The residue at position 359 may be absent or any nucleotide
<220>
   <221>misc_feature
   <222> (360) .. (360)
   <223> The residue at position 360 may be absent or any nucleotide
<400> 49
   gccgtgacgt tggacgagtc cgggggcggc ctccagacgc ccggaggggg cctcagcctc     60
   gtctgcaagg cctccgggtt tgacttcagc agttatgaca tggtgnnnnn ntgggtgcga    120
   caggcgcccg gcaagggact ggaatgggtc gctggtatta gcagcnnnnn nnnnggtacg    180
   tccccaaact acggggcggc ggtgaagggc cgtgccacca tctcgaggga caacgggcag    240
   agcacagtga ggctgcagct gaacaacctc agggctgagg acaccgccac ctactactgc    300
   gccaaaaatg agggtgctga ttggtgtggt cattattatt gtagtgtagc ctacnnnnnn    360
   atcgacgcat ggggccacgg gaccgaggtc accgtctcct ca                       402
<210> 80
   <211> 369
   <212> DNA
   <213> Artificial sequence
<220>
   <223> Chicken scFc scaffold for light chain variable region SEQ ID NO: 16
<220>
   <221>misc_feature
   <222> (28) .. (28)
   <223> The residue at position 28 may be absent or any nucleotide
<220>
   <221>misc_feature
   <222> (29) .. (29)
   <223> The residue at position 29 may be absent or any nucleotide
<220>
   <221>misc_feature
   <222> (30) .. (30)
   <223> The residue at position 30 may be absent or any nucleotide
<220>
   <221>misc_feature
   <222> (82) .. (82)
   <223> The residue at position 82 may be absent or any nucleotide
<220>
   <221>misc_feature
   <222> (83) .. (83)
   <223> The residue at position 83 may be absent or any nucleotide
<220>
   <221>misc_feature
   <222> (84) .. (84)
   <223> The residue at position 84 may be absent or any nucleotide
<220>
   <221>misc_feature
   <222> (85) .. (85)
   <223> The residue at position 85 may be absent or any nucleotide
<220>
   <221>misc_feature
   <222> (86) .. (86)
   <223> The residue at position 29 may be absent or any nucleotide
<220>
   <221>misc_feature
   <222> (87) .. (87)
   <223> The residue at position 87 may be absent or any nucleotide
<220>
   <221>misc_feature
   <222> (88) .. (88)
   <223> The residue at position 88 may be absent or any nucleotide
<220>
   <221>misc_feature
   <222> (89) .. (89)
   <223> The residue at position 99 may be absent or any nucleotide
<220>
   <221>misc_feature
   <222> (90) .. (90)
   <223> The residue at position 90 may be absent or any nucleotide
<220>
   <221>misc_feature
   <222> (91) .. (91)
   <223> The residue at position 91 may be absent or any nucleotide
<220>
   <221>misc_feature
   <222> (92) .. (92)
   <223> The residue at position 92 may be absent or any nucleotide
<220>
   <221>misc_feature
   <222> (93) .. (93)
   <223> The residue at position 93 may be absent or any nucleotide
<220>
   <221>misc_feature
   <222> (94) .. (94)
   <223> The residue at position 94 may be absent or any nucleotide
<220>
   <221>misc_feature
   <222> (95) .. (95)
   <223> The residue at position 95 may be absent or any nucleotide
<220>
   <221>misc_feature
   <222> (96) .. (96)
   <223> The residue at position 96 may be absent or any nucleotide
<220>
   <221>misc_feature
   <222> (97) .. (97)
   <223> The residue at position 97 may be absent or any nucleotide
<220>
   <221>misc_feature
   <222> (98) .. (98)
   <223> The residue at position 98 may be absent or any nucleotide
<220>
   <221>misc_feature
   <222> (99) .. (99)
   <223> The residue at position 99 may be absent or any nucleotide
<220>
   <221>misc_feature
   <222> (100) .. (100)
   <223> The residue at position 100 may be absent or any nucleotide
<220>
   <221>misc_feature
   <222> (101) .. (101)
   <223> The residue at position 101 may be absent or any nucleotide
<220>
   <221>misc_feature
   <222> (102) .. (102)
   <223> The residue at position 102 may be absent or any nucleotide
<220>
   <221>misc_feature
   <222> (103) .. (103)
   <223> The residue at position 103 may be absent or any nucleotide
<220>
   <221>misc_feature
   <222> (104) .. (104)
   <223> The residue at position 104 may be absent or any nucleotide
<220>
   <221>misc_feature
   <222> (105) .. (105)
   <223> The residue at position 105 may be absent or any nucleotide
<220>
   <221>misc_feature
   <222> (307) .. (307)
   <223> The residue at position 307 may be absent or any nucleotide
<220>
   <221>misc_feature
   <222> (308) .. (308)
   <223> The residue at position 308 may be absent or any nucleotide
<220>
   <221>misc_feature
   <222> (309) .. (309)
   <223> The residue at position 309 may be absent or any nucleotide
<220>
   <221>misc_feature
   <222> (310) .. (310)
   <223> The residue at position 310 may be absent or any nucleotide
<220>
   <221>misc_feature
   <222> (311) .. (311)
   <223> The residue at position 311 may be absent or any nucleotide
<220>
   <221>misc_feature
   <222> (312) .. (312)
   <223> The residue at position 312 may be absent or any nucleotide
<220>
   <221>misc_feature
   <222> (313) .. (313)
   <223> The residue at position 313 may be absent or any nucleotide
<220>
   <221>misc_feature
   <222> (314) .. (314)
   <223> The residue at position 314 may be absent or any nucleotide
<220>
   <221>misc_feature
   <222> (315) .. (315)
   <223> The residue at position 315 may be absent or any nucleotide
<220>
   <221>misc_feature
   <222> (316) .. (316)
   <223> The residue at position 316 may be absent or any nucleotide
<220>
   <221>misc_feature
   <222> (317) .. (317)
   <223> The residue at position 317 may be absent or any nucleotide
<220>
   <221>misc_feature
   <222> (318) .. (318)
   <223> The residue at position 318 may be absent or any nucleotide
<220>
   <221>misc_feature
   <222> (319) .. (319)
   <223> The residue at position 319 may be absent or any nucleotide
<220>
   <221>misc_feature
   <222> (320) .. (320)
   <223> The residue at position 320 may be absent or any nucleotide
<220>
   <221>misc_feature
   <222> (321) .. (321)
   <223> The residue at position 321 may be absent or any nucleotide
<220>
   <221>misc_feature
   <222> (322) .. (322)
   <223> The residue at position 322 may be absent or any nucleotide
<220>
   <221>misc_feature
   <222> (323) .. (323)
   <223> The residue at position 323 may be absent or any nucleotide
<220>
   <221>misc_feature
   <222> (324) .. (324)
   <223> The residue at position 324 may be absent or any nucleotide
<400> 50
<210> 51
   <211> 253
   <212> PRT
   <213> Artificial sequence
<220>
   <223> ScFvG19
<400> 51
   Ala Val Thr Leu Asp Glu Ser Gly Gly Gly Leu Gln Thr Pro Gly Gly
   1               5                   10                  15
   Gly Leu Ser Leu Val Cys Lys Ala Ser Gly Phe Asp Phe Ser Ser Tyr
               20                  25                  30
   Asp Met Val Trp Val Arg Gln Ala Pro Gly Lys Gly Leu Glu Trp Val
           35                  40                  45
   Ala Gly Ile Ser Ser Gly Thr Ser Pro Asn Tyr Gly Ala Ala Val Lys
       50                  55                  60
   Gly Arg Ala Thr Ile Ser Arg Asp Asn Gly Gln Ser Thr Val Arg Leu
   65                  70                  75                  80
   Gln Leu Asn Asn Leu Arg Ala Glu Asp Thr Ala Thr Tyr Tyr Cys Ala
                   85                  90                  95
   Lys Asn Glu Gly Ala Asp Trp Cys Gly His Tyr Tyr Cys Ser Val Ala
               100                 105                 110
   Tyr Ile Asp Ala Trp Gly His Gly Thr Glu Val Thr Val Ser Ser Gly
           115                 120                 125
   Ser Thr Ser Gly Ser Gly Lys Pro Gly Pro Gly Glu Gly Ser Thr Lys
       130                 135                 140
   Gly Ala Pro Ala Leu Thr Gln Pro Ser Ser Val Ser Ala Asn Pro Gly
   145                 150                 155                 160
   Glu Thr Val Lys Ile Thr Cys Ser Gly Gly Ser Gly Asp His Tyr Gly
                   165                 170                 175
   Trp Phe Gln Gln Lys Ser Pro Gly Ser Ala Pro Val Thr Val Ile Tyr
               180                 185                 190
   Asn Asn Asp Asn Arg Pro Ser Asp Ile Pro Ser Arg Phe Ser Gly Ser
           195                 200                 205
   Lys Ser Gly Ser Thr His Thr Leu Thr Ile Thr Gly Val Gln Val Glu
       210                 215                 220
   Asp Glu Ala Val Tyr Phe Cys Gly Asn Arg Asp Ser Ser Ala Gly Ile
   225                 230                 235                 240
   Phe Gly Ala Gly Thr Thr Leu Thr Val Leu Gly Gln Pro
                   245                 250
<210> 52
   <211> 253
   <212> PRT
   <213> Artificial sequence
<220>
   <223> ScFvN5
<400> 52
   Ala Val Thr Leu Asp Glu Ser Gly Gly Gly Leu Gln Thr Pro Gly Gly
   1               5                   10                  15
   Gly Leu Ser Leu Val Cys Lys Ala Ser Gly Phe Asp Phe Ser Ser Tyr
               20                  25                  30
   Asp Met Val Trp Val Arg Gln Ala Pro Gly Lys Gly Leu Glu Trp Val
           35                  40                  45
   Ala Gly Ile Ser Ser Gly Ser Val Pro Asn Tyr Gly Ala Ala Val Lys
       50                  55                  60
   Gly Arg Ala Thr Ile Ser Arg Asp Asn Gly Gln Ser Thr Val Arg Leu
   65                  70                  75                      80
   Gln Leu Asn Asn Leu Arg Ala Glu Asp Thr Ala Thr Tyr Tyr Cys Ala
   85 90 95
   Lys Asn Glu Gly Ala Asp Trp Cys Gly His Tyr Tyr Cys Ser Val Ala
   100 105 110
   Tyr Ile Asp Ala Trp Gly His Gly Thr Glu Val Thr Val Ser Ser Gly
   115 120 125
   Ser Thr Ser Gly Ser Gly Lys Pro Gly Pro Gly Glu Gly Ser Thr Lys
   130 135 140
   Gly Ala Pro Ala Leu Thr Gln Pro Ser Ser Val Ser Ala Asn Pro Gly
   145 150 155 160
   Glu Thr Val Lys Ile Thr Cys Ser Gly Gly Ser Gly Asp His Tyr Gly
   165 170 175
   Trp Phe Gln Gln Lys Ser Pro Gly Ser Ala Pro Val Thr Val Ile Tyr
   180 185 190
   Lys Asn Glu Asn Arg Pro Ser Asp Ile Pro Ser Arg Phe Ser Gly Ser
   195 200 205
   Lys Ser Gly Ser Thr His Thr Leu Thr Ile Thr Gly Val Gln Val Glu
   210 215 220
   Asp Glu Ala Val Tyr Phe Cys Gly Asn Arg Asp Ser Ser Ala Gly Ile
   225 230 235 240
   Phe Gly Ala Gly Thr Thr Leu Thr Val Leu Gly Gln Pro
   245 250
<210> 53
   <211> 253
   <212> PRT
   <213> Artificial sequence
<220>
   <223> ScFv N12 <210> 54
   <211> 250
   <212> PRT
   <213> Artificial sequence
<200>
   <223> ScFv N10
<400> 54
<210> 55
   <211> 258
   <212> PRT
   <213> Artificial sequence
<200>
   <223> ScFv P6
<400> 55
   Ala Val Thr Leu Asp Glu Ser Gly Gly Gly Leu Gln Thr Pro Gly Gly
   1               5                   10                  15
   Ala Leu Ser Leu Val Cys Lys Ala Ser Gly Phe Asp Phe Ser Ser Tyr
               20                  25                  30
   Gly Met Val Trp Val Arg Gln Ala Pro Gly Lys Gly Leu Glu Trp Val
           35                  40                  45
   Ala Gly Ile Asp Ala Asp Gly Ile Tyr Thr Asn Tyr Gly Ala Ala Val
       50                  55                  60
   Lys Gly Arg Ala Thr Ile Ser Arg Asp Asn Gly Gln Ser Thr Val Arg
   65                  70                  75                  80
   Leu Gln Leu Asn Asn Leu Arg Ala Glu Asp Thr Gly Thr Tyr Tyr Cys
                   85                  90                  95
   Ala Lys Gly Ala Tyr Gly Tyr Cys Asp Ser Gly Thr Trp Cys Ala Asp
               100                 105                 110
   Asp Tyr Ile Asp Ala Trp Gly His Gly Thr Glu Val Thr Val Ser Ser
           115                 120                 125
   Gly Ser Thr Ser Gly Ser Gly Lys Pro Gly Pro Gly Glu Gly Ser Thr
       130                 135                 140
   Lys Gly Ala Pro Ala Leu Thr Gln Pro Ser Ser Val Ser Ala Asn Pro
   145                 150                 155                 160
   Gly Glu Thr Val Glu Ile Thr Cys Thr Gly Ser Ser Ser Tyr Tyr Gly
                   165                 170                 175
   Trp Tyr Gln Gln Lys Ser Pro Gly Ser Ala Pro Val Thr Val Ile Tyr
   180 185 190
   Glu Ser Ser Ser Arg Pro Ser Asp Ile Pro Ser Arg Phe Ser Gly Ser
   195 200 205
   Lys Ser Gly Ser Thr Ala Thr Leu Thr Ile Thr Gly Val Gln Val Glu
   210 215 220
   Asp Glu Ala Val Tyr Phe Cys Gly Ser Tyr Asp Gly Ser Ile Asp Ala
   225 230 235 240
   Gly Tyr Val Gly Ile Phe Gly Ala Gly Thr Ile Leu Thr Val Leu Gly
   245 250 255
   Gln Pro
<210> 56
   <211> 254
   <212> PRT
   <213> Artificial sequence
<200>
   <223> ScFv G5
<400> 56
   Ala Val Thr Leu Asp Glu Ser Gly Gly Gly Leu Gln Thr Pro Gly Gly
   1               5                   10                  15
   Thr Leu Ser Leu Val Cys Lys Gly Ser Gly Phe Asp Phe Ser Ser Tyr
               20                  25                  30
   Glu Met Gln Trp Val Arg Gln Ala Pro Gly Lys Gly Leu Glu Trp Val
           35                  40                  45
   Ala Ala Ile Ser Asn Asp Gly Ser Trp Thr Gly Tyr Gly Ala Ala Val
       50                  55                  60
   Lys Gly Arg Ala Thr Ile Ser Arg Asp Asp Gly Gln Ser Thr Val Arg
   65                  70                  75                  80
   Leu Gln Leu Asn Asn Leu Arg Ala Glu Asp Thr Gly Thr Tyr Tyr Cys
                   85                  90                  95
   Ala Lys Ser Val Tyr Gly Gly Cys Gly Asn Ala Ala Ala Gln Ile Asp
               100                 105                 110
   Ala Trp Gly His Gly Thr Glu Val Thr Val Ser Ser Gly Ser Thr Ser
           115                 120                 125
   Gly Ser Gly Lys Pro Gly Pro Gly Glu Gly Ser Thr Lys Gly Ala Pro
       130                 135                 140
   Ala Leu Thr Gln Pro Ser Ser Val Ser Ala Asn Leu Gly Glu Thr Val
   145                 150                 155                 160
   Lys Ile Thr Cys Ser Gly Thr Ser Gly Ser Tyr Tyr Ser Trp His Gln
                   165                 170                 175
   Gln Lys Ser Pro Gly Ser Ala Pro Val Thr Val Ile Tyr Asp Asn Asn
               180                 185                 190
   Lys Arg Pro Ser Asn Ile Pro Ser Arg Phe Ser Gly Ser Thr Ser Gly
           195                 200                 205
   Ser Thr Asn Thr Leu Thr Ile Thr Gly Ile Gln Ala Glu Asp Glu Ala
       210                 215                 220
   Val Tyr Phe Cys Gly Gly Tyr Asp Ser Asp Ser Ala Arg Tyr Val Pro
   225                 230                 235                 240
   Ile Phe Gly Ala Gly Thr Thr Leu Thr Val Leu Gly Gln Pro
                   245                 250
<210> 57
   <211> 257
   <212> PRT
   <213> Artificial sequence
<200>
   <223> ScFv P17
<400> 57
   Ala Val Thr Leu Asp Glu Ser Gly Gly Gly Leu Gln Thr Pro Gly Gly
   1 5 10 15
   Gly Leu Ser Leu Val Cys Lys Ala Ser Gly Phe Ser Ile Gly Gly Tyr
   20 25 30
   Ile Met His Trp Val Arg Gln Ala Pro Gly Lys Gly Leu Glu Tyr Val
   35 40 45
   Ala Gly Ile Asp Ala Gly Gly Gly Val Thr Trp Tyr Gly Ala Ala Val
   50 55 60
   Gln Gly Arg Ala Thr Ile Ser Arg Asp Asn Gly Gln Ser Thr Val Arg
   65 70 75 80
   Leu Gln Leu Asn Ser Leu Arg Ala Glu Asp Thr Ala Thr Tyr Tyr Cys
   85 90 95
   Ala Lys Asp Gly Asp Asn Cys Cys Thr Thr Ser Gly Ala Asp Gln Ile
   100 105 110
   Asp Ala Trp Gly His Gly Thr Glu Val Thr Val Ser Ser Gly Ser Thr
   115 120 125
   Ser Gly Ser Gly Lys Pro Gly Pro Gly Glu Gly Ser Thr Lys Gly Ala
   130 135 140
   Pro Ala Leu Thr Gln Pro Ser Ser Val Ser Ala Asn Pro Gly Glu Thr
   145 150 155 160
   Val Lys Ile Thr Cys Ser Gly Gly Gly Ser Asn Ser Gly Ser Tyr Tyr
   165 170 175
   Tyr Gly Trp Tyr Gln Gln Lys Ser Pro Gly Ser Ala Pro Val Thr Val
   180 185 190
   Ile Tyr Asp Asn Thr Lys Arg Pro Ser Asp Ile Pro Ser Arg Phe Ser
   195 200 205
   Gly Ser Lys Ser Gly Ser Thr Gly Thr Leu Thr Ile Thr Gly Val Gln
   210 215 220
   Ala Asp Asp Glu Ala Val Tyr Tyr Cys Gly Ser Gly Asp Ser Tyr Ser
   225 230 235 240
   Ser Val Gly Ile Phe Gly Ala Gly Thr Thr Leu Thr Val Leu Gly Gln
   245 250 255
   Pro
<210> 58
   <211> 252
   <212> PRT
   <213> Artificial sequence
<200>
   <223> ScFv P7
<400> 58
   Ala Val Thr Leu Asp Glu Ser Gly Gly Gly Leu Gln Thr Pro Gly Gly
   1               5                   10                  15
   Ala Leu Ser Leu Val Cys Lys Gly Ser Gly Phe Thr Phe Ser Ser Asn
               20                  25                  30
   Asp Met Gly Trp Val Arg Gln Ala Pro Gly Lys Gly Leu Glu Trp Val
           35                  40                  45
   Ala Ala Ile Gly Asn Thr Gly Ser Trp Thr Gly Tyr Gly Ala Ala Val
       50                  55                  60
   Lys Gly Arg Ala Thr Ile Ser Arg Asp Asn Gly Gln Ser Thr Val Arg
   65                  70                  75                  80
   Leu Gln Leu Ser Asn Leu Arg Ala Glu Asp Thr Gly Thr Tyr Phe Cys
                   85                  90                  95
   Ala Lys Ala Ala Gly Tyr Cys Gly Ser Gln Ser Cys Gly Ser Ala Ala
               100                 105                 110
   Tyr Ile Asp Ala Trp Gly His Gly Thr Glu Val Thr Val Ser Ser Gly
           115                 120                 125
   Ser Thr Ser Gly Ser Gly Lys Pro Gly Pro Gly Glu Gly Ser Thr Lys
       130                 135                 140
   Gly Ala Pro Ala Leu Thr Gln Pro Ser Ser Val Ser Ala Asn Pro Gly
   145                 150                 155                 160
   Glu Thr Val Lys Ile Ala Cys Ser Gly Ala Ser Tyr Tyr Gly Trp Phe
                   165                 170                 175
   Gln Gln Lys Ser Pro Gly Ser Ala Pro Val Thr Leu Ile Tyr Phe Asp
               180                 185                 190
   Asn Lys Arg Pro Ser Asp Ile Pro Ser Arg Phe Ser Gly Ser Ala Ser
           195                 200                 205
   Gly Ser Thr Ala Thr Leu Thr Ile Thr Gly Val Gln Gly Asp Asp Glu
       210                 215                 220
   Ala Val Tyr Phe Cys Gly Ser Thr Asp Asn Thr Asn Tyr Asp Ile Phe
   225 230 235 240
   Gly Ala Gly Thr Thr Leu Thr Val Leu Gly Gln Pro
   245 250
<210> 59
   <211> 252
   <212> PRT
   <213> Artificial sequence
<200>
   <223> ScFv G6
<400> 59
   Ala Val Thr Leu Asp Glu Ser Arg Gly Gly Leu Gln Thr Pro Gly Gly
   1               5                   10                  15
   Ala Leu Ser Leu Val Cys Lys Gly Ser Gly Phe Val Phe Asp Thr Tyr
               20                  25                  30
   Glu Met Gln Trp Val Arg Gln Ala Pro Gly Lys Gly Leu Glu Trp Val
           35                  40                  45
   Ala Gly Ile Asp Asp Asp Gly Ser Ser Thr Tyr Tyr Ala Thr Ala Val
       50                  55                  60
   Lys Gly Arg Ala Thr Ile Ser Arg Asp Asn Gly Gln Ser Thr Leu Arg
   65                  70                  75                  80
   Leu Gln Leu Asn Asn Leu Arg Ala Glu Asp Thr Gly Ser Tyr Tyr Cys
                   85                  90                  95
   Ala Lys Asp Val Ser Asp Asp Gly Val Cys Gly Gly Ala Ile Trp Ile
               100                 105                 110
   Asp Ala Trp Gly His Gly Thr Glu Val Thr Val Ser Ser Gly Ser Thr
           115                 120                 125
   Ser Gly Ser Gly Lys Pro Gly Pro Gly Glu Gly Ser Thr Lys Gly Ala
       130                 135                 140
   Pro Ala Leu Thr Gln Pro Ser Ser Val Leu Ala Asn Pro Gly Glu Thr
   145                 150                 155                 160
   Val Lys Ile Thr Cys Ser Gly Asp Ala Trp Tyr Gly Trp Tyr Gln Gln
                   165                 170                 175
   Lys Ser Pro Gly Ser Ala Pro Val Thr Leu Ile Tyr Gln Asn Asp Lys
               180                 185                 190
   Arg Pro Ser Asp Ile Pro Ser Arg Phe Ser Gly Ser Thr Ser Gly Ser
           195                 200                 205
   Thr Gly Thr Leu Thr Ile Thr Gly Val Gln Val Glu Asp Glu Ala Val
       210                 215                 220
   Tyr Phe Cys Gly Ser Gly Asp Ser Ser Ala Gly Tyr Val Gly Ile Phe
   225                 230                 235                 240
   Gly Ala Gly Thr Thr Leu Thr Val Leu Gly Gln Pro
                   245                 250
<210> 60
   <211> 250
   <212> PRT
   <213> Artificial sequence
<200>
   <223> ScFv P29
<400> 60
   Ala Val Thr Leu Asp Glu Ser Gly Gly Gly Leu Gln Thr Pro Gly Gly
   1               5                   10                  15
   Ala Leu Ser Leu Val Cys Lys Ala Ser Gly Phe Thr Phe Ser Ser Tyr
               20                  25                  30
   Ala Met Tyr Trp Val Arg Gln Ala Pro Gly Lys Gly Leu Glu Trp Val
           35                  40                  45
   Ala Gly Ile Tyr Ser Ser Gly Ser Ser Thr Tyr Tyr Ala Pro Ala Val
       50                  55                  60
   Lys Gly Arg Ala Thr Ile Ser Arg Asp Asn Gly Gln Ser Thr Val Arg
   65                  70                  75                  80
   Leu Gln Leu Ser Asn Leu Thr Ala Glu Asp Thr Ala Thr Tyr Tyr Cys
                   85                  90                  95
   Ala Lys Glu Ser Tyr Ser Lys Tyr Tyr Gly Pro Gly Glu Ile Asp Ala
               100                 105                 110
   Trp Gly His Gly Thr Glu Val Thr Val Ser Ser Gly Ser Thr Ser Gly
           115                 120                 125
   Ser Gly Lys Pro Gly Pro Gly Glu Gly Ser Thr Lys Gly Ala Pro Ala
       130                 135                 140
   Leu Thr Gln Pro Ser Ser Val Ser Ala Asn Pro Gly Glu Thr Val Lys
   145                 150                 155                 160
   Ile Thr Cys Ser Gly Gly Gly Ser Ser Asp Tyr Gly Trp Tyr Gln Gln
                   165                 170                 175
   Lys Ser Pro Gly Ser Ala Pro Val Thr Val Ile Tyr Gln Lys Asn Gln
               180                 185                 190
   Arg Pro Ser Asp Ile Pro Ser Arg Phe Ser Gly Ser Thr Ser Gly Ser
           195                 200                 205
   Thr Asp Thr Leu Thr Ile Thr Gly Val Arg Ala Glu Asp Glu Ala Val
       210                 215                 220
   Tyr Tyr Cys Gly Ser Thr Asp Asp Ser Ala Thr Val Ile Phe Gly Ala
   225                 230                 235                 240
   Gly Thr Thr Leu Thr Val Leu Gly Gln Pro
                   245                 250
<210> 61
   <211> 257
   <212> PRT
   <213> Artificial sequence
<200>
   <223> ScFv P11
<400> 61
   Ala Val Thr Leu Asp Glu Ser Gly Gly Gly Leu Gln Thr Pro Gly Gly
   1 5 10 15
   Ala Leu Ser Leu Val Cys Lys Ala Ser Gly Phe Ser Ile Asn Ser Tyr
   20 25 30
   Gly Phe Asn Trp Val Arg Gln Ala Pro Gly Lys Gly Leu Glu Trp Val
   35 40 45
   Ala Gly Ile Asn Ala Asp Gly Ser Glu Thr Ala Tyr Gly Ala Ala Val
   50 55 60
   Lys Gly Arg Ala Thr Ile Ser Arg Asp Asn Gly Gln Ser Thr Gly Gly
   65 70 75 80
   Leu Gln Leu Asn Asn Leu Arg Ala Glu Asp Thr Ala Thr Tyr Leu Cys
   85 90 95
   Ala Lys Ser Ile Gly Gly Ser Tyr Cys Gly Ser Ser Gly Cys Tyr Ile
   100 105 110
   Asn Ile Gly Thr Ile Asp Ala Trp Gly His Gly Thr Glu Val Thr Val
   115 120 125
   Ser Ser Gly Ser Thr Ser Gly Ser Gly Lys Pro Gly Pro Gly Glu Gly
   130 135 140
   Ser Thr Lys Gly Ala Pro Ala Met Thr Gln Pro Ser Ser Val Ser Ala
   145 150 155 160
   Asn Pro Gly Glu Thr Val Lys Ile Thr Cys Ser Gly Ser Ser Gly Ser
   165 170 175
   Tyr Gly Trp Tyr Gln Gln Lys Ser Pro Gly Ser Ala Pro Val Thr Val
   180 185 190
   Ile Tyr Thr Asn Asp Lys Arg Pro Ser Asp Ile Ser Ser Arg Phe Ser
   195 200 205
   Gly Ser Lys Ser Gly Ser Thr Ala Thr Leu Thr Ile Thr Gly Val Gln
   210 215 220
   Ala Glu Asp Glu Ala Val Tyr Phe Cys Gly Gly Tyr Asp Tyr Asn Ala
   225 230 235 240
   Asn Thr Gly Ile Phe Gly Ala Gly Thr Thr Leu Thr Val Leu Gly Gln
   245 250 255
   Pro
<210> 62
   <211> 250
   <212> PRT
   <213> Artificial sequence
<200>
   <223> ScFv N3
<400> 62
   Ala Val Thr Leu Asp Glu Ser Gly Gly Gly Leu Gln Thr Pro Gly Gly
   1               5                   10                  15
   Gly Leu Ser Leu Val Cys Lys Ala Ser Gly Phe Ser Phe Ser Ser Tyr
               20                  25                  30
   Ser Met Gly Trp Val Arg Gln Ala Pro Gly Lys Gly Leu Glu Trp Val
           35                  40                  45
   Ala Gly Ile Gly Ser Ser Val Ile Arg Thr Tyr Tyr Ala Pro Ala Val
       50                  55                  60
   Lys Gly Arg Ala Thr Ile Pro Arg Asp Asn Gly Gln Ser Thr Val Arg
   65                  70                  75                  80
   Leu Gln Leu Asn Asp Leu Arg Ala Glu Asp Thr Gly Thr Tyr Tyr Cys
                   85                  90                  95
   Ala Lys Glu Ser Gly Ser Gly Lys Trp Phe Ser Ile Gly Gln Ile Asp
               100                 105                 110
   Ala Trp Gly His Gly Thr Glu Val Thr Val Ser Ser Gly Ser Thr Ser
           115                 120                 125
   Gly Ser Gly Lys Pro Gly Pro Gly Glu Gly Ser Thr Lys Gly Ala Pro
       130                 135                 140
   Ala Leu Thr Gln Pro Ser Ser Val Ser Ala Asn Pro Gly Gly Thr Val
   145                 150                 155                 160
   Gln Ile Thr Cys Ser Gly Gly Ser Gly Ser Tyr Gly Trp Phe Gln Gln
                   165                 170                 175
   Lys Ser Pro Gly Ser Ala Pro Val Thr Val Ile Tyr Ser Ser Asp Lys
               180                 185                 190
   Arg Pro Ser Asp Ile Pro Ser Arg Phe Ser Gly Ser Lys Ser Gly Ser
           195                 200                 205
   Thr Gly Thr Leu Thr Ile Thr Gly Val Gln Ala Asp Asp Glu Ala Val
       210                 215                 220
   Tyr Phe Cys Gly Ser Ala Asp Ser Ser His Leu Gly Ile Phe Gly Ala
   225                 230                 235                 240
   Gly Thr Thr Leu Thr Val Leu Gly Gln Pro
                   245                 250
<210> 63
   <211> 118
   <212> PRT
   <213> Artificial sequence
<200>
   <223> Heavy chain of scFv29
<400> 63
   Glu Val Gln Leu Gln Gln Ser Gly Pro Glu Leu Val Arg Pro Gly Ala
   1               5                   10                  15
   Ser Val Lys Met Ser Cys Lys Ala Ser Gly Tyr Thr Phe Thr Arg Tyr
               20                  25                  30
   Phe Ile Glu Trp Ile Lys Gln Lys Pro Gly Gln Gly Leu Glu Trp Ile
           35                  40                  45
   Gly Tyr Ile Asn Pro Tyr Thr Asp Gly Thr Lys Tyr Asn Glu Lys Phe
       50                  55                  60
   Lys Gly Lys Val Thr Leu Thr Ser Asp Lys Ser Ser Ser Thr Ala Tyr
   65                  70                  75                  80
   Met Glu Leu Ser Ser Leu Thr Ser Glu Asp Ser Ala Val Tyr Tyr Cys
                   85                  90                  95
   Gly Ala Ser Asn Gly Tyr His Ala Leu Asp Tyr Trp Gly Gln Gly Ile
               100                 105                 110
   Ser Val Thr Val Ser Ser
           115
<210> 64
   <211> 111
   <212> PRT
   <213> Artificial sequence
<200>
   <223> Light chain of scFv29
<400> 64
   Asp Ile Glu Leu Thr Gln Ser Pro Ala Ser Leu Thr Val Phe Leu Gly
   1               5                   10                  15
   Gln Arg Ala Thr Ile Ser Cys Arg Ala Ser Glu Ser Val Asp Asp Leu
               20                  25                  30
   Gly Ile Ser Phe Met Asn Trp Phe Gln Gln Lys Pro Gly Gln Pro Pro
           35                  40                  45
   Lys Leu Leu Ile Tyr Ala Ala Ser Asn Leu Gly Ser Gly Val Pro Ala
       50                  55                  60
   Arg Phe Thr Gly Ser Gly Ser Gly Thr Asp Phe Ser Leu Asn Ile His
   65                  70                  75                  80
   Pro Val Glu Glu Gly Asp Thr Ala Met Phe Phe Cys His His Ser Lys
                   85                  90                  95
   Glu Val Pro Tyr Thr Phe Gly Gly Gly Thr Lys Leu Glu Ile Glu
               100                 105                 110
<210> 65
   <211> 864
   <212> DNA
   <213> Artificial sequence
<200>
   <223> Nucleotide sequence encoding scFv29-19
<400> 65
   ttgcctacgg cagccgctgg attgttattc ctgcaggccc agccggccat ggctgccgtg      60
   acgttggacg agtccggggg cggcctccag acgcccggag ggggcctcag cctcgtctgc      120
   aaggcctccg ggtwcacatt cactcgctac ttcatcgagt gggtgcgaca ggcgcccggc     180
   aagggactgg aatggattgg atatattaat ccttacactg atggtactaa gtacaatgag     240
   aagttcaaag gccgtgccac catctcgagg gacaacgggc agagcacagt gaggctgcag     300
   ctgaacaacc tcagggctga ggacaccgcc acctactact gcggggcctc taatgggtac     360
   catgctttgg actactgggg ccacgggacc gaggtcaccg tgagctcagg ctccacctca     420
   ggctccggta aacctggccc aggggaggga tcaactaagg gcgcgattgc gctgactcag     480
   ccgtcctcgg tgtcagcaaa cccgggagaa accgtcaaga tcacctgcag agccagcgaa     540
   agtgttgatg acctgggcat cagcttcatg aactggttcc agcagaagtc tcctggcagt     600
   gcacctgtcc tcctcatcta tgctgcatcc aacttaggat ccgacatccc ttcacgattc     660
   tccggttcca aatccggctc cacattcaca ttaaccatca ctggggtcca agtcgaggac     720
   gaggctgtct atttctgtca tcacagtaag gaggttccgt acacgtttgg ggccgggaca     780
   accctgaccg tcctaggcca gcccgtcgac gcggccgcag aacaaaaact catctcagaa     840
   gaggatctga atggggccgc atag                                            864
<210> 66
   <211> 291
   <212> PRT
   <213> Artificial Sequence
<200>
   <223> Sequence of scFv19-29
<400> 66
   Leu Pro Thr Ala Ala Ala Gly Leu Leu Phe Leu Gln Ala Gln Pro Ala
   1               5                   10                  15
   Met Ala Ala Val Thr Leu Asp Glu Ser Gly Gly Gly Leu Gln Thr Pro
               20                  25                  30
   Gly Gly Gly Leu Ser Leu Val Cys Lys Ala Ser Gly Phe Thr Phe Thr
           35                  40                  45
   Arg Tyr Phe Ile Glu Trp Val Arg Gln Ala Pro Gly Lys Gly Leu Glu
       50                  55                  60
   Trp Ile Gly Tyr Ile Asn Pro Tyr Thr Asp Gly Thr Lys Tyr Asn Glu
   65                  70                  75                  80
   Trp Lys Phe Lys Gly Arg Ala Thr Ile Ser Arg Asp Asn Gly Gln Ser
                   85                  90                  95
   Thr Val Arg Leu Gln Leu Asn Asn Leu Arg Ala Glu Asp Thr Ala Thr
               100                 105                 110
   Tyr Tyr Cys Gly Ala Ser Asn Gly Tyr Trp His Ala Leu Asp Tyr Trp
           115                 120                 125
   Gly His Gly Thr Glu Val Thr Val Ser Ser Gly Ser Thr Ser Gly Ser
       130                 135                 140
   Gly Lys Pro Gly Pro Gly Glu Gly Ser Thr Lys Gly Ala Ile Pro Ala
   145                 150                 155                 160
   Leu Thr Gln Pro Ser Ser Val Ser Ala Asn Pro Gly Glu Thr Val Lys
                   165                 170                 175
   Ile Thr Cys Arg Ala Ser Glu Trp Ser Val Asp Asp Leu Gly Ile Ser
               180                 185                 190
   Phe Met Asn Trp Phe Gln Gln Lys Ser Pro Gly Ser Ala Pro Val Leu
           195                 200                 205
   Leu Ile Tyr Ala Ala Ser Asn Leu Gly Ser Asp Ile Pro Ser Arg Phe
   210 215 220
   Ser Gly Ser Lys Ser Gly Ser Thr Phe Thr Leu Thr Ile Thr Gly Val
   225 230 235 240
   Gln Val Glu Asp Glu Ala Val Tyr Phe Cys His His Ser Lys Glu Val
   245 250 255
   Pro Tyr Thr Phe Gly Ala Gly Thr Thr Leu Thr Val Leu Gly Gln Pro
   260 265 270
   Val Asp Ala Ala Ala Glu Gln Lys Leu Ile Ser Glu Glu Asp Leu Asn
   275 280 285
   Gly Ala Ala
   290
<210> 67
   <211> 54
   <212> DNA
   <213> Artificial Sequence
<200>
   <223> Oligonucleotide Primer
<400> 67
   ggattgttat tcctgcaggc ccagccggcc atggctgccg tgacgttgga cgag 54
<210> 68
   <211> 57
   <212> DNA
   <213> Artificial Sequence
<200>
   <223> Oligonucleotide primer
<220>
   <221>misc_feature
   <222> (34) .. (34)
   <223> The nucleotide at position 34 is a wbble nucleotide such that Phe or Tyr is encoded
<400> 68
   cgcacccact cgatgaagta gcgagtgaat gtgnacccgg aggccttgca gacgagg 57
<210> 69
   <211> 57
   <212> DNA
   <213> Artificial Sequence
<200>
   <223> Oligonucleotide primer
<400> 69
   atccaatcca ttccagtccc ttgccgggcg cctgtcgcac ccactcgatg aagtagc 57
<210> 70
   <211> 58
   <212> DNA
   <213> Artificial Sequence
<200>
   <223> Oligonucleotide primer
<400> 70
   ctcattgtac ttagtaccat cagtgtaagg attaatatat ccaatccatt ccagtccc 58
<210> 71
   <211> 56
   <212> DNA
   <213> Artificial Sequence
<200>
   <223> Oligonucleotide primer
<400> 71
   ccgttgtccc tcgagatggt ggcacggcct ttgaacttct cattgtactt agtacc 56
<210> 72
   <211> 29
   <212> DNA
   <213> Artificial Sequence
<200>
   <223> Oligonucleotide primer
<400> 72
   ggccgtgcca ccatctcgag ggacaacgg 29
<210> 73
   <211> 62
   <212> DNA
   <213> Artificial Sequence
<200>
   <223> Oligonucleotide primer
<400> 73
   ggccccatga gtccaaagca tggtacccat tagaggcccc gcagtagtag gtggcggtgt 60
   cc 62
<210> 74
   <211> 60
   <212> DNA
   <213> Artificial Sequence
<200>
   <223> Oligonucleotide primer
<400> 74
   gaggtggagc ctgagctcac ggtgacctcg gtcccgtggc cccagtagtc caaagcatgg 60
<210> 75
   <211> 28
   <212> DNA
   <213> Artificial Sequence
<200>
   <223> Oligonucleotide primer
<400> 75
   ggggcctcta atgggtacca tgctttgg 28
<210> 76
   <211> 53
   <212> DNA
   <213> Artificial Sequence
<200>
   <223> Oligonucleotide primer
<400> 76
   ttgctgacac cgaggacggc tgagtcagcg caatcgcgcc cttagttgat ccc 53
<210> 77
   <211> 54
   <212> DNA
   <213> Artificial Sequence
<200>
   <223> Oligonucleotide primer
<400> 77
   ggctctgcag gtgatcttga cggtttctcc cgggtttgct gacaccgagg acgg 54
<210> 78
   <211> 55
   <212> DNA
   <213> Artificial Sequence
<200>
   <223> Oligonucleotide primer
<400> 78
   catgaagctg atgcccaggt catcaacact ttgctggct ctgcaggtga tcttg 55
<210> 79
   <211> 53
   <212> DNA
   <213> Artificial Sequence
<200>
   <223> Oligonucleotide primer
<400> 79
   gcactgccag gagacttctg ctggaaccag ttcatgaagc tgatgcccag gtc 53
<210> 80
   <211> 54
   <212> DNA
   <213> Artificial Sequence
<200>
   <223> Oligonucleotide primer
<400> 80
   ccagcagaag tctcctggca gtgcacctgt cctcctcatc tatgctgcat ccaa 54
<210> 81
   <211> 58
   <212> DNA
   <213> Artificial Sequence
<200>
   <223> Oligonucleotide primer
<400> 81
   ggaaccggag aatcgtgaag ggatgtcgga tcctaagttg gatgcagcat agatgagg 58
<210> 82
   <211> 61
   <212> DNA
   <213> Artificial Sequence
<200>
   <223> Oligonucleotide primer
<400> 82
   cccttcacga ttctccggtt ccaaatccgg ctccacattc acattaacca tcactggggt 60
   c 61
<210> 83
   <211> 60
   <212> DNA
   <213> Artificial Sequence
<200>
   <223> Oligonucleotide primer
<400> 83
   gtcccggccc caaacgtgta cggaacctcc ttactgtgat gacagaaata gacagcctcg 60
<210> 84
   <211> 63
   <212> DNA
   <213> Artificial Sequence
<200>
   <223> Oligonucleotide primer
<400> 84
   tgcggccgcg tcgacgggct ggcctaggac ggtcagggtt gtcccggccc caaacgtgta     60
   cgg                                                                   63